(19) Europäisches Patentamt
European Patent Office
Office européen des brevets

(11) Publication number: **0 095 295**
**B1**

(12) **EUROPEAN PATENT SPECIFICATION**

(45) Date of publication of patent specification: **14.01.87**

(21) Application number: **83302744.4**

(22) Date of filing: **16.05.83**

(51) Int. Cl.⁴: **C 07 K 7/64,** C 12 P 21/04, A 61 K 37/02 // (C12R1/045, 1:62)

(54) **Cyclic peptide derivatives.**

(30) Priority: **21.05.82 US 380497**
**21.05.82 US 380498**
**21.05.82 US 380499**
**21.05.82 US 382012**

(43) Date of publication of application:
**30.11.83 Bulletin 83/48**

(45) Publication of the grant of the patent:
**14.01.87 Bulletin 87/03**

(84) Designated Contracting States:
**BE CH DE FR GB IT LI LU NL SE**

(56) References cited:
**EP-A-0 010 421**
**EP-A-0 031 221**
**EP-A-0 055 070**

(73) Proprietor: **ELI LILLY AND COMPANY**
**307, East McCarty Street**
**Indianapolis Indiana 46285 (US)**

(72) Inventor: **Abbott, Bernard John**
**3019 San Jose Drive**
**Greenwood Indiana 46142 (US)**
Inventor: **Debono, Manuel**
**5257 Hinesley Avenue**
**Indianapolis Indiana 46208 (US)**
Inventor: **Fukuda, David Shuichi**
**10873 North 1000 East**
**Brownsburg Indiana 46112 (US)**

(74) Representative: **Hudson, Christopher Mark et al**
**Erl Wood Manor**
**Windlesham Surrey GU20 6PH (GB)**

**EP 0 095 295 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel derivatives of cyclic peptides which possess antibiotic properties and to the method of producing these antibiotic derivatives by semisynthetic means.

There is a great need to develop new antibiotics because of the great possibility and constant threat that antibiotic-specific resistant strains of pathogenic microorganisms will develop. In particular, pathogens within the gram positive genera *Staphylococcus* and *Streptococcus* often are resistant to commonly used antibiotics such as penicillin and erythromycin; see for example, W.O. Foye, *Principles of Medicinal Chemistry,* pp. 684-686 (1974).

In accordance with the invention, new derivatives of the A-21978C cyclic peptides and the pharmaceutically-acceptable salts thereof, have been found to be effective antibiotics.

These new derivatives can be prepared by reacting the A-21978C nucleus or protected derivatives thereof, prepared by deacylating the appropriately blocked A-21978C complex or any of its appropriately blocked individual factors $C_0$, $C_1$, $C_2$, $C_3$, $C_4$, or $C_5$, with the desired acylating agent or an activated derivative thereof.

In accordance with the invention, A-21978C cyclic peptides of formula 1:

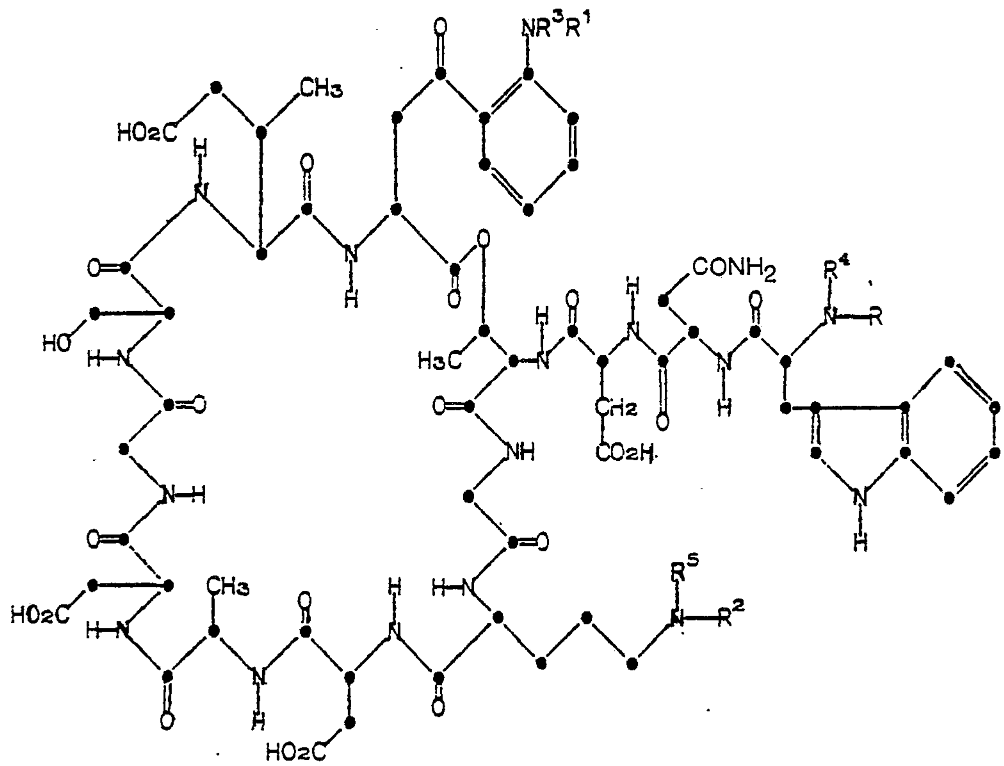

1

in which R, $R^1$, and $R^2$ are, independently, hydrogen, 8-methyldecanoyl, 10-methyldodecanoyl, 10-methylundecanoyl, the specific $C_{10}$-alkanoyl group of A-21978$C_0$ the specific $C_{12}$-alkanoyl groups of A-21978C factors $C_4$ or $C_5$, an amino-protecting group,

(A) an aminoacyl group of the formula

$$-\underset{\underset{O}{\|}}{C}-Q-NH_2$$

in which Q is $C_1$—$C_{16}$ alkylene, or an N-alkanoylaminoacyl group of the formula

$$-W-\underset{\underset{O}{\|}}{C}-R^7$$

in which W is a divalent aminoacyl radical of the formula

(1) 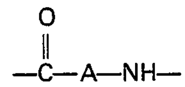

in which A is $C_1$—$C_{10}$ alkylene or $C_5$—$C_6$ cycloalkylene;

(2) 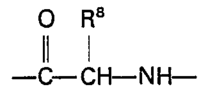

in which $R^8$ is hydroxymethyl, hydroxyethyl, mercaptomethyl, mercaptoethyl, methylthioethyl, 2-thienyl, 3-indole-methyl, phenyl, benzyl, or substituted phenyl or substituted benzyl in which the benzene ring thereof is substituted with chloro, bromo, iodo, nitro, $C_1$—$C_3$ alkyl, hydroxy, $C_1$—$C_3$-alkoxy, $C_1$—$C_3$ alkylthio, carbamyl, or $C_1$—$C_3$ alkylcarbamyl;

(3) 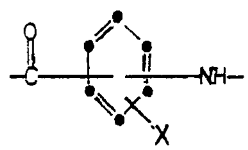

in which X is hydrogen, chloro, bromo, iodo, amino, nitro, $C_1$—$C_3$ alkyl, hydroxy, $C_1$—$C_3$ alkoxy, mercapto, $C_1$—$C_3$ alkylthio, carbamyl, or $C_1$—$C_3$ alkylcarbamyl;

(4) 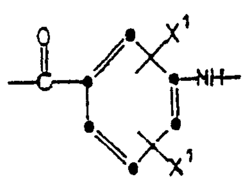 or 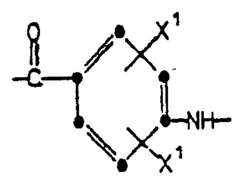

in which $X^1$ is chloro, bromo, iodo, amino, hydroxy, $C_1$—$C_3$ alkyl or $C_1$—$C_3$ alkoxy;

(5) 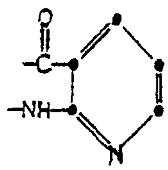 or 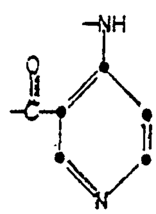 ; or (6) 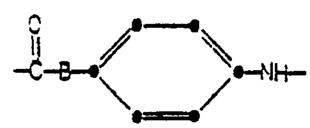

in which B is a divalent radical of the formula: —$(CH_2)_N$— and n is an integer from 1 to 3; —CH=CH—; —CH=CH—$CH_2$—; or

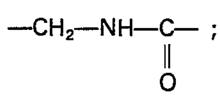 ;

$R^7$ is $C_1$—$C_{17}$ alkyl or $C_2$—$C_{17}$ alkenyl;
(B) a substituted benzoyl group of the formula

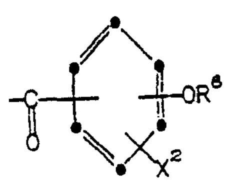

in which $R^6$ is $C_8$—$C_{15}$ alkyl;

$X^2$ is hydrogen, chloro, bromo, iodo, nitro, $C_1$—$C_3$ alkyl, hydroxy, $C_1$—$C_3$ alkoxy or $C_1$—$C_3$ alkylthio;

(C) optionally substituted $C_2$—$C_{19}$ alkanoyl, $C_5$—$C_{19}$ alkenoyl, $C_4$—$C_{14}$ alkyl provided that R, $R^1$ and $R^2$ groups together must contain at least four carbon atoms;

and in which $R^3$, $R^4$ and $R^5$ (i) may represent hydrogen of (ii) taken together with an appropriate adjacent R, $R^1$, $R^2$ group may represent a $C_4$—$C_{14}$ alkylidene group; provided that when $R^1$ and $R^2$ are both selected from hydrogen, R cannot be 8-methyldecanoyl, 10-methylundecanoyl, 10-methyldodecanoyl, the specific $C_{10}$-alkanoyl group of A-21978C factor $C_0$ or the specific $C_{12}$-alkanoyl groups of A-21978C factors $C_4$ and $C_5$; or a pharmaceutically-acceptable salt thereof, are useful in the preparation of, or as semi-synthetic antibiotics.

The compounds of formula 1 in which R is 8-methyldecanoyl, 10-methylundecanoyl, 10-methyldodecanoyl, the $C_{10}$-alkanoyl group of A-21978C factor $C_0$ and the $C_{12}$-alkanoyl groups of A-21978C factors $C_4$ and $C_5$ and one of $R^1$ or $R^2$ is an amino-protecting group are blocked antibiotic A-21978C factors $C_0$, $C_1$, $C_2$, $C_3$, $C_4$ and $C_5$. These blocked antibiotics compounds are useful intermediates to certain peptides of formula 1, *i.e.* those in which R is hydrogen and at least one of $R^1$ and $R^2$ is an amino-protecting group.

The compound of formula 1 in which R, $R^1$ and $R^2$ each represent hydrogen is the common cyclic peptide present in antibiotic A-21978C factors $C_0$, $C_1$, $C_2$, $C_3$, $C_4$ and $C_5$. For convenience, this compound will be called the A-21978C nucleus. This compound can be represented also by formula 2:

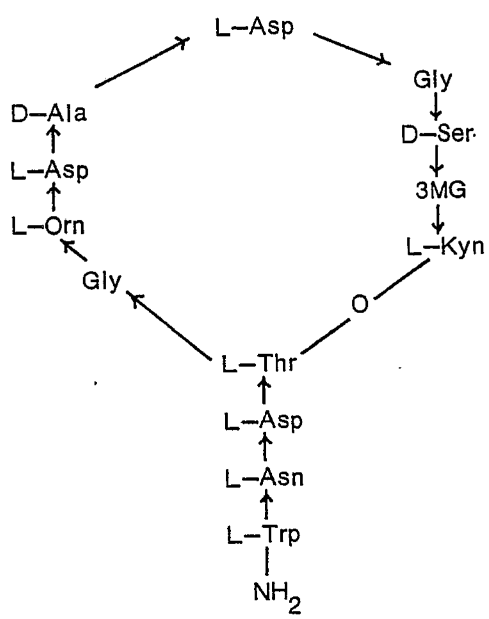

2

in which 3MG represents L-*threo*-3-methylglutamic acid.

In another aspect, this invention relates to a method of enzymatically deacylating an antibiotic selected from A-21978C complex, A-21978C factors $C_0$, $C_1$, $C_2$, $C_3$, $C_4$, and $C_5$, blocked A-21978C complex, and blocked A-21978C factors $C_0$, $C_1$, $C_2$, $C_3$, $C_4$ and $C_5$. The naturally occurring A-21978C factors have a common cyclic peptide nucleus, but each has a different fatty acid group attached to the α-amino group of the tryptophan residue. The method of removing the fatty acid groups from the A-21978C factors and blocked factors provided by this invention comprises exposing the antibiotic or blocked antibiotic, in an aqueous medium, to an enzyme produced by a microorganism of the family *Actinoplanaceae* until substantial deacylation is accomplished.

A preferred method of this invention comprises using an enzyme produced by the microorganism *Actinoplanes utahensis* NRRL 12052 to cleave the fatty acid side chain. Deactylation is ordinarily accomplished by adding the appropriate antibiotic or blocked antibiotic to a culture of *A. utahensis* and permitting the culture to incubate until deacylation is accomplished. The A-21978C cyclic peptide or blocked peptide thereby obtained is separated from the fermentation broth by known methods.

The A-21978C nuclei are useful in that they can be reactylated to provide new antibiotic substances. These new substances are described in detail later.

The accompanying infrared absorption spectra, run in KBr, represent the following:

Figure 1 — A-21978C nucleus (formula 1, R, $R^1$, $R^2$ = H)

Figure 2 — A-21978C $N_{Orn}$-t-BOC nucleus (formula 1, R and $R^1$ = H; $R^2$ = *tert*-butyloxycarbonyl)

In this specification the following abbreviations, most of which are commonly known in the art, are used:

Ala: alanine
Asp: aspartic acid
Gly: glycine
Kyn: kynurenine
Orn: ornithine
Ser: serine
Thr: threonine
Trp: tryptophan
t-BOC: *tert*-butoxycarbonyl
Cbz: benzyloxycarbonyl
DMF: dimethylformamide
THF: tetrahydrofuran
HPLC: high-performance liquid chromatography
TLC: thin-layer chromatography
UV: ultraviolet

The A-21978C antibiotics are closely related acidic peptide antibiotics described in U.S. Patent No. 4,208,403, which is incorporated herein by reference. As described in U.S. Patent No. 4,208,403, the A-21978 antibiotic complex contains a major component, factor C, which is itself a complex of closely related factors A-21978 factor C, which is called the A-21978C complex, contains individual factors $C_0$, $C_1$, $C_2$, $C_3$, $C_4$ and $C_5$. Factors $C_1$, $C_2$ and $C_3$ are major factors; and factors $C_0$, $C_4$ and $C_5$ are minor factors. The structure of the A-21978C factors is shown in formula 3:

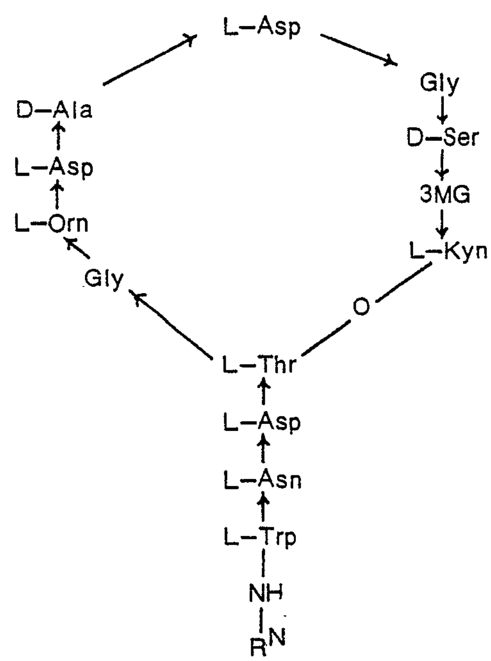

3

in which 3MG represents L-*threo*-3-methylglutamic acid, and $R^N$ represents a specific fatty acid moiety. The specific $R^N$ groups of the factors are as follows:

| A-21978C Factor | $R^N$ Moiety |
|---|---|
| $C_1$ | 8-methyldecanoyl |
| $C_2$ | 10-methylundecanoyl |
| $C_3$ | 10-methyldodecanoyl |
| $C_0$ | $C_{10}$-alkanoyl* |
| $C_4$ | $C_{12}$-alkanoyl* |
| $C_5$ | $C_{12}$-alkanoyl* |

*Identity not yet determined

It is extremely difficult, when confronted with the problem of deacrylating a peptide antibiotic to know whether an enzyme exists which can be used for this purpose. Finding such an enzyme is even more difficult when the substrate antibiotic contains a cyclic peptide nucleus. Because enzymes have a high degree of specificity, differences in the peptide moiety and in the side chain of the substrate will affect the outcome of the deacylation attempt. In addition, many microorganisms make a large number of peptidases which attack different portions of the peptide moiety. This frequently leads to intractable mixtures of products.

In each of the A-21978C antibiotics (formula 3), the fatty acid side chain ($R^N$) is attached at the α-amino group of the tryptophan residue. We have discovered that the fatty acid side chain can be cleaved by an enzyme without affecting the chemical integrity of the remainder of the A-21978C peptide.

The present invention relates also to a novel process for obtaining A-21978C cyclic peptide nuclei having formula 4

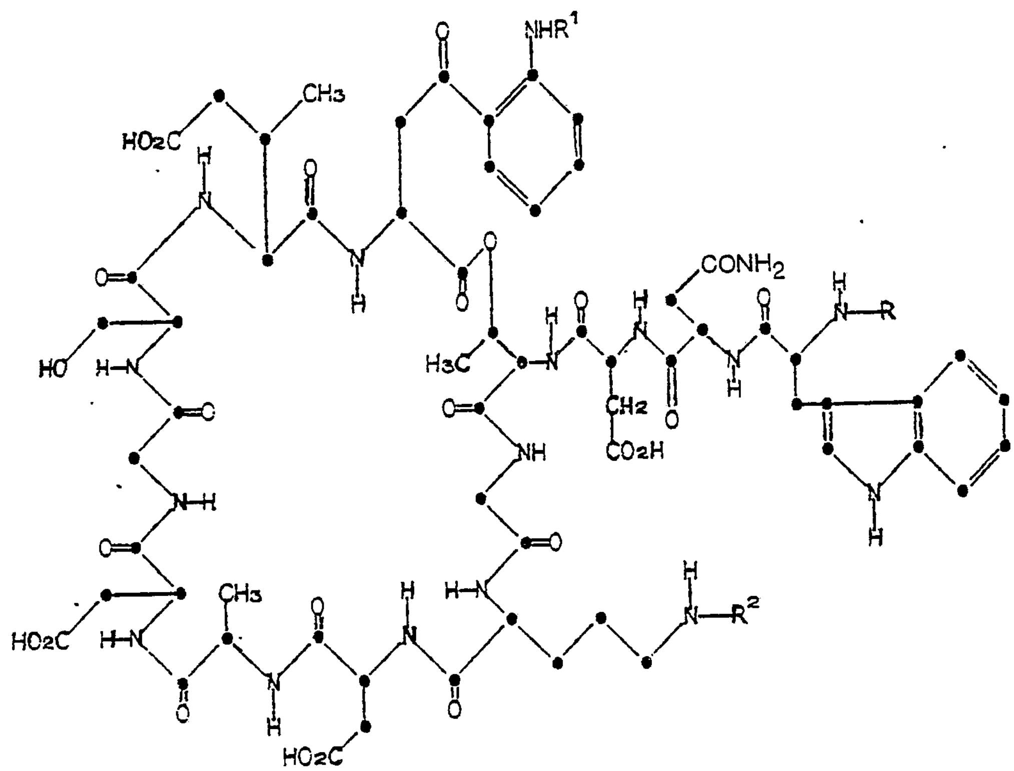

4

in which R is selected from the group consisting of hydrogen, an amino-protecting group, 8-methyldecanoyl, 10-methylundecanoyl, 10-methyldodecanoyl, the specific $C_{10}$-alkanoyl group of A-21978C factor $C_0$ and the specific $C_{12}$-alkanoyl moieties of A-21978C factors $C_4$ and $C_5$; $R^1$ and $R^2$ are, independently,

hydrogen or an amino-protecting group; provided that, when R is other than hydrogen or an amino-protecting group, at least one of $R^1$ and $R^2$ must be an amino-protecting group; or to a pharmaceutically-acceptable salt of these compounds. The cyclic peptides of formula 4 or their pharmaceutically-acceptable salts are useful intermediates in the preparation of new semi-synthetic antibacterial agents which are especially useful against gram-positive microorganisms.

The term "amino-protecting group" as used throughout this description refers to a recognized amino-protecting group which is compatible with the other functional groups in the A-21978C molecule. Preferably, amino-protecting groups are those which can be readily removed subsequently. Examples of suitable protecting groups can be found in "Protective Groups in Organic Synthesis" by Theodora W. Greene, John Wiley and Sons, New York, 1981, Chapter 7. Especially preferred amino-protecting groups are the *tert*-butoxycarbonyl and benzyloxycarbonyl groups.

The cyclic peptides of formula 4 in which R is hydrogen and $R^1$ or $R^2$ is an amino-protecting group or hydrogen include the common cyclic peptide of A-21978C factors $C_0$, $C_1$, $C_2$, $C_3$, $C_4$ and $C_5$ (A-21978C nucleus) and blocked derivatives of A-21978C nucleus. A-21978C nucleus, *i.e.* the compound of formula 4 in which each of R, $R^1$ and $R^2$ represents hydrogen, is alternately described by formula 2.

A-21978C nucleus has the following characteristics:

Form: white amorphous solid which fluoresces under short-wave UV

Empirical formula: $C_{62}H_{83}N_{17}O_{25}$

Molecular weight: 1465

Solubility: soluble in water

Infrared absorption spectrum (KBr): shown in Figure 1 of the accompanying drawings; absorption maxima are observed at the following frequencies ($cm^{-1}$):

3300 (broad), 3042 (weak), 2909 (weak), 1655 (strong), 1530 (strong), 1451 (weak), 1399 (medium), 1222 (medium), 1165 (weak), 1063 (weak) and 758 (medium to weak)

Ultraviolet (UV) absorption spectrum (methanol): UV maxima 223 nm (ε 41,482) and 260 mm (ε 8,687)

Electrometric titration (66% aqueous dimethylformamide): indicates the presence of four titratable groups with $pK_a$ values of about 5.2, 6.7, 8.5 and 11.1 (initial pH 6.12)

A particularly useful cyclic peptide of formula 4 is the compound in which R and $R^1$ represent hydrogen and $R^2$ represents *tert*-butoxycarbonyl. For convenience, this compound is designated "t-BOC nucleus". A-21978C t-BOC nucleus has the following characteristics:

Form: white amorphous solid which fluoresces under short-wave UV

Empirical formula: $C_{67}H_{91}N_{17}O_{27}$

Molecular weight: 1565

Solubility: soluble in water

Infrared absorption spectrum (KBr): shown in Figure 2 of the accompanying drawings; absorption maxima are observed at the following frequencies ($cm^{-1}$):

3345 (broad), 3065 (weak), 2975 (weak), 2936 (weak), ~1710 (shoulder), 1660 (strong), 1530 (strong), 1452 (weak), 1395 (medium), 1368 (weak), 1341 (weak), 1250 (medium), 1228 (medium), 1166 (medium to weak) and 1063 (weak)

Ultraviolet absorption spectrum (90% ethanol): UV maxima 220 nm (ε 42,000) and 260 nm (ε 10,600)

High-performance liquid chromatography: retention time = 6 min on 4.6- × 300-mm silica-gel $C_{18}$ column, using $H_2O/CH_3CN/CH_3OH$ (80:15:5) solvent containing 0.2% $NH_4OAc$ at a flow rate of 2 ml/min with UV detection.

The blocked A-21978C factors of this invention are those compounds of formula 1 in which at least one of $R^1$ and $R^2$ is an amino-protecting group and R is selected from 8-methyldecanoyl, 10-methylundecanoyl, 10-methyldodecanoyl, the specific $C_{10}$-alkanoyl group of A-21978C factor $C_0$ and the specific $C_{12}$-alkanoyl groups of A-21978C factors $C_4$ and $C_5$.

The A-21978C blocked factors and cyclic peptides of this invention are capable of forming salts which are also part of this invention. Such salts are useful, for example, for separating and purifying the compounds. Pharmaceutically-acceptable alkali-metal, alkaline-earth-metal, amine and acid-addition salts are particularly useful. "Pharmaceutically-acceptable" salts are salts which are useful in the chemotherapy of warm-blooded animals.

For example, the A-21978C cyclic peptides of formula 1 have four free carboxyl groups which can form salts. Partial, mixed and complete salts of these carboxyl groups are contemplated, therefore, as part of this invention. In preparing these salts, pH levels greater than 10 should be avoided due to the instability of the compounds at such levels.

Representative and suitable alkali-metal and alkaline-earth metal salts of the A-21978C cyclic peptides of formula 1 include, among others, the sodium, potassium, lithium, cesium, rubidium, barium, calcium and magnesium salts. Suitable amine salts of the A-21978C cyclic peptides include the ammonium and the primary, secondary, and tertiary $C_1$—$C_4$-alkylammonium and hydroxy-$C_2$—$C_4$-alkylammonium salts. Illustrative amine salts include, among others, those formed by reaction of an A-21978C cyclic peptide with ammonium hydroxide, methylamine, *sec*-butylamine, isopropylamine, diethylamine, di-isopropylamine, cyclohexylamine, ethanolamine, triethylamine, and 3-amino-1-propanol.

The alkali-metal and alkaline-earth-metal cationic salts of the A-21978C cyclic peptides of formula 1 are prepared according to procedures commonly used for the preparation of cationic salts. For example, the

free acid form of the A-21978C cyclic peptide is dissolved in a suitable solvent such as warm methanol or ethanol; a solution containing the stoichiometric quantity of the desired inorganic base in aqueous methanol then is added. The salt thus formed can be isolated by routine methods, such as filtration or evaporation of the solvent. Another convenient method of preparing salts is by the use of ion-exchange resins.

The salts formed with organic amines can be prepared in a similar manner. For example, the gaseous or liquid amine can be added to a solution of an A-21978C cyclic peptide in a suitable solvent such as acetone; the solvent and excess amine can be removed by evaporation.

The A-21978C cyclic peptide of this invention also have free amino groups and can form, therefore, acid addition salts which are also part of this invention. Representative and suitable acid-addition salts of the A-21978C cyclic peptides include, among others, those salts formed by standard reaction with both organic and inorganic acids such as, for example, hydrochloric, sulfuric, phosphoric, acetic, succinic, citric, lactic, maleic, fumaric, palmitic, cholic, pamoic, mucic, D-glutamic, *d*-camphoric, glutaric, glycolic, phthalic, tartaric, lauric, stearic, salicyclic, methanesulfonic, benzenesulfonic, sorbic, picric, benzoic, or cinnamic acids.

Preparation of the A-21978C Cyclic Peptides

The A-21978C cyclic peptides of formula 1 in which R is hydrogen and $R^1$ or $R^2$ is an amino-protecting group or hydrogen are obtained by deacylating a peptide antibiotic selected from the group consisting of A-21978C factors $C_0$, $C_1$, $C_2$, $C_3$, $C_4$ and $C_5$ and blocked A-21978C factors $C_0$, $C_1$, $C_2$, $C_3$, $C_4$ and $C_5$.

I. Preparation of the Substrates

A-21978C factors $C_0$, $C_1$, $C_2$, $C_3$, $C_4$ and $C_5$ are prepared as described in U.S. Patent No. 4,208,403. These factors are components of the A-21978C complex which is part of the A-21978 complex.

The A-21978 complex may be produced by culturing *Streptomyces roseosporus* NRRL 11379 (deposited August 29, 1978) under submerged aerobic fermentation conditions until a substantial level of antibiotic activity is produced. The A-21978 complex is separated by filtering the fermentation broth, lowering the pH of the filtrate to about pH 3, allowing the complex to precipitate, and separating the complex by filtration. The separated complex may be purified further by extraction techniques. For isolation of the individual A-21978C complex and factors, chromatographic separations are required.

Blocked A-21978C complex and the blocked A-21978C factors $C_0$, $C_1$, $C_2$, $C_3$, $C_4$ and $C_5$ of this invention (compounds of formula 1 in which R is selected from the group consisting of 8-methyldecanoyl, 10-methylundecanoyl, 10-methyldodecanoyl, and the $C_{10}$-and $C_{12}$-alkanoyl groups of factors $C_0$, $C_4$ and $C_5$) are prepared using procedures for protecting amino groups in peptides. The protecting groups are selected from the various known amino-protecting groups such as, for example, benzyloxycarbonyl, t-butoxycarbonyl, t-amyloxycarbonyl, isobornyloxycarbonyl, adamantyloxycarbonyl, o-nitrophenylthio, diphenylphosphinothioyl, chloro- or nitrobenzyloxycarbonyl.

Blocked A-21978C complex is especially advantageous as a substrate. It is prepared from the A-21978C complex, thereby avoiding the separation steps required to obtain the individual A-21978C factors; but, when it is deacylated, it gives a single product, *i.e.,* the appropriate blocked nucleus.

II. The Deacylation Procedure

A. Preparation of the Enzyme

*1. The Producing Microorganisms*

The enzymes which are useful for deacylation of A-21978C factors $C_0$, $C_1$, $C_2$, $C_3$, $C_4$ and $C_5$ and blocked factors $C_0$, $C_1$, $C_2$, $C_3$, $C_4$ and $C_5$ may be produced by certain microorganisms of the family *Actinoplanaceae,* preferably the microorganism *Actinoplanes utahensis* NRRL 12052 (deposited October 9, 1979). Although a preferred method of cultivating *A. utahensis* NRRL 12052 to produce this enzyme is described in Example 1, those skilled in the art will recognize that other methods may be used.

The *Actinoplanaceae* are a family of microorganisms of the order Actinomycetales. First described by Dr. John N. Couch, this family was established in 1955 [*J. Elisha Mitchell Sci. Soc. 71,* 148-155 (1955)]. The characteristics of the family and of many individual genera are found in "Bergey's Manual of Determinative Bacteriology", 8th ed., R. E. Buchanan and N. E. Gibbons, Eds., The Williams & Wilkins Co., Baltimore, Md., 1974, pages 706—723. Ten genera have thus far been distinguished: I. *Actinoplanes* (the type genus and thus far the most common genus); II. *Spirillospora;* III. *Streptosporangium;* IV. *Amorphosporangium;* V. *Ampullariella;* VI. *Pilimelia;* VII. *Planomonospora;* VIII. *Planobispora;* IX. *Dactylosporangium;* and X. *Kitasatoa.*

Some of the species and varieties which have been isolated and characterized so far are: *Actinoplanes philippinensis, Actinoplanes armeniacus, Actinoplanes utahensis,* and *Actinoplanes missouriensis; Spirillospora albida; Streptosporiangium roseum, Streptosporangium vulgare, Streptosporangium roseum* var. *hollandensis, Streptosporangium album, Streptosporangium viridialbum, Amorphosporangium auranticolor, Ampullariella regularis, Ampullariella campanulata, Ampullariella lobata, Ampullariella digitata, Pilimelia terevasa, Pilimelia anulata, Planomonospora parontospora, Planomonospora venezuelensis, Planobispora longispora, Planobispora rosea, Dactylosporangium aurantiacum,* and *Dactylosporangium thailandense.*

The genus *Actinoplanes* is a preferred source of the enzyme which is useful for this invention. Within the genus *Actinoplanes,* the species *Actinoplanes utahensis* is an especially preferred source.

Cultures of other representatives useful species which produce the enzyme are available to the public from the Nothern Regional Research Center, Agricultural Research Culture Collection (NRRL), U.S. Department of Agriculture, 1815 North University St., Peoria, Illinois 61604, U.S.A. under the following accession numbers;

*Actinoplanes utahensis* NRRL 12052 (deposited October 9, 1979)
*Actinoplanes missouriensis* NRRL 12053 (deposited October 9, 1979)
*Actinoplanes sp.* NRRL 8122 (deposited October 17, 1975)
*Actinoplanes sp.* NRRL 12065 (deposited November 5, 1979)
*Streptosporangium roseum* var. *hollandensis* NRRL 12064 (deposited November 5, 1979)

*A. utahensis* NRRL 12052 was derived from a parent culture which was also deposited with the American Type Culture Collection (ATCC), 12301 Parklawn Drive, Rockville, Md. 20852 (*A. utahensis* ATCC 14539). The *A. utahensis* ATCC 14539 culture may also be used as a source of the enzyme.

*A. missouriensis* NRRL 12053 was derived from a culture which was also deposited with ATCC (*A. missouriensis* ATCC 14538) and which is another source of the enzyme.

The effectiveness of any given strain of microorganism within the family *Actinoplanaceae* for carrying out the deacylation of this invention is determined by the following procedure. A suitable growth medium is inoculated with the microorganism. The culture is incubated at about 30°C. for two or three days on a rotary shaker. One of the substrate antibiotics is added then to the culture maintaining the pH of the fermentation medium at about pH 7.0. The culture is monitored for activity using a *Micrococcus luteus* assay. This procedure is described in Sect. D. Loss of antibiotic activity is an indication that the microorganism produces the requisite enzyme for deacylation. This must be verified, however, using one of the following methods: 1) analysis by HPLC for presence of the intact nucleus; or 2) re-acylation with an appropriate side chain (e.g. lauroyl, *n*-decanoyl or n-dodecanoyl) to restore activity. Reduction in antibiotic activity is difficult to distinguish when deacylating blocked A-21978C material because addition of the blocking group causes an 80—90% reduction in antibiotic activity.

*2. Conditions for Enzyme Production*

Production of the enzyme occurs under conditions satisfactory for growth of the *Actinoplanaceae,* i.e., a temperature between about 25 and about 30°C. and a pH of between about 5.0 and about 8.0, with agitation and aeration. The culture medium should contain a) an assimilable carbon source such as sucrose, glucose, glycerol, or the like; b) a nitrogen source such as peptone, urea, ammonium sulfate, or the like; c) a phosphate source such as a soluble phosphate salt; and d) inorganic salts found generally to be effective in promoting the growth of microorganisms. An effective amount of the enzyme is generally obtained in from about 40 to about 60 hours after the beginning of the growth cycle and persists for some time after the effective growth has been reached. The amount of enzyme produced varies from species to species of the organism and in response to different growth conditions.

As will be apparent, the microorganisms which produce the enzyme, such as *Actinoplanes utahensis* NRRL 12052, are subject to variation. For example, artificial variants and mutants of these strains may be obtained by treatment with various known mutagens such as ultraviolet rays, X-rays, high-frequency waves, radioactive rays, and chemicals. All natural and artificial variants and mutants which are obtained from the *Actinoplanaceae* and which produce the enzyme may be used in this invention.

B. Deacylation Conditions

The substrate used as the starting material preferably is added to the culture of *Actinoplanaceae* after the culture has been incubated for about 48 hours. The concentration of substrate in the conversion medium can vary widely. For maximum use of the enzyme and for substantially complete deacylation within a three-hour period, however, the concentration of substrate will generally range from about one to about three mg/ml. Lower concentrations can be used, but may not make maximum use of the enzyme; higher concentrations can be used also, but the substrate may not be completely deacylated unless the fermentation time is extended.

Conversion of the substrate antibiotic to A-21978C nucleus according to this invention proceeds best when the pH of the fermentation medium is maintained in the range of from about 7.0 to about 7.2. Below pH 7, deacylation proceeds slowly; as pH values move above pH 7.2, the nucleus which is formed is increasingly subject to alkaline hydrolysis. In stirred fermentors the pH may be controlled by sensor controllers. Where this is impractical, such as in flask fermentors, pH may be controlled by adding 0.1 molar phosphate buffer to the medium prior to addition of the substrate.

After addition of the substrate, incubation of the culture should be continued for about 3—6 hours or longer. The purity of the substrate will affect the rate of deacylation. For example, a substrate having a purity of greater than 50 percent is deacylated at a rate of about 2.5 mg/ml of antibiotic in 3 hours. When substrates of lower purity are used, the deacylation proceeds at a somewhat slower rate.

Multiple substrate feedings may be made. For example, 0.75 mg/ml of antibiotic may be fed at 24-hour intervals for at least five or more additions.

The deacylation can be carried out over a broad temperature range, e.g. from about 20 to about 45°C. It

is preferable, however, to carry out the deacylation at a temperature of about 30°C. for optimum deacylation and stability of substrate and nucleus.

C. The Substrate

It is preferable, but not essential, to use purified antibiotic as the substrate. Because purified substrate is soluble in water or in buffer, it can be handled more conveniently. Moreover, with purified substrate the deacylation proceeds more rapidly. Semipurified substrates containing as little as 15 percent of the starting antibiotic have been deacylated successfully.

The substrate antibiotics have antibacterial activity. Thus, although the substrate materials (especially those of low purity) may harbor bacterial cells or spores which presumably could grow in the deacylation medium and affect the deacylation reaction or the stability of the starting antibiotic or the product nucleus, this has not been observed. It is not necessary, therefore, that the substrates be sterile, especially for short deacylation periods.

D. Monitoring the Deacylation

A-21978C factors $C_0$, $C_1$, $C_2$, $C_3$, $C_4$ and $C_5$ are antibacterial agents which are especially active against *Micrococcus luteus.* For this reason an assay using *M. luteus* is preferred for determining quantities of substrate present. The A-21978C nucleus which is formed is water soluble, but is biologically inactive. Reduction in biological activity is, therefore, a quick, presumptive test for deacylation.

The amount of nucleus formed can be quantitated by HPLC analysis, using the system described.

E. Use of Resting Cells

An alternate method of deacylation involves removing the *Actinoplanaceae* cells from the culture medium, resuspending the cells in a buffer solution, and carrying out the deacylation as described in Sect. B. When this method is used, the enzymatically active mycelia can be reused. For example, *A. utahensis* NRRL 12052 mycelia retain deacylase activity after storage for one month or longer under refrigeration (4—8°C.) or in the frozen state (−20°C.). A preferred buffer solution is 0.1 molar phosphate buffer.

F. Immobilized Enzymes

Yet another method of carrying out the deacylation is to immobilize the enzyme by methods known in the art. (See, for example, "Biomedical Applications of Immobilized Enzymes and Proteins", Thomas Ming Swi Chang, Ed., Plenum Press, New York, 1977; Vol. 1.) The immobilized enzyme can then be used in a column (or other suitable type of reactor) to effect the deacylation.

In addition, the microorganism itself can be immobilized and used to catalyze the deacylation reaction.

Utility of the A-21978C Cyclic Peptides

The A-21978C cyclic peptide nuclei and their salts are useful intermediates in the preparation of semi-synthetic antibacterial compounds.

Therefore, in another aspect of this invention, compounds having the structure shown in formula 1:

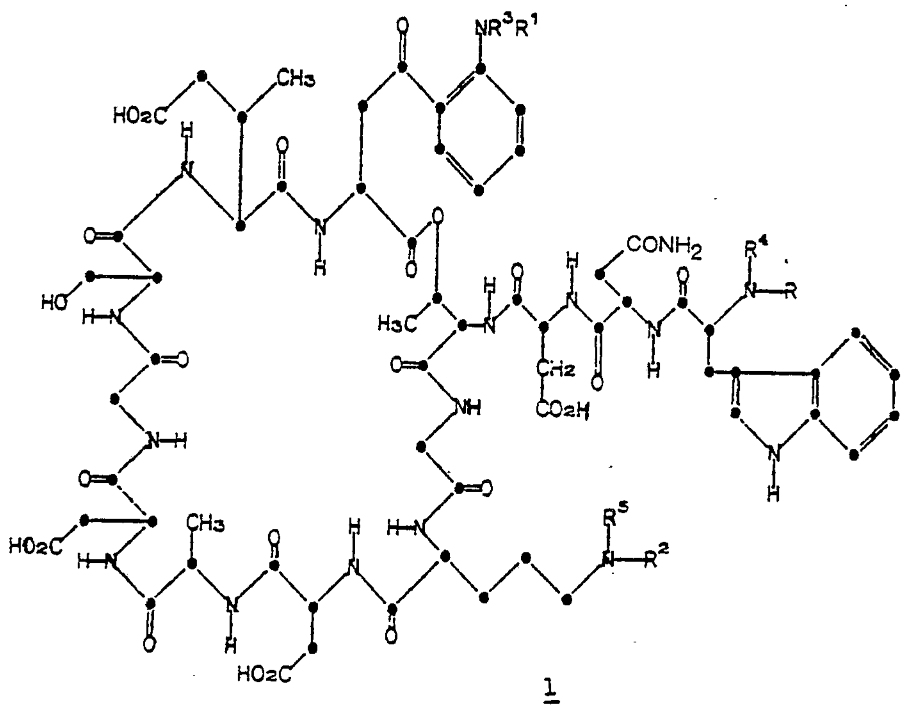

1

in which R, $R^1$ and $R^2$ are, independently, hydrogen, $C_4$—$C_{14}$-alkyl, optionally substituted $C_2$—$C_{19}$-alkanoyl, $C_5$—$C_{19}$-alkenoyl or an amino-protecting group, $R^3$, $R^4$ and $R^5$ are all hydrogen, or (i) $R^3$ and $R^1$; and/or (ii) $R^4$ and R, and/or (iii) $R^5$ and $R^2$, taken together may represent a $C_4$—$C_{14}$ alkylidene group, provided that 1) the R, $R^1$ and $R^2$ groups must together contain at least four carbon atoms, and 2) when $R^1$ and $R^2$ are both selected from hydrogen group, R cannot be 8-methyldecanoyl, 10-methylundecanoyl, 10-methyldodecanoyl, the specific $C_{10}$-alkanoyl group of A-21978C factor $C_0$ or the specific $C_{12}$-alkanoyl groups of A-21978C factors $C_4$ and $C_5$; or a pharmaceutically-acceptable salt thereof, are chemotherapeutically-useful antibiotics.

The term "$C_4$—$C_{14}$-alkylidenyl" refers to a group of the formula

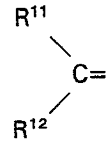

in which $R^{11}$ and $R^{12}$ are hydrogen or an alkyl group of from 3 to 13 carbon atoms, provided that one of $R^{11}$ and $R^{12}$ must be other than hydrogen and further provided that the sum of the carbon atoms in $R^{11}$ and $R^{12}$ must be no greater than 13. Those compounds in which at least one of R, $R^1$ or $R^2$ is $C_4$—$C_{14}$-alkylidenyl are known as Schiff's bases.

The term "$C_4$—$C_{14}$-alkyl" refers to a univalent saturated, straight- or branched-chain alkyl group containing from 4 to 14 carbon atoms. Those compounds in which one of R, $R^1$ or $R^2$ are $C_4$—$C_{14}$-alkyl are prepared by reduction of the corresponding compounds where the R, $R^1$ or $R^2$ group is $C_4$—$C_{14}$-alkylidenyl. The resulting alkyl derivatives are referred to as "reduced Schiff's bases".

The compounds of formula 1 in which one or more of the amino groups are substituted by an alkylidenyl (the Schiff's bases) or alkyl group (the reduced Schiff's bases) can be prepared by known methods for preparing Schiff's bases and reducing such bases, respectively. Thus, the Schiff's bases are prepared by reaction (condensation) of the primary amino group of tryptophan, ornithine or kynurenine of A-21978C with an appropriate aldehyde or ketone in a suitable solvent. Reduction of the imine bond of the Schiff's base, to obtain the corresponding formula 1 compound in which R, $R^1$ or $R^2$ is alkyl, can be accomplished by known selective reduction procedures. A preferred reducing agent for this reaction is sodium cyanoborohydride.

Under the *in vitro* test conditions used, the Schiff's bases do not show antibacterial activity, possibly due to their instability in the assay medium. They are useful, however, as intermediates to the reduced Schiff's bases. When the Schiff's base is used as an intermediate, it is not necessary to isolate the intermediate prior to reducing it to form the reduced Schiff's base.

The terms "optionally substituted $C_2$—$C_{19}$-alkanoyl" and "$C_5$—$C_{19}$-alkenoyl" refer to acyl groups derived from carboxylic acids containing from 2 to 19 and 5 to 19 carbon atoms, respectively. When the R group is alkanoyl, the alkyl portion is a univalent saturated, straight-chain or branched-chain hydrocarbon radical which optionally can bear one hydroxyl group or from one to three halo substituents selected from chlorine, bromide, and fluorine. When R is alkenoyl the alkenyl portion is a univalent, unsaturated, straight-chain or branched-chain hydrocarbon radical containing not more than three double bonds. Any one of the double bond(s) of the unsaturated hydrocarbon chain may be in the *cis* or *trans* configuration.

The term "amino-protecting group" refers to a recognized amino-protecting group as defined *supra.*

The following are preferred embodiments of these compounds of formula 1:

(a) The compounds in which R is alkanoyl of the formula

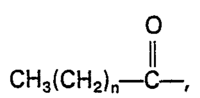

in which n is an integer from 3 to 17;

(b) The compounds in which R is alkanoyl of the formula

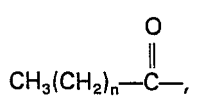

in which n is 5 to 14;

(c) The compounds in which R is alkanoyl of the formula

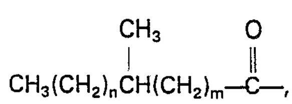

in which n and m are each, independently, an integer from 0 to 14, provided that n + m must be no less than 1 and no greater than 15; and further provided that, if $R^1$ and $R^2$ are hydrogen, when n is 0, m cannot be 8 and, when n is 1, m cannot be 6 or 8;

(d) The compounds in which R is *cis* or *trans* alkenyl of the formula

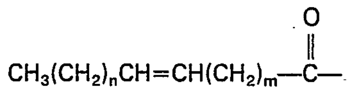

in which n and m are each, independently, an integer from 0 to 14, provided that n + m must be no less than 1 and no greater than 15;

(e) The compounds in which R is *cis* or *trans* alkenyl of the formula

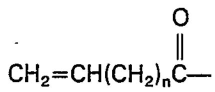

in which n is an integer from 4 to 15;

(f) The compounds in which R is alkyl of the formula $CH_3(CH_2)_n$— and n is an integer from 5 to 12; and

(g) The compounds in which R is:

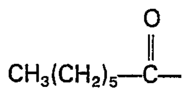

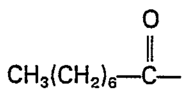

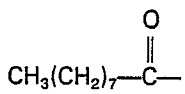

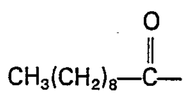

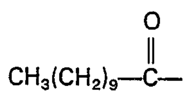

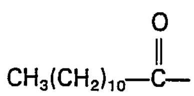

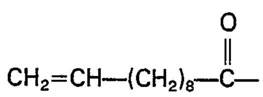

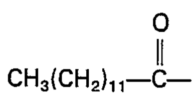

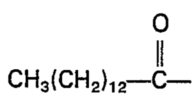

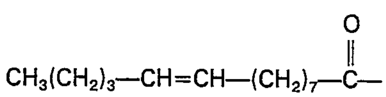

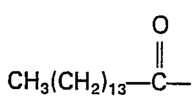

$$CH_3CH_2CH(CH_3)-(CH_2)_6-\overset{\overset{O}{\|}}{C}-$$

$$CH_3(CH_2)_{11}-$$

$$CH_3(CH_2)_{10}CH=$$

$$CH_3(CH_2)_6-$$

$$CH_3(CH_2)_5CH=$$

$$CH_3(CH_2)_9-$$

$$CH_3(CH_2)_8CH=$$

These alkanoyl and alkenoyl derivatives of formula 1 are prepared by acylating an A-21978C nucleus with the desired alkanoyl or alkenoyl side chain using conventional methods for forming an amide bond. In general, the acylation is accomplished by reacting a nucleus with an activated derivative of the acid (formula 7) corresponding to the desired acyl side chain.

$$R^{10}-COOH \qquad (7)$$

($R^{10}$ is optionally substituted $C_1-C_{18}$ alkyl or $C_4-C_{18}$ alkenyl).

In another aspect of this invention, other chemotherapeutically-useful compounds have the general formula 1:

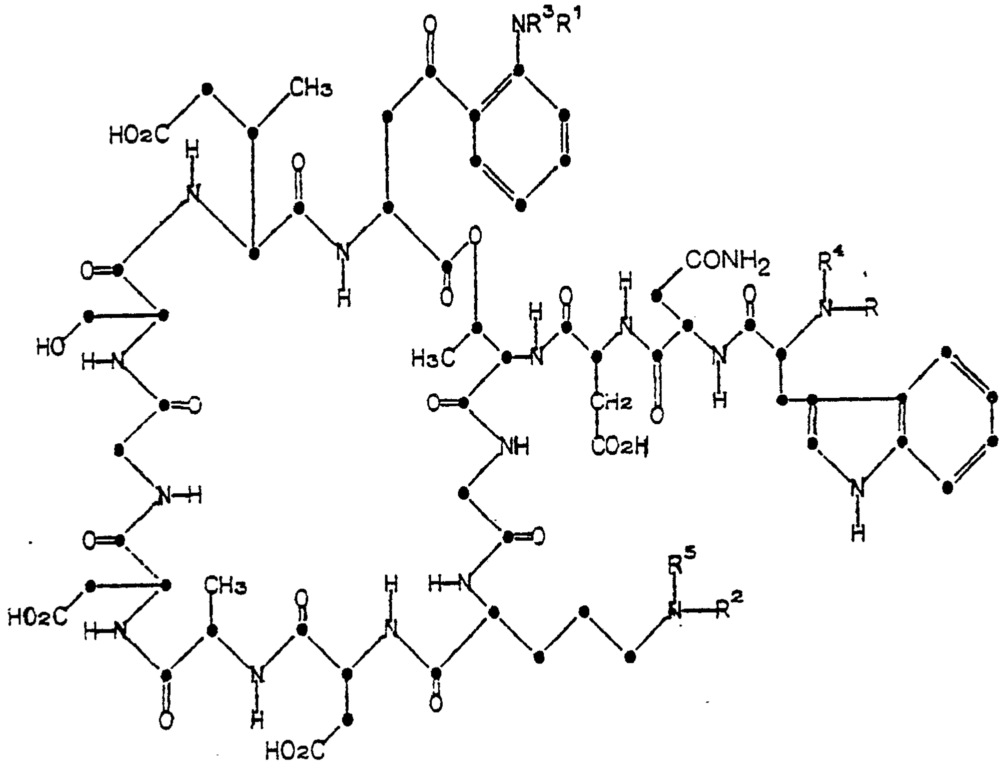

1

These additional derivatives are those in which R is hydrogen, 8-methyldecanoyl, 10-methyldodecanoyl, 10-methylundecanoyl, the specific $C_{10}$-alkanoyl group of A-21978$C_0$ or the specific $C_{12}$-alkanoyl groups of A-21978C factors $C_4$ and $C_5$, and amino-protecting group, an aminoacyl group of the formula

$$-\overset{\overset{O}{\|}}{C}-Q-NH_2$$

in which Q is $C_1$—$C_{16}$ alkylene or an N-alkanoylamino acyl group of the formula

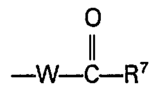

in which:

W is a divalent aminoacyl radical of the formula:

(a) 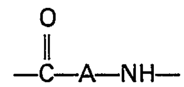

in which A is $C_1$—$C_{10}$ alkylene or $C_5$—$C_6$ cycloalkylene;

(b) 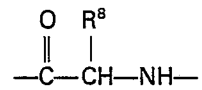

in which $R^8$ is hydroxymethyl, hydroxyethyl, mercaptomethyl, mercaptoethyl, methylthioethyl, 2-thienyl, 3-indole-methyl, phenyl, benzyl, or substituted phenyl or substituted benzyl in which the benzene ring thereof is substituted with chloro, bromo, iodo, nitro, $C_1$—$C_3$ alkyl, hydroxy, $C_1$—$C_3$ alkoxy, $C_1$—$C_3$ alkylthio, carbamyl, or $C_1$—$C_3$ alkylcarbamyl;

(c) 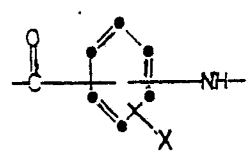

in which X is hydrogen, chloro, bromo, iodo, amino, nitro, $C_1$—$C_3$ alkyl, hydroxy, $C_1$—$C_3$ alkoxy, mercapto, $C_1$—$C_3$ alkylthio, carbamyl, or $C_1$—$C_3$ alkylcarbamyl;

(d) 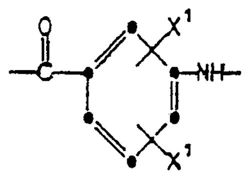 or 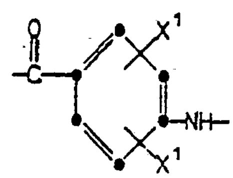

in which $X^1$ is chloro, bromo, iodo, amino, hydroxy, $C_1$—$C_3$-alkyl or $C_1$—$C_3$-alkoxy;

(e) 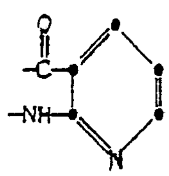 or 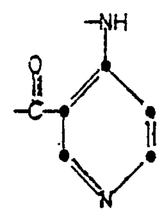 ; or

(f) 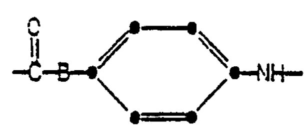

in which B is a divalent radical of the formula: $—CH_2)_n—$ and n is an integer from 1 to 3; —CH=CH—; $—CH=CH—CH_2—$; or

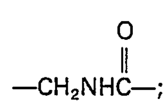

$R^7$ is $C_1$—$C_{17}$ alkyl or $C_2$—$C_{17}$ alkenyl; $R^1$, $R^3$, $R^4$ and $R^5$ are hydrogen and $R^2$ is hydrogen, an amino-protecting group, an aminoacyl group of the formula

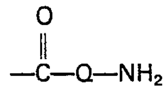

as herein defined, or an N-alkanoylaminoacyl group of the formula

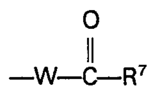

as herein defined; provided that when R is other than hydrogen, aminoacyl, an amino-protecting group or N-alkanoylaminoacyl, $R^2$ must be aminoacyl, N-alkanoylaminoacyl or an amino-protecting group; or a pharmaceutically-acceptable salt thereof.

The terms "alkylene", "alkyl", "alkoxy", "alkylthio", and "alkenyl" as used here comprehend both straight and branched hydrocarbon chains. "Alkyl" means a univalent saturated hydrocarbon radical. "Alkenyl" means a univalent unsaturated hydrocarbon radical containing one, two, or three double bonds, each oriented in the *cis* or *trans* configuration. "Alkylene" means a divalent saturated hydrocarbon radical. "Cycloalkylene" means a divalent cyclic saturated hydrocarbon radical.

Illustrative $C_1$—$C_{10}$ or $C_1$—$C_{16}$ alkylene radicals which are preferred are:

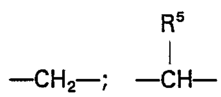

in which $R^5$ is $C_1$—$C_4$ alkyl (i.e. methyl, ethyl, *n*-propyl, *i*-propyl, *n*-butyl, *t*-butyl, *i*-butyl, or 1-methylpropyl); —$(CH_2)_m$— in which m is an integer from 2 to 10; and $CH_3$—$(CHs_2)_q$—CH—$(CH_2)_p$—, in which p is an integer from 1 to 8 and q is an integer from 0 to 7, provided that p + q must be no greater than 8.

For this aspect of the invention, illustrative $C_1$—$C_{17}$ alkyl groups which are preferred are:

(a) $CH_3$—;

(b) —$(CH_2)_nCH_3$ in which n is an integer from 1 to 16; and

(c) 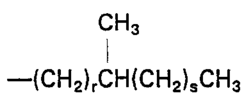

in which r and s are, independently, an integer from 0 to 14 provided that r + s can be no greater than 14.

Illustrative $C_2$—$C_{17}$ alkenyl radicals which are preferred are:

(a) —$(CH_2)_t$—CH=CH—$(CH_2)_u$—$CH_3$ in which t and u are, independently, an integer from 0 to 14 provided that t + u can be no greater than 14.

(b) —$(CH_2)_v$—CH=CH—$(CH_2)_y$—CH=CH—$(CH_2)_z$—$CH_3$ in which v and z are, independently, an integer from 0 to 11 and y is an integer from 1 to 12 provided that v + y + z can be no greater than 12.

In particular, the following embodiments of the $C_1$—$C_{17}$ alkyl groups are preferred:

$CH_3$—
$CH_3(CH_2)_5$—
$CH_3(CH_2)_6$—
$CH_3(CH_2)_8$—
$CH_3(CH_2)_{10}$—
$CH_3(CH_2)_{12}$—
$CH_3(CH_2)_{14}$—
$CH_3(CH_2)_{16}$—

In particular, the following embodiments of the $C_2$—$C_{17}$ alkenyl groups are preferred:

*cis*-$CH_3(CH_2)_5CH=CH(CH_2)_7$—
*trans*-$CH_3(CH_2)_5CH=CH(CH_2)_7$—
*cis*-$CH_3(CH_2)_{10}CH=CH(CH_2)_4$—
*trans*-$CH_3(CH_2)_{10}CH=CH(CH_2)_4$—
*cis*-$CH_3(CH_2)_7CH=CH(CH_2)_7$—
*trans*-$CH_3(CH_2)_7CH=CH(CH_2)_7$—
*cis*-$CH_3(CH_2)_5CH=CH(CH_2)_9$—
*trans*-$CH_3(CH_2)_5CH=CH(CH_2)_9$—
*cis,cis*-$CH_3(CH_2)_4CH=CHCH_2CH=CH(CH_2)_7$—
*trans,trans*-$CH_3(CH_2)_4CH=CHCH_2CH=CH(CH_2)_7$—
*cis,cis,cis*-$CH_3CH_2CH=CHCH_2CH=CHCH_2CH=CH$—$(CH_2)_7$—.

When "W" is a divalent radical of the formula

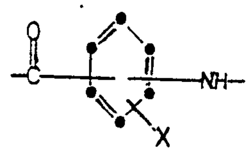

it will be recognized by those skilled in the art that the

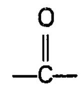

function and the —NH— function may be oriented on the benzene ring in the *ortho, meta,* or *para* configuration relative to each other. The substituent represented by X may be substituted at any available position of the benzene ring. Preferred embodiments are those in which X is hydrogen and the

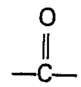

and —NH— functions are oriented in the *para* configuration.

The terms "substituted phenyl" and "substituted benzyl", as defined in connection with $R^8$, contemplate substitution of a group at any of the available positions in the benzene ring — i.e. the substituent may be in the *ortho, meta,* or *para* configuration. The term "$C_1$—$C_3$ alkyl" as defined in connection with $R^8$ or X includes the methyl, ethyl, *n*-propyl, or *i*-propyl groups.

Illustrative R and/or $R^2$ aminoacyl and N-alkanoylaminoacyl groups are provided in the Examples, *infra*. Other such illustrative R and/or $R^8$ groups are:

4-[N-(*n*-octanoyl)amino]cyclohexan-1-carbonyl,
7-[N-(*n*-heptanoyl)amino]-n-octanoyl,
α-hydroxymethyl-α-[N-[*n*-pentadecanoyl)amino]acetyl,
α-(*m*-methoxyphenyl)-α-[N-(*n*-heptanoyl)amino]acetyl,
*m*-chloro-*p*-[N-(*n*-nonanoyl)amino]benzoyl,
2,4-dihydroxy-5-[N-(*n*-decanoyl)amino]benzoyl,
4-[N-(3-methylbutanoyl)amino]nicotinoyl
4-[N-(*n*-heptadecanoyl)amino]phenylpropionyl and
p-[N-(n-hexadecanoyl)amino]hippuryl.

These compounds of formula 1 are prepared by acylating an A—21978C nucleus with the appropriate N-alkanoylaminoacyl or N-alkenoylaminoacyl side chain using methods conventional in the art for forming an amide bond. The acylation is accomplished, in general, by reacting a nucleus with an activated derivative of the acid (formula 5) corresponding to the desired acyl side chain group.

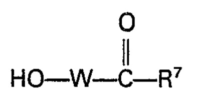 5

(W and $R^7$ have the meaning described herein *supra*).

In yet another aspect of this invention, other derivatives of structure 1 are those in which R is a substituted benzoyl group of the formula

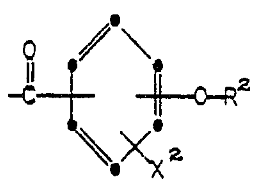

in which $X^2$ is hydrogen, chloro, bromo, iodo, nitro, $C_1$—$C_3$ alkyl, hydroxy, $C_1$—$C_3$ alkoxy, or $C_1$—$C_3$ alkylthio; $R^1$, $R^3$, $R^4$ and $R^5$ are hydrogen; $R^2$ is hydrogen or an amino-protecting group; and $R^6$ is $C_8$—$C_{15}$ alkyl; or a pharmaceutically-acceptable salt thereof.

The substituted benzoyl group, the

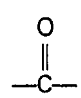

function and the $-OR^6$ function may be oriented on the benzene ring in the *ortho, meta,* or *para* position relative to each other. The *para* orientation for these groups is preferred. The substituent represented by $X^2$ may be substiuted at any available position of the benzene ring not occupied by these two groups.

The term "alkyl" as used in this aspect of the invention comprehends both straight and branched hydrocarbon chains.

Illustrative $C_8$—$C_{15}$ alkyl radicals which are preferred for $R^6$ are:

(a) $-(CH_2)_nCH_3$ in which n is an integer from 7 to 14; and

$$\begin{array}{l} \qquad\qquad\quad CH_3 \\ \qquad\qquad\quad | \\ \text{(b) } -(CH_2)_rCH(CH_2)_sCH_3 \end{array}$$

in which r and s are, independently, an integer from 0 to 12, provided that r + s can be no greater than 12 or no less than 5.

These compounds of formula 1 are prepared by acylating an A—21978C nucleus, using methods conventional in the art for forming an amide bond. The acylation is accomplished, in general, by reacting the selected compound with an activated derivative of the substituted benzoic acid (formula 6) corresponding to the desired acyl group (R, $R^1$, $R^2$):

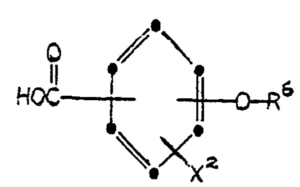

6

($X^2$ and $R^6$ have the meanings described *supra*).

Illustrative compounds of formula 6 include the following: *p*-(*n*-octyloxy)benzoic acid, *p*-(*n*-decyloxy)benzoic acid, *p*-(*n*-dodecyloxy)benzoic acid, *p*-(*n*-pentadecyloxy)benzoic acid, *m*-chloro-*p*-(n-dodecyloxy)benzoic acid, *p*-chloro-*m*-(*n*-decyloxy)benzoic acid, *m*-(*n*-dodecyloxy)-*p*-methylbenzoic acid, *m*-methoxy-*p*-(*n*-octyloxy)benzoic acid and *m*-hydroxy-*p*-(*n*-pentyloxy)benzoic acid.

"Activated derivative", as used throughout the specification, means a derivative which renders the carboxyl function of the acylating agent reactive to coupling with a primary amino group to form the amide bond which links the acyl side chain to the nucleus. Suitable activated derivatives, their methods of preparation, and their use as acylating agents for a primary amine will be recognized by those skilled in the art. Preferred activated derivatives are: (a) an acid halide (e.g. an acid chloride), (b) an acid anhydride (e.g. an alkoxyformic acid anhydride or aryloxyformic acid anhydride) or (c) an activated ester (e.g. a 2,4,5-trichlorophenyl ester, an N-hydroxy-benztriazole ester, or an N-hydroxysuccinimide ester). Other methods for activating the carboxyl function include reaction of the carboxylic acid with a carbonyldiimide (e.g. N,N'-dicyclohexylcarbodiimide or N,N'-diisopropylcarbodiimide) to give a reactive intermediate which, because of instability, is not isolated. Such a reaction with the primary amine is carried out *in situ*.

Those skilled in the art will recognize that the compound of formula 1 are prepared using selective acylation procedures with the assistance of amino-protecting groups. For example, when a compound of formula 1 in which R, $R^1$ and $R^2$ are hydrogen is the starting material, acylation can occur at both the α-amino group of tryptophan and the δ-amino group of ornithine to give $N_{Trp}$, $N_{Orn}$-diacyl derivatives. To obtain derivatives monoacylated at the α-amino group of tryptophan, therfore, it is preferable to acylate a compound of formula 1 in which the δ-amino group of ornithine (the $R^2$ position) is blocked by an amino-protecting group. Such starting materials are preferably obtained by blocking the A—21978C factor at this position before it is deacylated. The aromatic amino group of kynurenine is the least reactive of the three free amino groups in the A—21978C nucleus. Acylation at kynurenine involves appropriate blocking of the amino groups of tryptophan and ornithine, but acylation at R or $R^2$ does not usually involve blocking the amino group of kynurenine.

Schemes I, II, and III outline general procedures for the preparation of the compounds of formula 1 in which one of R, $R^1$ or $R^2$ is alkanoyl, alkenoyl, or an amino-protecting group. In these Schemes the following symbols are used:

[*] = remainder of A—21978C
$N_T$ = α-amino group of tryptophan
$N_o$ = δ-amino group of ornithine
$N_K$ = aromatic amino group of kynurenine
R, $R^1$, $R^2$ = substituents as defined
$R_N$ = acyl group of natural factor
B, $B^1$ = amino-protecting groups
Acyl = an acylation step
Deacyl = a deacylation step
Block = acylation with an amino-protecting group
Deblock = removal of an amino-protecting group

In Scheme I the $N_{Trp}$-monoacyl derivatives of A—21978C are represented by general formula 3 and the $N_{Trp}$, $N_{Orn}$-diacyl derivatives of A—21978C are represented by general formula 4. Those $N_{Trp}$, $N_{Orn}$-diacyl derivatives in which the $N_{Trp}$-acyl group is that of a natural A—21978C factor are represented by general formula 8.

Scheme I: Preparation of $N_{Trp}$-Monoacyl and $N_{Trp}$, $N_{Orn}$-Diacyl-A21978C Derivatives

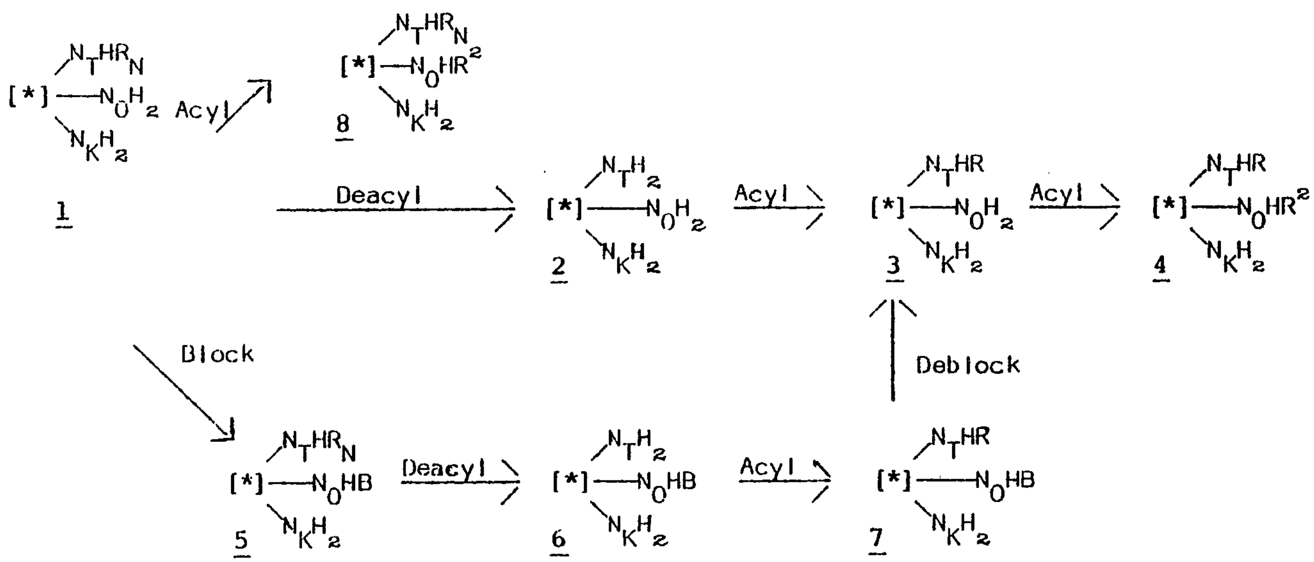


1 8 2 3 4 5 6 7

In Scheme II the $N_{Trp}$, $N_{Kyn}$-diacyl derivatives of A—21978C are represented by general formula 10. Those $N_{Trp}$, $N_{Kyn}$-diacyl derivatives in which the $N_{Trp}$-acyl group is that of a natural A—21978C factor are represented by general formula 12. The compounds having general formulas 5, 6 and 7 are also described in Scheme I.

Scheme II: Preparation of $N_{Trp}$, $N_{Kyn}$-Diacyl-A—21978C Derivatives

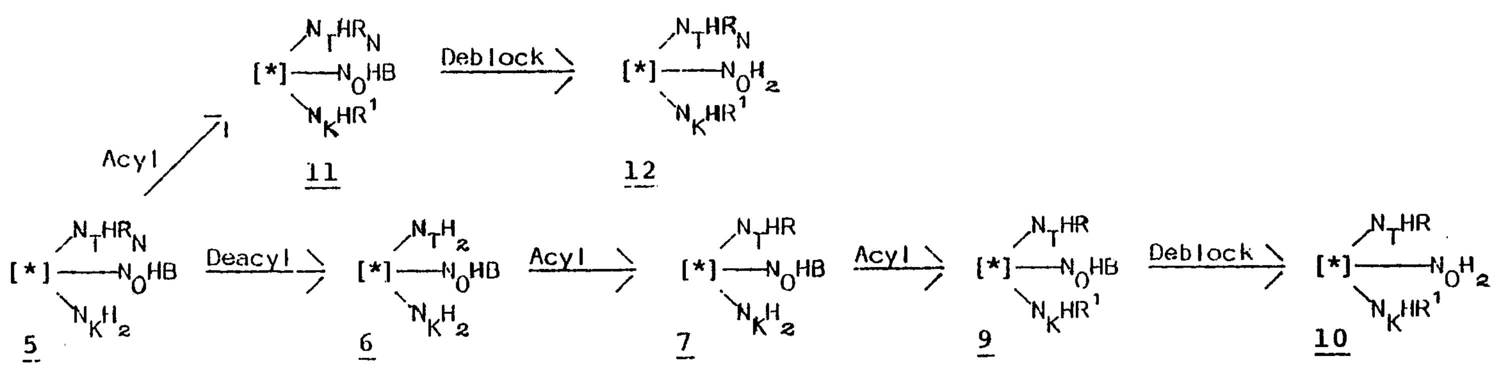


5 6 7 9 10 11 12

In Scheme III the $N_{Orn}$-monoacyl derivatives of A—21978C are represented by general formula 18, the $N_{Kyn}$-monoacyl derivatives of A—21978C are represented by general formula 15, and the $N_{Orn}$, $N_{Kyn}$-diacyl derivatives of A—21978C are represented by general formula 20. The compounds having general formula 6 are described also in Schemes I and II.

Scheme III: Preparation of $N_{Orn}$-Monoacyl, $N_{Kyn}$-monoacyl and $N_{Orn}$, $N_{Kyn}$-Diacyl- A—21978C Derivatives

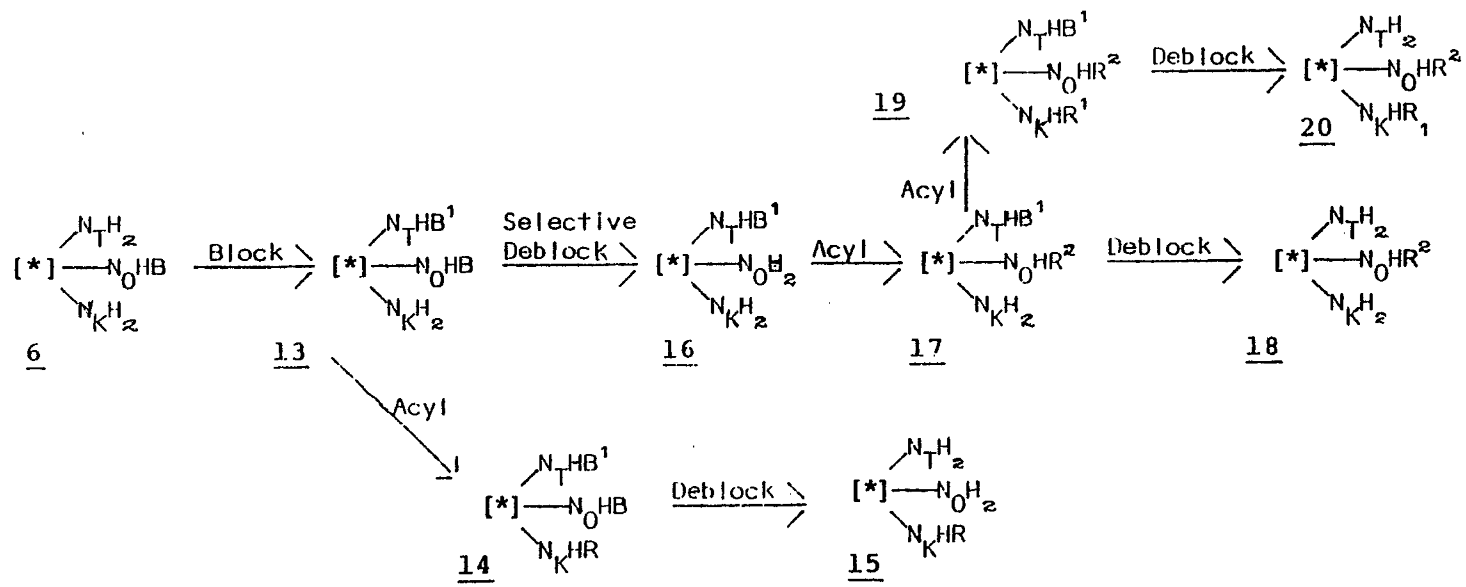


6 13 14 15 16 17 18 19 20

A preferred method for preparing the compounds of formula 1 is the active ester method. The 2,4,5-trichlorophenyl ester of the desired acid is a preferred acylating agent. In this method, an excess amount of the active ester is reacted with a formula 1 compound at room temperature in a non-reactive organic solvent such as DMF, THF, diethyl ether or dichloromethane. The reaction time is not critical, although a time of about 6 to about 120 hours is preferred. At the conclusion of the reaction, the solvent is removed, and the residue is purified by a recognized method, such as by column chromatography. t-BOC groups can be removed by treatment with trifluoroacetic acid/anisole/triethylsilane or, preferably trifluoroacetic acid/1,2-ethanedithiol for from about three to about five minutes at room temperature. After the solvent is removed, the residue can be purified by reversed-phase HPLC.

The 2,4,5-trichlorophenyl esters of the corresponding acids can be prepared conveniently by treating the desired acid with 2,4,5-trichlorophenol in the presence of a coupling agent, such as N,N'-dicyclohexylcarbodiimide. Other methods suitable for preparing acid esters will be apparent to those skilled in the art.

The alkanoic and alkenoic acids used in one aspect of this invention as starting materials and the activated derivatives thereof (in particular, the acid chlorides and the 2,4,5-trichlorophenyl esters), are known compounds or can be prepared from known compounds by known methods. The 2,4,5-trichlorophenyl esters are made conveniently by treating the acid chloride of the alkanoic or alkenoic acid with 2,4,5-trichlorophenol in the presence of pyridine or by treating the free alkanoic or alkenoic acid with 2,4,5-trichlorophenol in the presence of N,N'-dicyclohexylcarbodiimide. The 2,4,5-trichlorophenyl ester derivative can be purified by column chromatography over silica gel.

An alternative acylation method for preparing the alkanoyl and alkenoyl derivatives is a modified Schotten-Baumann procedure in which the unblocked nucleus is treated with the acid chloride of the desired alkanoic acid or alkenoic acid in a pyridine/water mixture. In this method, an excess of the acid chloride in a non-reactive organic solvent (such as acetone) is added slowly to a solution of the nucleus in 90% pyridine/10% water (by volume). The unreacted acid chloride is separated from the reaction product by extraction into an immiscible organic solvent (e.g., diethyl ether). Final purification is by reversed-phase HPLC, as previously described.

The N-alkanoylamino acids or N-alkenoylamino acids used as starting materials for derivatives of formula 1 are either known compounds or they can be made by acylating the appropriate amino acid with the desired alkanoyl or alkenoyl group using conventional methods. A preferred way of preparing the N-alkanoylamino acids is by treating the appropriate amino acid with an alkanoic acid chloride in pyridine. The alkanoic or alkenoic acids, the activated derivatives thereof, and the amino acids used are either known compounds or they can be made by known methods or by modification of known methods apparent to those skilled in the art.

If a particular amino acid contains an acylable functional group other than the amino group, it will be undertstood by those skilled in the art that such a group must be protected prior to reaction of the amino acid with the reagent used to attach the N-alkanoyl or N-alkenoyl group. Suitable protecting groups can be any group known in the art to be useful for the protection of a side chain functional group in peptide synthesis. Such groups are well known, and the selection of a particular protecting group and its method of use will be readily known to one skilled in the art [see, for example, "Protective Groups In Organic Chemistry", M. McOmie, Editor, Plenum Press, N.Y., 1973].

Certain amino acids used in the synthesis of these product may exist in optically active forms. Both the natural configuration (L-configuration) and D-configuration may be used as starting materials.

The substituted benzoic acids used as starting materials for some of the A—21978C derivatives, and the activated derivatives thereof are either known compounds or they can be made from known compounds by methods known in the art. The alkoxybenzoic acids can be prepared conveniently from an appropriate hydroxybenzoic acid by reacting an appropriate alkyl halide with the disodium salt of the appropriate hydroxybenzoic acid.

The hydroxybenzoic acids and substituted derivatives thereof used as starting materials in the processes described are either known compounds or can be prepared by conventional methods.

The compounds prepared from an A—21978C nucleus inhibit the growth of pathogenic bacteria as evidenced by standard biological test procedures. The compounds are useful, therefore, for controlling the growth of bacteria on environmental surfaces (as an antiseptic) or in treating infections caused by bacteria. The antibiotic activity of the compounds has been demonstrated *in vitro* in agar-plate disc-diffusion tests and in agar-dilution tests and *in vivo* in tests in mice infected with *Staphylococcus aureus* and *Streptococcus pyogenes*. The compounds are particularly useful in treating infections caused by gram-positive organisms.

When an A—21978C cyclic peptide of this invention is used as an antibiotic, it may be administered either orally or parenterally. As will be appreciated by those skilled in the art, the A—21978C compound is commonly administered together with a pharmaceutically acceptable carrier or diluent.

The compounds can be administered intravenously or intramuscularly by injection in the form of a sterile aqueous solution of suspension to which may be added, if desired, various conventional pharmaceutically acceptable preserving, buffering, solubilizing, or suspending agents. Other additives, such as saline or glucose may be added to make the solutions isotonic. The proportions and nature of such additives will be apparent to those skilled in the art.

For oral use, the compounds can be administered in combination with pharmaceutically acceptable

carriers or excipients in the form of capsules, tablets or powders. The nature and proportion of such carriers or excipients will be recognized by those skilled in the art.

The dosage of A—21978C compound will depend upon a variety of considerations, such as, for example, the particular compounds being used and the nature and severity of the infection to be treated. Those skilled in the art will recognize that appropriate dosage ranges or dosage units for administration may be determined by considering the MIC and $ED_{50}$ values and toxicity data provided together with factors such as pharmacokinetics, characteristics of the patient or host and the infecting microorganism.

In order to illustrate more fully the invention, the following non-limiting examples are provided.

Example 1
Preparation of A—21978C Nucleus

A. Fermentation of *Actinoplanes utahensis*

A stock culture of *Actinoplanes utahensis* NRRL 12052 is prepared and maintained on an agar slant. The medium used to prepare the slant is selected from one of the following:

MEDIUM A

| Ingredient | Amount |
|---|---|
| Pre-cooked oatmeal | 60.0 g |
| Yeast | 2.5 g |
| $K_2HPO_4$ | 1.0 g |
| Czapek's mineral stock* | 5.0 ml |
| Agar | 25.0 g |
| Deionized water | q.s. to 1 liter |

pH before autoclaving is about 5.9; adjust to pH 7.2 by addition of NaOH; after autoclaving, pH is about 6.7.

*Czapek's mineral stock has the following composition:

| Ingredient | Amount |
|---|---|
| $FeSO_4 \cdot 7H_2O$ (dissolved in 2 ml conc HCl) | 2 g |
| KCl | 100 g |
| $MgSO_4 \cdot 7H_2O$ | 100 g |
| Deionized water | q.s. to 1 liter |

MEDIUM B

| Ingredient | Amount |
|---|---|
| Potato dextrin | 5.0 g |
| Yeast extract | 0.5 g |
| Enzymatic hydrolysate of casein* | 3.0 g |
| Beef extract | 0.5 g |
| Glucose | 12.5 g |
| Corn starch | 5:0 g |
| Meat peptone | 5.0 g |
| Blackstrap molasses | 2.5 g |
| $MgSO_4 \cdot 7H_2O$ | 0.25 g |
| $CaCO_3$ | 1.0 g |
| Czapek's mineral stock | 2.0 ml |
| Agar | 20.0 g |
| Deionized water | q.s. to 1 liter |

*N—Z-Amine A, Humko Sheffield Chemical, Lyndhurst, N.J.

The slant is inoculated with *Actinoplanes utahensis* NRRL 12052, and the inoculated slant is incubated at 30°C for about 8 to 10 days. About ½ of the slant growth is used to inoculate 50 ml of a vegetative medium having the following composition:

| Ingredient | Amount |
|---|---|
| Pre-cooked oatmeal | 20.0 g |
| Sucrose | 20.0 g |
| Yeast | 2.5 g |
| Distiller's Dried Grain* | 5.0 g |
| $K_2HPO_4$ | 1.0 g |
| Czapek's mineral stock | 5.0 ml |
| Deionized water | q.s. to 1 liter |

adjust to pH 7.4 with NaOH; after autoclaving, pH is about 6.8.

*National Distillers Products Co., 99 Park Ave., New York, N.Y.

The inoculated vegetative medium is incubated in a 250-ml wide-mouth Erlenmeyer flask at 30°C for about 72 hours on a shaker rotating through an arc 5 cm (two inches) in diameter at 250 RPM.

This incubated vegetative medium may be used directly to inoculate a second-stage vegetative medium. Alternatively and preferably, it can be stored for later use by maintaining the culture in the vapor phase of liquid nitrogen. The culture is prepared for such storage in multiple small vials as follows: In each vial is placed 2 ml of incubated vegetative medium and 2 ml of a glycerol-lactose solution [see W. A. Dailey and C. E. Higgens, "Preservation and Storage of Microorganisms in the Gas Phase of Liquid Nitrogen, *Cyrobiol 10*, 364—367 (1973) for details]. The prepared suspensions are stored in the vapor phase of liquid nitrogen.

A stored suspension (1 ml) thus prepared is used to inoculate 50 ml of a first-stage vegetative medium (having the composition earlier described). The inoculated first-stage vegetative medium is incubated as above-described.

In order to provide a larger volume of inoculum, 10 ml of the incubated first-stage vegetative medium is used to inoculate 400 ml of a second-stage vegetative medium having the same compositions as the first-stage vegetative medium. The second-stage medium is incubated in a two-liter wide-mouth Erlenmeyer flask at 30°C for about 48 hours on a shaker rotating through an arc 5 cm (two inches) in diameter at 250 RPM.

Incubated second-stage vegetative medium (80 ml), prepared as above-described, is used to inoculate 10 liters of sterile production medium selected from one of the following:

MEDIUM I

| Ingredient | Amount (g/L) |
|---|---|
| Peanut meal | 10.0 |
| Soluble meat peptone | 5.0 |
| Sucrose | 20.0 |
| $KH_2PO_4$ | 0.5 |
| $K_2HPO_4$ | 1.2 |
| $MgSO_4 \cdot 7H_2O$ | 0.25 |
| Tap water | q.s. to 1 liter |

The pH of the medium is about 6.9 after sterilization by autoclaving at 121°C for 45 minutes at about (110—124) × 103 Pa (16—18 psi).

MEDIUM II

| Ingredient | Amount (g/L) |
|---|---|
| Sucrose | 30.0 |
| Peptone | 5.0 |
| $K_2HPO_4$ | 1.0 |
| KCl | 0.5 |
| $MgSO_4 \cdot 7H_2O$ | 0.5 |
| $FeSO_4 \cdot 7H_2O$ | 0.002 |
| Deionized water | q.s. to 1 liter |

Adjust to pH 7.0 with HCl; after autoclaving, pH is about 7.0.

MEDIUM III

| Ingredient | Amount (g/L) |
|---|---|
| Glucose | 20.0 |
| $NH_4Cl$ | 3.0 |
| $Na_2SO_4$ | 2.0 |
| $ZnCl_2$ | 0.019 |
| $MgCl_2 \cdot 6H_2O$ | 0.304 |
| $FeCl_3 \cdot 6H_2O$ | 0.062 |
| $MnCl_2 \cdot 4H_2O$ | 0.035 |
| $CuCl_2 \cdot 2H_2O$ | 0.005 |
| $CaCO_3$ | 6.0 |
| $KH_2PO_4$* | 0.67 |
| Tap water | q.s. to 1 liter |

* Sterilized separately and added aseptically

Final pH about 6.6.

The inoculated production medium is allowed to ferment in a 14-liter fermentation vessel at a temperature of about 30°C for about 66 hours. The fermentation medium is stirred with conventional agitators at about 600 RPM and aerated with sterile air to maintain the dissolved oxygen level about 30% of air saturated at atmospheric pressure.

B. Deacylation of A—21978C

A fermentation of *A. utahensis* is carried out as described in Section A, using slant medium A and production medium I and incubating the production medium for about 67 hours. Crude A—21978C complex, prepared as described in U.S. Patent No. 4,208,403, (100 g) is added to the fermentation medium.

Deacylation of the A—21978C complex is monitored by assay against *Micrococcus luteus.* The fermentation is allowed to continue until deacylation is complete as indicated by disappearance of activity vs. *M. luteus*, a period of about 24 hours.

This same procedure was used to determine whether other microorganisms would produce the desired A—21978C nucleus. In particular, *Actinoplanes missouriensis* NRRL 12053, *Actinoplanes sp.* NRRL 8122, *Actinoplanes sp.* NRRL 12065, and *Streptosporangium roseum* var. *hollandensis* NRRL 12064, when used in place of *Actinoplanes utahensis* NRRL 12052 in the procedure above, were found to produce the desired nucleus. HPLC comparisons using authentic samples of the nucleus obtained from this procedure using *A. utahensis* as the deacylating enzyme confirmed that there other microorganisms produced the A—21978C nucleus.

C. Isolation of A—21978C Nucleus

Whole fermentation broth (20 liters), obtained as described in Section B, was filtered with a filter aid (Hyflo Super-Cel, Johns Manville Corp.). The mycelial cake was discarded. The filtrate thus obtained was passed through a column containing 1.5 liters of HP—20 resin (DIAION High Porous Polymer, HP-Series, Mitsubishi Chemical Industries Limited, Tokyo, Japan). The effluent thus obtained was discarded. The column was then washed with deionized water (10 L.) to remove residual filtered broth. This wash water was discarded. The column was then eluted with water:acetonitrile mixtures (10 L. each of 95:5, 9:1, and 4:1), collecting 1-liter fractions.

Elution was monitored by analytical HPLC, using silica gel/$C_{18}$ and a solvent system of water:—methanol (3:1) containing 0.1% ammonium acetate, detecting the nucleus with a UV monitor at 254 nm. Elution can be monitored also by paper chromatography, using an n-butane:pyridine:acetic acid:water (15:10:3:12) solvent system and detecting the compounds by UV fluorescence. In this system, the A—21978C factors have an $R_f$ of about 0.56 and A—21978C nucleus has an $R_f$ of about 0.32. Fractions containing the nucleus were combined, concentrated under vacuum to remove the acetonitrile and freeze-dried to give 40.6 g of semi-purified A—21978C nucleus.

D. Purification of A—21978C Nucleus

Semi-purified A—21978C nucleus (15 g), obtained as described in Section C, was dissolved in 75 ml of water:methanol:acetonitrile (82:10:8) containing 0.2% acetic acid and 0.8% pyridine. This solution was pumped onto a 4.7- × 192-cm column containing 3.33 L. of silica gel (Quantum LP—1)/$C_{18}$. The column was developed with the same solvent system. Fractions having a volume of 350 ml were collected. Separation was monitored at 280 nm with a UV monitor. Fractions containing the nucleus were combined, concentrated under vacuum to remove solvents and freeze-dried to give 5.2 g of purified A—21978C nucleus.

Example 2

Alternate Preparation of A—21978C Nucleus

A—21978C nucleus was prepared according to the method of Example 1 except for certain changes in Section B. The *A. utahensis* culture was incubated initially for about 48 hours; the substrate was semi-purified A—21978C complex (50 g); and incubation after addition of the substrate was about 16 hours. The broth filtrate was passed over a column containing 3.1 liters of HP—20 resin. The column was washed with 10 volumes of water and then was eluted with water:acetonitrile (95:5). Elution was monitored as in Example 1. After collecting 24 liters, the eluting solvent was changed to water:acetonitrile (9:1). Fractions containing the nucleus were eluted with this solvent. These fractions were combined, concentrated under vacuum to remove acetonitrile, and freeze-dried to give 24.3 g of semi-purified A—21978C nucleus.

This semi-purified A—21978C nucleus (24.3 g) was dissolved in water (400 ml). The solution was pumped onto a 4.7- × 192-cm steel column containing 3.33 liters of silica gel (Quantum LP—1)/$C_{18}$ prepared in water:methanol:acetonitrile (8:1:1) containing 0.2% acetic acid and 0.8% pyridine. The column was developed with the same solvent at a pressure of about $13.8 \times 10^6$ Pa (2000 psi), collecting 350 ml fractions. Elution was monitored by UV at 280 nm. Fractions containing the nucleus were combined, concentrated under vacuum to remove solvents, and freeze-dried to give 14 g of highly purified A—21978C nucleus.

Example 3

Preparation of $N_{Orn}$-t-BOC A—21978C Factors $C_2$ and $C_3$

A mixture of A—21978C factors $C_2$ and $C_3$ (10 g), prepared as described in U.S. Patent No. 4,208,403, was dissolved in water (50 ml) with sonication (200 mg/ml). The pH of the solution was adjusted from 4.05 to 9.5 with 5N NaOH (3.6 ml). Di-*tert*-butyl dicarbonate (3.0 ml) was added, and the reaction mixture was stirred at room temperature for 2 hours. The pH of the reaction was maintained at 9.5 by manual addition of 5N NaOH (6.5 ml added in 2 hours).

The reaction was monitored periodically by TLC on silica gel, using $CH_3CN:H_2O$ (7:3 and 8:2) solvent systems and detecting by UV.

After about 10 minutes the reaction solution became rapidly turbid, and base consumption increased. After 30 minutes, the rate of increase in turbidity and the rate of base consumption decreased, indicating that the reaction was complete. Nevertheless, the reaction was continued for an additional 90 minutes to insure completion. At the end of the two-hour reaction, the reaction material was lyophilized immediately to give 12.7 g of $N_{Orn}$-t-BOC-A—21978 factors $C_2$ and $C_3$.

Using similar procedures, two 10-g reactions and a 30-g reaction were run. In each of these the reaction time was only 40 minutes. The two 10-g experiments gave 11.9 and 12.1 g of product, respectively. The 30 g reaction gave 35.4 g of product.

Example 4

Preparation of A—21978C $N_{Orn}$-t-BOC Nucleus

A. Fermentation of *A. utahensis*

A fermentation of *A. utahensis* was carried out as described in Example 1, Section A, using slant medium A and production medium I and incubating the production medium for about 66 hours.

B. Deacylation of $N_{Orn}$-t-BOC Complex

The A—21978C $N_{Orn}$-t-BOC complex (1185 g of crude substrate which contained about 176 g of A—21978C complex) was added to the fermentation medium. Deacylation was carried out as described in Example 1, Section B. Deacylation was complete, as indicated by HPLC, after about 24 hours.

C. Isolation of A—21978C $N_{Orn}$-t-BOC Nucleus

Fermentation broth (100 L.), obtained as described in Section B, was filtered with a filter aid (Hyflo Super-cel). The filtrate was passed over a column containing 7.5 L. of HP—20 resin (DIAION); the column was washed with water (38 L.). Elution was monitored by silica gel/$C_{18}$ HPLC with UV detection at 254 nm. Some nucleus was eluted in the wash. Subsequent elution of nucleus was carried out with water:acetonitrile mixtures as follows: (95:5)—40 L.; (9:1)—40 L.; and (85:15)—100 L. Fractions containing the nucleus were combined, concentrated under vacuum to remove solvent, and freeze-dried to give 298.5 g of semi-purified A—21978C $N_{Orn}$-t-BOC nucleus.

D. Purification of A—21978C $N_{Orn}$-t-BOC Nucleus

Semi-purified A—21978C $N_{Orn}$-t-BOC nucleus (30 g), obtained as described in Section C, was dissolved in water:acetonitrile (9:1) containing 0.2% acetic acid and 0.8% pyridine (75 ml). This solution was applied to a 4.7 × 192-cm steel column containing 3.33 L. of silica gel (Quantum LP—1)/$C_{18}$ equilibrated in the same solvent system. The column was developed under pressure with water:acetonitrile:methanol (80:15:5) containing 0.2% acetic acid and 0.8% pyridine, collecting 350-ml fractions and detecting product by UV at 280 nm. Fractions containing the product were combined, concentrated under vacuum to remove solvent and freeze-dried to give 18.4 g of purified A—21978C $N_{Orn}$-t-BOC nucleus.

Example 5

Alternative Purification of A—21978C $N_{Orn}$-t-BOC Nucleus

Semi-purified A—21978C $N_{Orn}$-t-BOC nucleus (10.8 g), obtained as described in Example 4, Section C, was dissolved in water and applied to a column containing 80 ml of Amberlite IRA—68 (acetate cycle). The column was washed with water and, at a flow rate of 5 ml/min, was eluted sequentially with 0.05N acetic acid (1080 ml), 0.1N acetic acid (840 ml), and 0.2N acetic acid (3120 ml), collecting 120-ml fractions. The column was monitored with analytical HPLC over silica gel/$C_{18}$, using a system of water:acetonitrile:methanol (80:15:5) containing 0.2% ammonium acetate and detecting product with UV at 254 nm. Fractions containing the product were combined; the pH of the solution was adjusted to 5.8 with pyridine; the resulting solution was concentrated under vacuum to a volume of about 200 ml. Water was added to the concentrate, and the resulting solution was reconcentrated to remove pyridine. This concentrate was freeze-dried to give 3.46 g of purified A—21978C $N_{Orn}$-t-BOC nucleus.

Examples 6—21

The preparation of various alkanoyl and alkenoyl derivatives by acylation of the A—21978C nucleus or blocked derivatives thereof, representative of the preparation of the compounds of formula *1*, is shown in Table I, below. The derivatives in Table I are made either by the modified Schotten-Bauman reaction using an acid chloride as the acylating agent (Method A) or by the active ester method using the 2,4,5-trichlorophenyl ester as the acylating agent (Method B). The general procedures for carrying out the acylation reactions by Method A or Method B are set forth below:

*Method A* (Modified Schotten-Bauman Reaction)

This method involves reaction of a A—21978C nucleus with the alkanoic or alkenoic acid chloride that corresponds to the desired acyl side chain.

An A—21978C nucleus or blocked derivative thereof (1.95—2.16 g, 1.33—1.47 mmoles) or an A—21978C factor (409 mg, 0.25 mmole) is dissolved in 200 ml of pyridine/$H_2O$ (9:1). The acylating agent (18—20 mmoles excess acyl chloride dissolved in 15 ml acetone) is added dropwise over 1—3 hours, and the reaction is stirred at ambient temperature for an additional 2—3 hours. The reaction mixture is concentrated to remove the acetone. The aqueous phase which remains is diluted to a volume of about 200 ml with water. The pH of this solution is adjusted to pH 3.0 to 3.5 with glacial acetic acid. This solution is washed 8 times with equal volumes of diethyl ether and then is lyophilized.

The crude acylated derivative is purified by reversed-phase HPLC as follows: The sample, dissolved in water or the eluant system (about 4—6.5 ml), is injected onto a 82.5 × 1.6 cm (33 × $\frac{5}{8}$ inch) stainless-steel column, packed with $LP_1/C_{18}$ support. The column is eluted with a solvent system consisting of $H_2O$:MeOH:$CH_3CN$:pyridine:HOAC. Elution is performed at a pressure of about (10.3—13.8) × $10^6$ Pa (1500—2000 psi) with a flow rate of about 10—12 ml/min, using an LDC duplex pump (Milton-Roy). Effluent is monitored by UV detection, using an ISCO—UA—5 detector at 280 nm. The desired fractions are combined and evaporated to dryness *in vacuo* to yield the desired alkanoyl derivative. The purified product is analyzed by TLC using reversed-phase plates (Whatman $KC_{18}$) and a $H_2O$:MeOH:$CH_3CN$:pyridine:HOAc (45:15:40:2:2) solvent system. The plates are observed under UV light to detect the product. The products are analyzed also by UV (extinction coefficients at 220 nm and 260 nm) and by amino acid analysis. Purity is determined by analytical reversed-phase HPLC ($C_{18}$ Microbondapak, Waters Co.) with a $H_2O$:MeOH:$CH_3CN$:pyridine:HOAc solvent system, monitoring eluent with UV at 280 nm.

*Method B* (2,4,5-Trichlorophenyl Active Ester Method)

A solution of $N_{Orn}$-t-BOC A—21978C nucleus (1.0 g, 0.64 mmol), A—21978C nucleus (0.5—1.0 g, 0.34—0.68 mmole) or A—21978C, (946 mg, 0.58 mmole) and a 3.5 molar excess of trichlorophenyl-acyl active ester are dissolved in DMF (100 ml) and stirred at from about room temperature to about 50°C. for from about 6 to about 20 hours. The reaction mixture is concentrated to an oil *in vacuo*. The oil is triturated with 50 ml $Et_2O$:toluene (1:1) and washed with $Et_2O$. The mono-acylated and di-acylated A—21978C nucleus derivatives, acylated A—21978$C_1$, and acylated t-BOC A—21978C nucleus are purified as described in Method A.

The acylated t-BOC-A—21978C nucleus is deblocked using 50 mg/ml of trifluoroacetic acid:anisole:triethylsilane (10:1:1) at from about −10° to about −0°C. for 3—5 minutes. This reaction mixture is concentrated *in vacuo* to an oil that is triturated with two 20-ml volumes of $Et_2O$. The crude acyl product is purified by reversed-phase HPLC and analyzed as described in Method A.

The specific compounds in the examples summarized in Tables 1 through 23 which follow are compounds of formula 1:

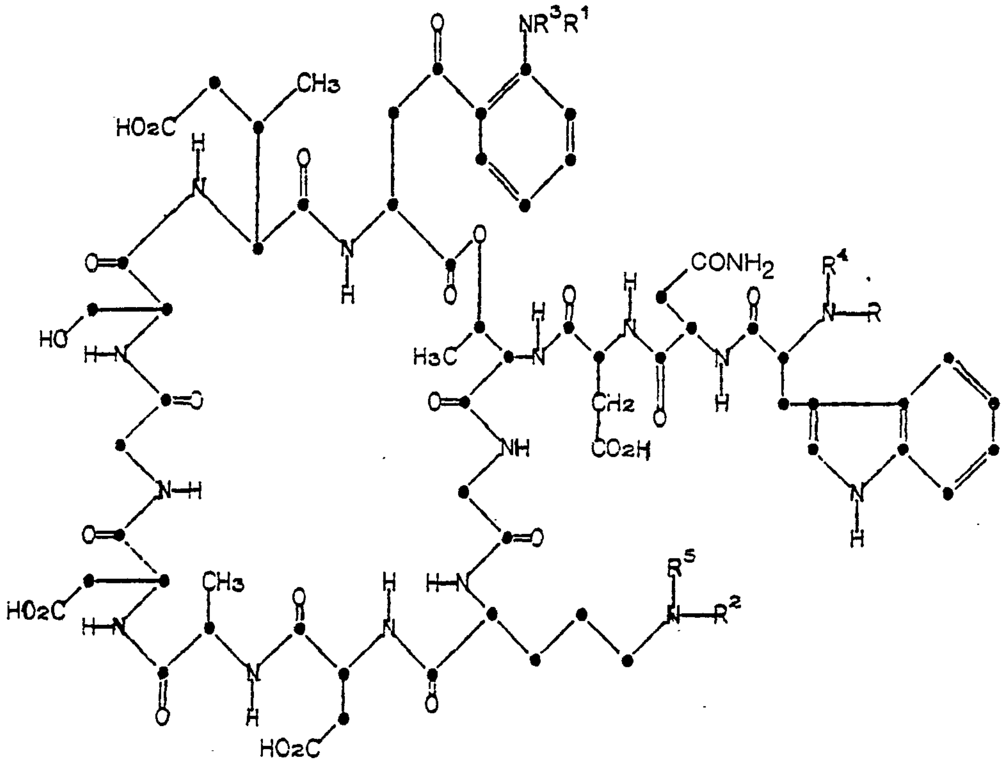

1

TABLE 1

Preparation of $N_{Trp}$-Monoacyl Derivatives of A-21978C Cyclic peptides

| Example No. | Compound[a] R | Method of Prep. | Nucleus wt (mg) | Acyl. Agent wt (mg) | Reaction Time (hr) | Intermediate[b] HPLC Eluent[c] | Product HPLC Eluent[c] | Product wt (mg) |
|---|---|---|---|---|---|---|---|---|
| 6 | $CH_3(CH_2)_5CO—$ | B | 1028 | 800 | 20 | 55:15:30 | 50:15:35 | 205 |
| 7 | $CH_3(CH_2)_6CO—$ | A | 1950 | 3421 | 3 | 50:15:35 | 55:15:30 | 355 |
| 8 | $CH_3(CH_2)_6CO—$ | B | 1000 | 350 | 24 | 50:15:35 | 115:15:64 | 264 |
| 9 | $CH_3(CH_2)_7CO—$ | A | 2000 | 882 | 2 | not purified | 50:15:35 | 212 |
| 10 | $CH_3(CH_2)_7CO—$ | B | 1000 | 400 | 20 | not purified | 50:15:35 | 240 |
| 11 | $CH_3(CH_2)_8CO—$ | A | 2160 | 4595 | 5 | | 45:15:40 | 334 |
| 12 | $CH_3(CH_2)_9CO—$ | B | 1000 | 288 | 22 | not purified | 45:15:40 | 392 |
| 13 | $CH_3(CH_2)_{10}CO—$ | A | 1000 | 3406 | 2 | | 45:15:40 | 370 |
| 14 | $CH_3(CH_2)_{11}CO—$ | B | 1000 | 800 | 24 | 10:15:75 | 40:15:45 | 313 |
| 15 | $CH_3(CH_2)_{12}CO—$ | B | 1000 | 812 | 22[d] | 50:15:35 | 1:0:1[e] | 267 |
| 16 | $CH_3(CH_2)_{12}CO—$ | B | 1000 | 900 | 20 | 50:15:35 | 50:15:35 | 215 |
| 17 | $CH_3(CH_2)_{13}CO—$ | B | 1000 | 1031 | 22[d] | not purified | 35:10:55[e] | 337.8 |
| 18 | $CH_3(CH_2)_{14}CO—$ | B | 1000 | 744 | 18[d] | not purified | 2:0:3[e] | 257 |
| 19 | $CH_2{=}CH—(CH_2)\delta CO—$ | B | 1000 | 237 | 20 | 20:15:65 | 45:15:40 | 290 |
| 20 | $CH_3(CH_2)_3CH{=}CH(CH_2)_7CO—$ | B | 1000 | 400 | 48 | not purified | 40:15:45 | 215 |
| 21 | $CH_3CH_2CH{=}CHCH_2CH{=}CH—CH_2CH{=}CH(CH_2)_7CO—$ | B | 1000 | 400 | 28 | 35:15:50 | 35:15:50 | 283 |

[a] $R^1$, $R^2$, $R^3$, $R^4$, $R^5$=H,
[b] $N_{Trp}$Acyl—$N_{Orn}$—tBOC,
[c] $H_2O:CH_3OH:CH_3CN$(v:v:v) containing 0.2% pyridine and 0.2% HOAc
[d] Reaction temperature maintained at 5°C.,
[e] Containing 1% pyridine and 1% HOAc

TABLE 2

Characteristics of $N_{Trp}$-Monoacyl Derivatives of A-21978C Cyclic Peptides

| Compound No. | $R^d$ | $R^1$ | $R^2$ | $R_f^a$ | Product: Anal. HPLC Eluent[b] | Product: UV $_\varepsilon\lambda max^{220nm}$ | Product: UV $_\varepsilon\lambda max^{260nm}$ |
|---|---|---|---|---|---|---|---|
| 1 | $CH_3(CH_2)_5CO—$ | H | H | 0.85 | 60:15:25 | 48,100 | 11,400 |
| 2 | $CH_3(CH_2)_6CO—$ | H | H | 0.80 | 60:15:25 | 46,900 | 10,500 |
| 3 | $CH_3(CH_2)_7CO—$ | H | H | 0.75 | 45:15:40 | 46,000 | 11,000 |
| 4 | $CH_3(CH_2)_8CO—$ | H | H | 0.70 | 45:15:40 | 46,500 | 10,000 |
| 5 | $CH_3(CH_2)_9CO—$ | H | H | 0.65 | 45:15:40 | 46,200 | 10,400 |
| 6 | $CH_3(CH_2)_{10}CO—$ | H | H | 0.59 | 45:15:40 | 44,000 | 9,500 |
| 7 | $CH_3(CH_2)_{11}CO—$ | H | H | 0.53 | 45:15:40 | 48,500 | 9,800 |
| 8 | $CH_3(CH_2)_{12}CO—$ | H | H | 0.42 | 50:0:49[c] | 48,447 | 9,170 |
| 9 | $CH_3(CH_2)_{13}CO—$ | H | H | 0.34 | 1:0:1[c] | 50,172 | 9,751 |
| 10 | $CH_3(CH_2)_{14}CO—$ | H | H | 0.25 | 39:0:60[c] | 54,000 | 12,000 |
| 11 | $CH_2{=}CH—(CH_2)_8CO—$ | H | H | 0.70 | 45:15:40 | 44,000 | 9,200 |
| 12 | $CH_3(CH_2)_3CH{=}CH(CH_2)_7CO—$ | H | H | 0.54 | 45:15:40 | 45,000 | 9,600 |
| 13 | $CH_3CH_2CH{=}CHCH_2CH{=}CH—$ $CH_2CH{=}CH(CH_2)_7CO—$ | H | H | 0.50 | 45:15:40[c] | 50,000 | 11,600 |

[a]$R^f$ by reversed-phase silica-gel TLC (Whatman $KC_{18}$ with fluorescent indicator) and $H_2O:CH_3OH:CH_3CN$(45:15:40) with 0.2% pyridine and 0.2% HOAc solvent system

[b]$H_2O:CH_3OH:CH_3N$ (v:v:v) containing 0.2% pyridine and 0.2% HOAc.

[c] Containing 1% pyridine and 1% HOAc

[d] $R^3$, $R^4$, $R^5$ = H

TABLE 3
Preparation of $N_{Trp}$-$N_{Orn}$-Diacyl A-21978C Derivatives

| Example No. | R[f] | R[1] | R[2] | Method of Prep. | Starting material wt (mg) | Acyl. Agent wt (mg) | Reaction Time (hr) | HPLC Eluent[a] | Product Wt (mg) |
|---|---|---|---|---|---|---|---|---|---|
| 22 | $CH_3CH_2CH(CH_3)(CH_2)_6CO$— | H | $CH_3CO$— | A | 409 | 2208 | 1.5 | 45:15:40 | 273 |
| 23 | $CH_3CH_2CH(CH_3)(CH_2)_6CO$— | H | $CH_3CO$— | B | 946 | 139 | 17 | 95:30:75 | 365 |
| 24 | $CH_3CH_2CH(CH_3)(CH_2)_6CO$— | H | $HOCOCH_2CH_2CO$— | C[b] | 500 | 62 | 20 | 45:15:40 | 201 |
| 25 | $CH_3(CH_2)_6CO$— | H | $CH_3(CH_2)_6CO$ | B | 500 | 530 | 18[c] | 35:24:40 | 60 |
| 26 | $CH_3(CH_2)_9CO$— | H | $CH_3(CH_2)_9CO$— | B | 1000 | 292 | 24 | 45:15:40 | 158 |
| 27 | $CH_3(CH_2)_9CO$— | H | $CH_3(CH_2)_9CO$— | B | 1000 | 1119 | 18[c] | 2: O:3[d] | 366 |
| 28 | $CH_3(CH_2)_{11}CO$— | H | $CH_3(CH_2)_{11}CO$— | B | 1000 | 1066.9 | 18[c] | | 245.9 |
| 29 | $CH_3CH_2CH(CH_3)(CH_2)_6CO$— | H | t—Boc | D[e] | | | | | |
| 30 | Cbz | H | $CH_3(CH_2)_{10}CO$— | | 1000 | 123 | 20 | | 550 |

[a] $H_2O:CH_3OH:CH_3N$ (v:v:v) containing 0.2% pyridine and 0.2 % HOAc
[b] Succinic anhydride, 90% pyridine
[c] Reaction temperature maintained at 5°C.
[d] Containing 1% pyridine and 1% HOAc.
[e] $(t\text{-}BOC)_2/H_2O$
[f] $R^3$, $R^4$, $R^5$ = H

TABLE 4

Characteristics of $N_{Trp}$, $N_{Orn}$-Diacyl A-21978C Derivatives

| Compound No. | $R^e$ | $R^1$ | $R^2$ | $R_f^a$ | Anal. HPLC Eluent[b] | UV $_\varepsilon\lambda max^{220nm}$ | UV $_\varepsilon\lambda max^{260nm}$ |
|---|---|---|---|---|---|---|---|
| 14 | $CH_3CH_2CH(CH_3)(CH_2)_6CO—$ | H | $CH_3CO—$ | 0.69 | 45:15:40 | 46,000 | 10,400 |
| 15 | $CH_3CH_2CH(CH_3)(CH_2)_6CO—$ | H | $HOCO(CH_2)_2CO—$ | 0.75 | 45:15:40 | 47,000 | 11,000 |
| 16 | $CH_3(CH_2)_6CO—$ | H | $CH_3(CH_2)_6CO—$ | 0.67 | 45:15:40[c] | 45,000 | 9,000 |
| 17 | $CH_3(CH_2)_9CO—$ | H | $CH_3(CH_2)_9CO—$ | 0.31 | 30:15:55<br>45:15:40[c] | 48,200 | 8,000 |
| 18 | $CH_3(CH_2)_{11}CO—$ | H | $CH_3(CH_2)_{11}CO—$ | 0.03 | 45:15:40[c] | 49,000 | 9,000 |
| 19 | $CH_3CH_2CH(CH_3)(CH_2)_6CO—$ | H | t-BOC | NT[d] | NT | NT | NT |
| 20 | Cbz | H | $CH_3(CH_2)_{10}CO—$ | 0.58 | NT | NT | NT |

[a]$R_f$ by reversed-phase silica-gel TLC (Whatman $KC_{18}$) with fluorescent indicator; solvent system $H_2O:CH_3OH:CH_3CN$ (45:15:40) with 0.2% pyridine and 0.2% HOAc

[b]$H_2O:CH_3OH:CH_3CN$ (v:v:v) containing 0.2% pyridine and 0.2% HOAc.

[c]Containing 1% pyridine and 1% HOAc

[d]NT = not tested

[e]$R^3$, $R^4$, $R^5$ = H

TABLE 5
Preparation of $N_{Trp}$, $N_{Kyn}$-Diacyl Derivative[a]

| Example No. | R,R[1] | $R^{2-5}$ | Method of Prep. | Starting Material wt (g) | Acyl Agent wt (g) | Reaction Time (hr) | tBOC Diacyl Intermediate HPLC Eluent[a] | Product | |
|---|---|---|---|---|---|---|---|---|---|
| | | | | | | | | HPLC Eluent[b] | wt (mg) |
| 31 | $CH_3(CH_2)_8CO$— | H | B | 15 | 15 | 30 | 50:15:35 | 50:15:35 | 211 |

[a] See Example 33, infra
[b] $H_2O:CH_3OH:CH_3CN$ (v:v:v) containing 0.2% pyridine and 0.2% HOAc

TABLE 6
Characteristics of $N_{Trp}$, $N_{Kyn}$-Diacyl Derivative

| Compound No. | R | R[1] | $R^{2-5}$ | $R_f$[a] | Anal. HPLC Eluent[b] | UV | |
|---|---|---|---|---|---|---|---|
| | | | | | | $\varepsilon\lambda max^{220}nm$ | $\varepsilon\lambda max^{260}nm$ |
| 21 | $CH_3(CH_2)_8CO$— | $CH_3(CH_2)_8CO$— | H | 0.66 | 61:15:23:1 | 41,800 | 11,500 |

[a] $R_f$ by reversed-phase silica-gel TLC (Whatman $KC_{18}$ with fluorescent indicator) and $H_2O:CH_3OH:CH_3CN$ (45:15:40) with 0.2% pyridine and 0.2% HOAc solvent system
[b] $H_2O:CH_3OH:CH_3CN:NH_4OAc$ containing 0.2% pyridine and 0.2 HOAc

Example 32

$N_{Trp}$-(*n*-Decanoyl) A-21978C Nucleus (Compound 4)

The following procedure illustrates the large-scale preparation of compounds by the active-ester method.

A. Preparation of 2,4,5-Trichlorophenyl *n*-decanoate

A solution of decanoyl chloride (Pfaltz and Bauer, 5.6 ml) and 2,4,5-trichlorophenol (5.6 g) in diethyl ether (1L) and pyridine (120 ml) is stirred for 4 hours. The reaction mixture is filtered and dried *in vacuo* . The 2,4,5-trichlorophenyl *n*-decanoate is purified on a silica-gel column (Woelm), using toluene as the eluent. Fractions are monitored by TLC, using short-wave UV for detection. Appropriate fractions are pooled and dried *in vacuo* to give 10.4 g of 2,4,5-trichlorophenyl *n*-decanoate.

B. Acylation of $N_{Orn}$-T-BOC-A-21978C Nucleus with 2,4,5-Trichlorophenyl *n*-decanoate

A solution of $N_{Orn}$-T-BOC-A-21978C nucleus (15.0 g) and 2,4,5-trichlorophenyl *n*-decanoate (15.0 g) in dry DMF (500 ml) is stirred under $N_2$ at ambient temperature for 25 hours. The mixture is then stirred at 60°C. for 5 hours or until TLC shows reaction completion. The reaction mixture is concentrated *in vacuo* to about 200 ml and is stirred with 1.2 litres of $Et_2O$/toluene (5:1). The product is separated by filtration, washed with $Et_2O$, and dried under vacuum to give 15.05 g of the $N_{Trp}$-(*n*-decanoyl)-$N_{Orn}$-t-BOC A-21978C nucleus intermediate (formula *1*; R = *n*-decanoyl, $R^1$ = H, $R^2$ = t-BOC).

C. Purification of $N_{Trp}$-(*n*-Decanoyl)-$N_{Orn}$-t-BOC-A-21978C Nucleus

The $H_{Trp}$-(*n*-decanoyl)-$N_{Orn}$-t-BOC-A-21978C nucleus intermediate is purified in the following manner: The crude preparation is dissolved in about 50 ml of the eluting solvent system, and then purified by HPLC, using the Water Prep/500 system containing a cartridge packed with reversed-phase $C_{18}$ silica-gel adsorbent. The system is eluted with $H_2O$:MeOH:$CH_3CN$ (50:15:35) containing 0.2% pyridine and 0.2% HOAc. Fractions are monitored by UV at 280 nm. Appropriate fractions ae combined and dried *in vacuo* to give 8.56 g of purified $N_{Trp}$-(*n*-decanoyl)-$N_{Orn}$-t-BOC-A-21978C nucleus.

D. Removal of the $N_{Orn}$-t-BOC Group

The t-BOC group is removed by stirring $N_{Trp}$-(*n*-decanoyl)-$N_{Orn}$-t-BOC A-21978C nucleus (1.47 g) in 15 ml of trifluoroacetic acid/1,2-ethanedithiol (10:1) at ambient temperature for 3 minutes. The reaction mixture is dried *in vacuo*, and the residue is triturated with $Et_2O$ (50 ml). After a 20 ml $Et_2O$ wash, the triturate is dried *in vacuo* to give 2.59 g of crude $N_{Trp}$-(*n*-decanoyl)-A-21978C nucleus (formula *1*: R= *n*-decanoyl; $R^1$ and $R^2$ = H).

E. Purification of $N_{Trp}$-(*n*-Decanoyl)-A-21978C Nucleus

The crude $N_{Trp}$-(*n*-decanoyl)-A-21978C nucleus is purified by reversed-phase HPLC in the following manner: The sample (2.59 g), dissolved in 4.0 ml of $H_2O$:MeOH:$CH_3CN$:pyridine:HOAC (50:15:35:2:2), is injected onto a 82.5 × 2.5 cm (33 × 1 inch) stainless-steel column packed with LP—1/$C_{18}$ adsorbent. The column is eluted with this same solvent system. Elution is performed at a pressure of 8.27—11.7 × $10^6$Pa (1200—1700 psi) with a flow rate of 10—12 ml/min, using an LDC duplex pump (Milton-Roy). The effluent is monitored by a UV detector (Isco Model UA—5, Instrument Specialist Co., 4700 Superior Avenue, Lincoln, NB 68504) at 280 nm. Fraction (20—24 ml) are collected every two minutes. The desired fractions, as indicated by antimicrobial activity, are combined and dried *in vacuo* to give 1.05 g of product.

This purification procedure was repeated with 4.35 g, 4.25 g, 2.14 g, 2.00 g and 1.75 g crude starting derivative to give a total of 5.58 g of purified $N_{Trp}$-(*n*-Decanoyl)-A-21978C nucleus.

Example 33

$N_{Trp}$-(*n*-Decanoyl)-$N_{Kyn}$-(*n*-decanoyl)-A-21978C (Compound *21*)

$N_{Trp}$-(*n*-Decanoyl)-$N_{Kyn}$-(*n*-decanoyl-A-21978C nucleus (formula *1*: R and $R^1$ = *n*-decanoyl; $R^2$ = H) is a minor reaction product in the preparation of the $N_{Trp}$-(*n*-decanoyl) derivative of Example 27. It is isolated during the reversed-phase HPLC purification described in Section E. Desired fractions are combined and dried *in vacuo* to give 211 mg of crude product. The compound is purified by analytical HPLC ($C_{18}$ Microbondpak, Waters Co.), using $H_2O$:MeOH:$CH_3CN$:$NH_4OH$:HOAc (6.23:15:0.5:0.5) as the eluent system (32 repetitions, 500 µg each injected sample) to give 4.4 mg of $N_{Trp}$-(*n*-decanoyl)-$N_{Kyn}$-(*n*--decanoyl)-A-21978C nucleus, identified by 360 MHz proton NMR.

Example 34

$N_{Trp}$-Cbz-$N_{Orn}$-Lauroyl A-21978C Nucleus (Compound 20)

This method involves protecting the tryptophan α-$NH_2$ of $N_{Orn}$-t-BOC-A-21978C nucleus with a Cbz group, then removing the t-BOC group and acylating (at the ornithine α-$NH_2$) with the trichlorophenyl-lauroyl ester.

A. Preparation of $N_{Trp}$-Cbz-$N_{Orn}$-t-BOC-A-21978C Nucleus

$N_{Orn}$-t-BOC-A-21978C nucleus (1.0 g) is dissolved in DMF (150 ml) and warmed to 60°C. N,O-bis-

trimethylsilylacetamide (0.55 ml) is added, and then benzyl pentachlorophenyl carbonate (257 mg) is added. After being stirred for 20 hours, the reaction mixture is concentrated to a volume of about 20-25 ml. Water (100 ml) is added, and the pH of this solution is adjusted to 6.0 with 1N NaOH. The mixture is washed with $Et_2O$ (6 times, 200-ml volumes) and the lyophilized.

The crude derivative is purified by reversed-phase HPLC as follows: the sample, dissolved in about 6 ml of $H_2O$:MeOH:$CH_3CN$:pyridine:HOAc (55:15:30:2:2), is injected onto a $82.5^3 \times 1.25$ cm ($33 \times 1/2$ inch) stainless-steel column packed with LP-1/$C_{18}$ support. The desired fractions are located by UV absorption (280 nm) and antimicrobial activity. These fractions are then combined and lyophilized to give 951 mg of the $N_{Trp}$-Cbz-$N_{Orn}$-t-BOC-A-21978C nucleus derivative.

B. Removal of t-BOC Group

The t-BOC group is removed by dissolving the intermediate at 50 mg/ml in trifluoroacetic acid:-anisole:triethylsilane (10:1:1) at −10°C. for 3 minutes. The reaction is concentrated to an oil that is triturated with two 25-ml volumes of $Et_2O$ to give 520 mg of crude $N_{Trp}$-Cbz-A-21988C nucleus

C. Acylation of $N_{Trp}$-Cbz-A-21978C Nucleus

The $N_{Trp}$-Cbz-A-21978C nucleus is acylated by the trichlorophenyl active-ester acylation method. $N_{Trp}$-Cbz-A-21978C (520 mg) is added to a solution of 1-hydroxybenzotriazole (7 mg) and lauroyltrichlorophenol active ester (123 mg) in pyridine (150 ml). After being stirred for 20 hours at 60°C., the reaction mixture is concentrated to a residue that is triturated with $Et_2O$ (3 times, 25-ml volumes) to give $N_{Trp}$-Cbz-$N_{Orn}$lauroyl-A-21978C nucleus (550 mg).

Example 35

Preparation of Schiff's Bases and Reduced Schiff's Bases

Several reactions were carried out as follows: A-21978C nucleus (40 mg) was dissolved in water (2 ml). The pH of the solution was adjusted from 4 to 9 with 1N NaOH. Aliquots (0.5 ml) of this solution were then mixed with each of the following aldehydes (5 μl):

1) heptaldehyde
2) octyl aldehyde
3) decyl aldehyde
4) undecyl aldehyde

in methanol (4 ml). The reactions were stirred overnight at room temperature. The Schiff's bases which formed were reduced with $NaBH_3CN$ (2.5 mg per reaction) for 5 minutes at room temperature.

The reactions were examined by silica-gel TLC, using a $CH_3CN$:$H_2O$ (7:3) solvent system, and by assay against *Staphylococcus aureus.* The Schiff's bases were not active against *S. aureus* in this test, perhaps due to their instability under the assay conditions. In each reduced reaction two factors were produced. These compounds were active against *S. aureus* and had a similar mobility in the TLC system, giving evidence that the following compounds of the formula *1* were produced (in each case $R^1$ is hydrogen):

Preparation of Schiff's Bases and Reduced Schiff's Bases

| Reaction of A21978C with | Product $R^b$ | $R^2$ | Active vs *S. aureus*[a] |
|---|---|---|---|
| heptaldehyde | $CH_3(CH_2)_5CH=$ | H | no |
| | $CH_3(CH_2)_6—$ | H | yes |
| | $CH_3(CH_2)_5CH=$ | $CH_3(CH_2)_5CH=$ | no |
| | $CH_3(CH_2)_6—$ | $CH_3(CH_2)_6—$ | yes |
| octyl aldehyde | $CH_3(CH_2)_6CH=$ | H | no |
| | $CH_3(CH_2)_7—$ | H | yes |
| | $CH_3(CH_2)_6CH=$ | $CH_3(CH_2)_6CH=$ | no |
| | $CH_3(CH_2)_7—$ | $CH_3(CH_2)_7—$ | yes |
| decyl aldehyde | $CH_3(CH_2)_8CH=$ | H | no |
| | $CH_3(CH_2)_9—$ | H | yes |
| | $CH_3(CH_2)_8CH=$ | $CH_3(CH_2)_8CH=$ | no |
| | $CH_3(CH_2)_9—$ | $CH_3(CH_2)_9—$ | yes |
| undecyl aldehyde | $CH_3(CH_2)_9CH=$ | H | no |
| | $CH_3(CH_2)_{10}—$ | H | yes |
| | $CH_3(CH_2)_9CH=$ | $CH_3(CH_2)_9CH=$ | no |
| | $CH_3(CH_2)_{10}—$ | $CH_3(CH_2)_{10}—$ | yes |

[a]The lack of activity of the Schiff's bases in this test may reflect their instability under the test conditions.
[b]where R or $R^2$ is alkyl, then $R^3$, $R^4$ or $R^5$ = H.

Example 36

Preparation of $N_{Trp}$-Lauraldehyde Schiff's Base and $N_{Trp}$, $N_{Orn}$-Di-Lauraldehyde Schiff's Base and Their Reduced Schiff's Base and Their Reduced Schiff's Bases (Compounds *22* and *23*)

A-21978C nucleus (1 g) was dissolved in water (50 ml); a solution of dodecyl aldehyde (500 µl) in methanol (200 ml) was added. The reaction mixture was stirred overnight at room temperature. The reaction then was reduced for 50 minutes by the addition of $NaBH_3CN$ (291 mg). The reaction mixture was filtered under vacuum, using Whatman No. 1 paper to remove particulates. The supernatant was concentrated to an aqueous solution which was lyophilized to give 1.256 g of product. This product was evaluated by analytical HPLC and purified by preparative reverse-phase HPLC in portions. Each portion (350 mg) was dissolved in 50% aqueous methanol (6 ml), sonicating and heating to dissolve the material. The solution then was passed over a 1.5 — × 80-cm $LP_1$—$C_{18}$ column, eluting with $CH_3OH$:$CH_3CN$:$H_2O$ (25:40:35) containing 0.2% pyridine and 0.2% acetic acid at a flow rate of about 7.5 ml/min. Elution was monitored by UV at 280 nm. Fractions containing the desired products were combined to give a total of 104 mg of $N_{Trp}$-(*n*-dodecyl)-A-21978C nucleus (Compound *22*) and 19.2 mg of $N_{Trp}$-(*n*-dodecyl)-$N_{Orn}$-(*n*-dodecyl)-A-21978C nucleus (Compound *23*).

Example 37

The antibacterial activity of the compounds of formula *1* can be demonstrated *in vitro.* The results of the antibacterial testing of representative compounds of formula *1* using standards agar-plate disc-diffusion tests are set forth in Table 7. In Table 7 activity is measured by the size (diameter in mm) of the observed zone in which growth of the microorganism is inhibited by the test compound.

TABLE 7
Antibacterial Activity of Formula 1 compounds by the Agar-Plate Disc-Diffusion Test

| Compound No. | $R^d$ | $R^1$ | $R^2$ | Size of Zone of Inhibition (mm)[a] *Staphylococcus aureus* ATCC 6738P | *Bacillus subtilis* ATCC 6633 | *Micrococcus luteus* ATCC 9341 | *B. subtilis* ATCC 6633[b] |
|---|---|---|---|---|---|---|---|
| 1 | $CH_3(CH_2)_5CO—$ | H | H | 17 | 15 | 21 | 16 |
| 2 | $CH_3(CH_2)_6CO—$ | H | H | 23 | 18 | 23 | 20 |
| 3 | $CH_3(CH_2)_7CO—$ | H | H | 20 | 16 | 18 | 27 |
| 4 | $CH_3(CH_2)_8CO—$ | H | H | 24 | 20 | 22 | 29 |
| 5 | $CH_3(CH_2)_9CO—$ | H | H | 19 | 19 | 21 | 21 |
| 6 | $CH_3(CH_2)_{10}CO—$ | H | H | 25 | 20 | 19 | 32 |
| 7 | $CH_3(CH_2)_{11}CO—$ | H | H | 21 | 17 | 19 | 29 |
| 8 | $CH_3(CH_2)_{12}CO—$ | H | H | 22 | 21 | 21 | 28 |
| 9 | $CH_3(CH_2)_{13}CO—$ | H | H | 20 | 19 | 19 | 24 |
| 10 | $CH_3(CH_2)_{14}CO—$ | H | H | 19 | 17 | 17 | 23 |
| 11 | $CH_2{=}CH—(CH_2)_8CO—$ | H | H | 21 | 17 | 20 | 25 |
| 12 | $CH_3(CH_2)_3CH{=}CH(CH_2)_7CO—$ | H | H | 22 | 19 | 20 | 30 |
| 13 | $CH_3CH_2CH{=}CHCH_2CH{=}CH—CH_2CH{=}CH(CH_2)_7CO—$ | H | H | 15 | 13 | 20 | 14 |
| 14 | $CH_3CH_2CH(CH_3)(CH_2)_6CO—$ | H | $CH_3CO—$ | 21 | 18 | 20 | 25 |
| 15 | $CH_3CH_2CH(CH_3)(CH_2)_6CO—$ | H | $HOCO(CH_2)_2CO—$ | 21 | 18 | 19 | 25 |
| 16 | $CH_3(CH_2)_6CO—$ | H | $CH_3(CH_2)_6CO—$ | 11 | tr[c] | 10 | 17 |
| 17 | $CH_3(CH_2)_9CO—$ | H | $CH_3(CH_2)_9CO—$ | 17 | 13 | 14 | 20 |

TABLE 7 (continued)
Antibacterial Activity of Formula 1 compounds by the Agar-Plate Disc-Diffusion Test

| Compound No. | $R^d$ | $R^1$ | $R^2$ | Size of Zone of Inhibition (mm)[a] Staphylococcus aureus ATCC 6738P | Bacillus subtilis ATCC 6633 | Micrococcus luteus ATCC 9341 | B. subtilis ATCC 6633[b] |
|---|---|---|---|---|---|---|---|
| 18 | $CH_3(CH_2)_{11}CO$— | H | $CH_3(CH_2)_{11}CO$— | 12 | tr | — | 13 |
| 19 | $CH_3CH_2CH(CH_3)(CH_2)_6CO$— | H | t-BOC | 14 | 11 | 10 | 22 |
| 20 | Cbz | H | $CH_3(CH_2)_{10}CO$— | 15 | 10 | 17 | 23 |

[a]Compounds were suspended in water at a concentration of 1 mg/ml; a 7-mm disc was dipped into the suspension and then placed on the agar surface; incubation: 24—48 hours at 25—37°C.
[b]Grown on minimal nutrient agar
[c]tr=trace
[d]$R^3$, $R^4$, $R^5$=H

The results of antibacterial testing of representative compounds of formula *1* by standard agar-dilution tests are summarized in Table 8. In Table 8 activity is measured by the minimal inhibitory concentration (MIC), i.e. the lowest concentration of compound at which growth of the microorganism is inhibited by the test compound.

TABLE 8
Antibiotic Activity of A—21978C Cyclic Peptides

| Test Organism | MIC Values of Test Compounds | | | | | | | | | | |
|---|---|---|---|---|---|---|---|---|---|---|---|
| | 1 | 2[d] | 3 | 4[e] | 5[d] | 6[e] | 7 | 8 | 9 | 10 | 11 |
| *Staphylococcus aureus* X1.1 | 8 | 4 | 2 | 1,0.5 | 0.25 | 0.5,0.5 | 0.125 | 0.5,1 | 1 | 1 | 0.5 |
| *Staphylococcus aureus* V41[b] | 8 | 4 | 2 | 1,0.5 | 0.25 | 0.5,1 | 0.125 | 0.5,2 | 1 | 1 | 0.5 |
| *Staphylococcus aureus* X400[c] | 8 | 8 | 4 | 2,1 | 0.5 | 1,1 | 0.5 | 1,2 | 2 | 2 | 0.5 |
| *Staphylococcus aureus* S13E | 8 | 4 | 2 | 1,0.5 | 0.5 | 0.5,0.5 | 0.25 | 1,2 | 1 | 1 | 0.25 |
| *Staphylococcus epidermidis* EPI1 | 16 | 8 | 4 | 2,1 | 0.5 | 1,1 | 0.5 | 1,4 | 2 | 2 | 1 |
| *Staphylococcus epidermis* EPI2 | 8 | 4 | 2 | 1,0.5 | 0.5 | 1,1 | 0.5 | 2,2 | 4 | 2 | 1 |
| *Streptococcus pyogenes* C203 | 2 | 1 | 0.25 | 0.25, 0.125 | 0.125 | 0.125, 0.25 | 0.25 | 0.5,2 | 1 | 1 | 0.5 |
| *Streptococcus pneumonia* Park I | 8 | 8 | 2 | 1,0.5 | 0.25 | 0.25, 0.25 | 0.25 | 0.03, 0.125 | 0.06 | 0.06 | 0.25 |
| *Streptococcus* Group D X66 | 128 | 64 | 32 | 16,16 | 2 | 2,4 | 0.03 | 0.5,2 | 2 | 2 | 1 |
| *Streptococcus* Group 9960 | 128 | 16 | 8 | 2,2 | 0.5 | 0.5,0.5 | 32 | 0.125, >128 | 0.25 | 0.25 | 0.5 |

[a]MIC in mcg/ml; compound numbers from Tables II, IV and VI and Example 36
[b]Penicillin-resistant strain
[c]Methicillin-resistant-strain
[d]Median value from three tests
[e]Two experiments

TABLE 8 (continued)

| Test Organism | MIC Values of Test Compounds[a] | | | | | | | | | | |
|---|---|---|---|---|---|---|---|---|---|---|---|
| | 12 | 13 | 14[e] | 15[e] | 16 | 17[d] | 18 | 20 | 21 | 22[d] | 23 |
| *Staphylococcus aureus* X1.1 | 1 | >128 | 4,4 | 4,4 | 8 | 1 | 8 | 8 | 1 | 0.5 | 8 |
| *Staphylococcus aureus* V41[b] | 1 | >128 | 4,4 | 8,8 | 16 | 4 | 64 | 16 | 4 | 1 | 8 |
| *Staphylococcus aureus* X400[c] | 2 | >128 | 8,8 | 4,8 | 8 | 4 | >128 | 32 | 32 | 1 | 16 |
| *Staphylococcus aureus* S13E | 1 | >128 | 8,4 | 4,8 | 8 | 1 | >128 | 8 | 16 | 0.5 | 8 |
| *Staphylococcus epidermis* EPI1 | 2 | >128 | 8,8 | 8,8 | 16 | 8 | >128 | 32 | 2 | 1 | 8 |
| *Staphylococcus epidermis* EPI2 | 2 | >128 | 8,4 | 8,8 | 16 | 8 | >128 | 32 | 2 | 1 | 8 |
| *Streptococcus pyogenes* C203 | 0.5 | >128 | 1,1 | 1,1 | 4 | 0.25 | 1 | 4 | 0.125 | 0.5 | 4 |
| *Streptococcus pneumoniae* Park I | 0.5 | >128 | 2,2 | 2,4 | 4 | 0.125 | 0.5 | — | 0.5 | 1 | 8 |
| *Streptococcus* Group D X66 | 2 | >128 | 64,32 | 64,64 | >128 | 8 | >128 | 128 | >128 | 4 | 32 |
| *Streptococcus* Group 9960 | 1 | >128 | 32,8 | 32,32 | 64 | 8 | >128 | 64 | 128 | 1 | 8 |

The A—21978C cyclic peptides of formula *1* have shown *in vivo* antimicrobial activity against experimental bacterial infections. When two doses of test compound were administered subcutaneously or orally to mice in illustrative infections, the activity observed was measured as an $ED_{50}$ value [effective dose in mg/kg to protect fifty percent of the test animals: See Warren Wick, et al., *J. Bacteriol. 81,* 233—235 (1961)]. The $ED_{50}$ values observed for representative A—21978C compounds of formula *1* are given in Table 9.

TABLE 9

In Vivo Activity of A—21978C Cyclic Peptides

| Compound No. | Formula 1 compound R[e] | R[1] | R[2] | ED₅₀ Values[a] *Staphylococcus aureus* Subcutaneous | *Streptococcus pyogenes* Subcutaneous | *Streptococcus pyogenes* Oral |
|---|---|---|---|---|---|---|
| 1 | $CH_3(CH_2)_5CO$— | H | H | 2.65 | 1.49,5.1[b] | NT[c] |
| 2 | $CH_3(CH_2)_6CO$— | H | H | 3.75,1.4 | <2.2, 0.65, 1.9[d] | >200 |
| 3 | $CH_3(CH_2)_7CO$— | H | H | 0.5 | 0.14, 0.243 | 92, 117 |
| 4 | $CH_3(CH_2)_8CO$— | H | H | 0.28 | 0.03 | 66 |
| 5 | $CH_3(CH_2)_9CO$— | H | H | 0.37 | 0.13 | 138 |
| 6 | $CH_3(CH_2)_{10}CO$— | H | H | 0.44 | 0.05 | 69 |
| 7 | $CH_3(CH_2)_{11}CO$— | H | H | 0.54 | 0.046 | 45 |
| 8 | $CH_3(CH_2)_{12}CO$— | H | H | 1.17, 3.08 | 0.98, 0.20, <0.54 | <50, <200 |
| 9 | $CH_3(CH_2)_{13}CO$— | H | H | 8.3 | <0.5, 0.18 | >200 |
| 10 | $CH_3(CH_2)_{14}CO$— | H | H | >1.1 | 0.18 | >200 |
| 11 | $CH_2$=CH—$(CH_9CO$— | H | H | 0.93 | 0.068 | 138 |
| 12 | $CH_3(CH_2)_3CH=CH(CH_2)_7CO$— | H | H | 3.28 | 0.134 | 75 |
| 14 | $CH_3CH_2CH(CH_3)(CH_2)_6CO$— | H | $CH_3CO$— | 1.3 | 2.2, 1.9 | 163, >200 |
| 15 | $CH_3CH_2CH(CH_3)(CH_2)_6CO$— | H | HOCO—$(CH_2)_2CO$— | 6.27 | 1.4 | >200 |
| 17 | $CH_3(CH_2)_9CO$— | H | $CH_3(CH_2)_9$—CO— | >35 | 2.85 | >200 |
| 22 | $CH_3(CH_2)_{11}$— | | H | 1.25 | 1.1, 0.85 | >200 |

[a]mg/kg × 2, [b]Two Experiments, [c]Not Tested, [d]Three experiments, [e]$R^3$, $R^4$, $R^5$ = H

The results of toxicity tests on some A—21978C cyclic peptides of formula *1* are summarized in Table 10.

TABLE 10

Toxicity of A—21978C Cyclic Peptides

Formula 1 Compound

| Compound No. | $R^c$ | $R^1$ | $R^2$ | $LD_{50}$ (mg/kg) in Mice[a] |
|---|---|---|---|---|
| 1 | $CH_3(CH_2)_5CO$— | H | H | >600 |
| 2 | $CH_3(CH_2)_6CO$— | H | H | >600 |
| 3 | $CH_3(CH_2)_7CO$— | H | H | >600 |
| 4 | $CH_3(CH_2)_8CO$— | H | H | >300 |
| 5 | $CH_3(CH_2)_9CO$— | H | H | >600 |
| 6 | $CH_3(CH_2)_{10}CO$— | H | H | 144, 265[b] |
| 7 | $CH_3(CH_2)_{11}CO$— | H | H | 112.5 |
| 8 | $CH_3(CH_2)_{12}CO$— | H | H | 62.5, <150 |
| 9 | $CH_3(CH_2)_{13}CO$— | H | H | 56.25 |
| 10 | $CH_3(CH_2)_{14}CO$— | H | H | 50 |
| 11 | $CH_2$=CH—$(CH_2)_8CO$— | H | H | ≥500 |
| 12 | $CH_3(CH_2)_3CH$=$CH(CH_2)_7CO$— | H | H | 450 |
| 14 | $CH_3CH_2CH(CH_3)(CH_2)_6CO$— | H | $CH_3CO$— | >600, 600 |
| 15 | $CH_3CH_2CH(CH_3)(CH_2)_6CO$— | H | $HOCO(CH_2)_2CO$— | 450, 600 |
| 16 | $CH_3(CH_2)_6CO$— | H | $CH_3(CH_2)_6CO$— | >600 |
| 17 | $CH_3(CH_2)_9CO$— | H | $CH_3(CH_2)_9CO$— | 94 |
| 22 | $CH_3(CH_2)_{11}$— | H | H | >300 |

[a]Administered intravenously, [b]Two experiments, [c]$R^3$, $R^4$, $R^5$ = H

Preparations 1—9

The preparation of a number of useful N-alkanoylamino acids is described in U.S. Patent 4,293,483. Such compounds are prepared according to the following general procedure.

The appropriated alkanoic acid chloride is added dropwise to the appropriate amino acid (1:1 mole ratio) dissolved in pyridine. The amount of pyridine used should be sufficient to make the concentration of reactants between 0.1 to 0.2 M. The solution is stirred at room temperature for about 3 to 6 hours, after which it is poured into a large volume of water. The product precipitates from solution and is collected by fitration and crystallized from methanol.

Other N-alkanoylamino acids prepared by this procedure are summarized in Table 11.

TABLE 11

Preparation of N-Alkanoyl Amino Acids

| Prep No. | Alkanoic acid chloride Formula | wt. (g) | Amino Acid Formula | wt. (g) | N-Alkanoyl Amino Acid Formula | wt. (g) |
|---|---|---|---|---|---|---|
| 1 | $CH_3(CH_2)_6COCl$ | 3.25 | L-phenylalanine | 3:30 | $CH_3(CH_2)_6CONHCH(CH_2C_6H_5)COOH$ | 4.85 |
| 2 | $CH_3(CH_2)_7COCl$ | 2.0 | L-phenylalanine | 1.82 | $CH_3(CH_2)_7CONHCH(CH_2C_6H_5)COOH$ | 2.8 |
| 3 | $CH_3(CH_2)_8COCl$ | 3.9 | L-phenylalanine | 3.30 | $CH_3(CH_2)_8CONHCH(CH_2C_6H_5)COOH$ | 5.35 |
| 4 | $CH_3(CH_2)_9COCl$ | 4.0 | L-phenylalanine | 3.23 | $CH_3(CH_2)_9CONHCH(CH_2C_6H_5)COOH$ | 4.5 |
| 5 | $CH_3(CH_2)_{10}COCl$ | 6.54 | L-phenylalanine | 4.95 | $CH_3(CH_2)_{10}CONHCH(CH_2C_6H_5)COOH$ | 5.2 |
| 6 | $CH_3(CH_2)_{11}COCl$ | 2.0 | L-phenylalanine | 1.42 | $CH_3(CH_2)_{11}CONHCH(CH_2C_6H_5)COOH$ | 1.7 |
| 7 | $CH_3(CH_2)_{12}COCl$ | 4.8 | L-phenylalanine | 3.30 | $CH_3(CH_2)_{12}CONHCH(CH_2C_6H_5)COOH$ | 6.6 |
| 8 | $CH_3(CH_2)_4COCl$ | 6.2 | L-tryptophan | 10.2 | 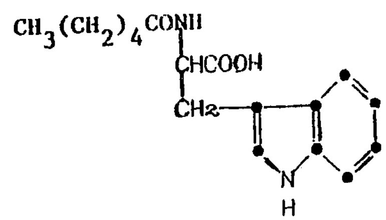 | 6.82 |
| 9 | $CH_3(CH_2)_{11}COCl$ | 10.9 | L-tryptophan | 10.2 | 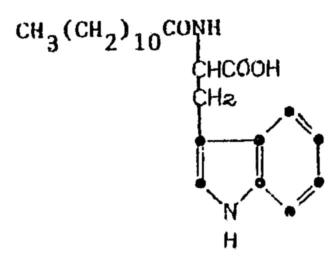 | 13.8 |

Preparations 10—19

The 2,4,5-trichlorophenyl esters of various N-alkanoylamino acids are described in U.S. Patent 4,293,483. Such compounds are prepared according to the following general procedure:

The N-alkanoylamino acid (1 mole), 2,4,5-trichlorophenol (1.1 mole), and DCC (1 mole) are dissolved in dichloromethane, diethyl ether or THF. The solution is stirred at room temperature for about 16 to about 20 hours after which it is filtered. The filtrate is taken to dryness, and the product is crystallized from either acetonitrile-water or diethyl ether-petroleum ether.

The preparation of other 2,4,5-trichlorophenyl esters of N-alkanoylamino acids prepared by this method is summarized in Table 12.

TABLE 12

Preparation of 2,4,5-Trichlorophenyl Esters

| Preparation No. | N-Alkanoyl Amino Acid Formula | wt. (g) | 2,4,5-Trichlorophenyl Ester Product wt. (g) |
|---|---|---|---|
| 10 | $CH_3(CH_2)_6CONHCH(CH_2C_6H_5)COOH$ | 2.9 | 4.1 |
| 11 | $CH_3(CH_2)_7CONHCH(CH_2C_6H_5)COOH$ | 2.8 | 3.86 |
| 12 | $CH_3(CH_2)_8CONHCH(CH_2C_6H_5)COOH$ | 3.19 | 1.5 |
| 13 | $CH_3(CH_2)_9CONHCH(CH_2C_6H_5)COOH$ | 3.29 | 5.01 |
| 14 | $CH_3(CH_2)_{11}CONHCH(CH_2C_6H_5)COOH$ | 1.7 | 2.01 |
| 15 | $CH_3(CH_2)_{12}CONHCH(CH_2C_6H_5)COOH$ | 3.75 | 4.18 |
| 16 | 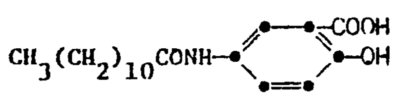 | 2.1 | 1.6 |
| 17 | $CH_3(CH_2)_{10}CONHCH(CH_2C_6H_5)COOH$ | 3.47 | 3.98 |
| 18 | 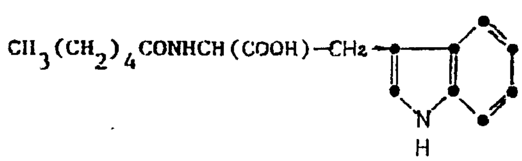 | 6.04 | 1.87 |
| 19 | 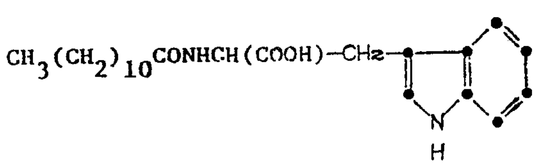 | 7.76 | 4.03 |

Examples 38—70

The N-alkanoylamino acid derivatives of A—21978C of formula *1* are prepared according to the following general procedure which involves acylating the nucleus using the activated-ester method:

A solution of $N_{Orn}$-t-BOC-blocked-A—21978C nucleus (t-BOC nucleus) in DMF was treated with the 2,4,5-trichlorophenyl ester ("active ester") of the corresponding N-alkanoylamino acid. The reaction mixture was stirred at room temperature for about 18 to about 24 hours under an atmosphere of nitrogen and then was evaproated to dryness under reduced pressure to give a residue. A small amount of methanol was added to the residue; a solid (N,N'-dicyclohexylurea) which did not dissolve in the methanol was removed by filtration and discarded. The filtrate was evaporated under vacuum to give a solid, the crude $N_{Orn}$-t-BOC-$N_{Trp}$ acyl-A—21978C analog. This analog was purified using a "Prep LC/System 500" unit (Waters Associates, Inc., Milford, MA) equipped with a Prep Pak-500/$C_{18}$ column (Water Associates Inc.) as a stationary phase. The column was eluted isocratically, using a water:methanol:acetonitrile (2:1:2) solvent system containing 0.1% pyridinium acetate, and eluting one 250-ml fraction per minute for approximately

40 fractions. Amounts of sample applied varied from about 1 g to about 5g. Early fractions containing unreacted starting materials were discarded. The desired product was always the major peak (using a UV detector) following the early fractions. Individual fractions were pooled on the basis of TLC [reversed-phase silica gel ($C_{18}$), a water-methanol-acetonitrile (3:3:4) solvent system, and Von Urk spray for detection] and bioautographic analysis [silica-gel TLC plates (Merck), an acetonitrile:acetone:water (2:2:1) solvent system and *Micrococcus luteus* as the assay organism].

The $N_{Orn}$-t-BOC-$N_{Trp}$-acyl-A—21978C analog, obtained as a single component by this method, was lyophilized and treated with anyhdrous trifluoroacetic-acid (10 ml per 0.3—0.5 g of analog) containing 2% anisole at 0°C. After about five minutes the reaction mixture was evaporated to dryness under reduced pressure. The residue obtained was triturated with a small amount of ether. The solid precipitate was collected and air-dried. This material was dissolved in water; the pH of the solution was raised to about 6 to 7 by the addition of pyridine; and the solution was then lyophilized. The resulting product was obtained as a single component and was characterized by its chromatographic properties and its amino-acid analysis.

Table 13 summarizes a group of $N_{Trp}$-alkanoyl-aminoacyl A—21978C derivatives prepared by this procedure.

TABLE 13

| Example No. | Product[a] R in formula 1 | Ester (mg) | A—21978C Nucleus (mg) | t-BOC A—21978C Product (mg)[b] | Product (mg) | $R_f^c$ |
|---|---|---|---|---|---|---|
| 38 | (D)$CH_3(CH_2)_{10}CONHCH(CH_2C_6H_5)CO$— | 900 | 900[d] | 556 | 436 | 0.82 |
| 39 | $CH_3(CH_2)_{10}CONH(CH_2)_4$—CO— | 900 | 900[d] | 489 | 485 | 0.65 |
| 40 | $CH_3(CH_2)_{10}CONH(CH_2)_{10}$—CO— | 600 | 900[d] | 326 | 242 | 0.83 |
| 41 | $CH_3(CH_2)_{10}CONH$— 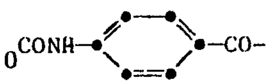 | 416 | 1000[e] | — | 195 | 0.34 |
| 42 | $CH_3(CH_2)_{10}CONH$— 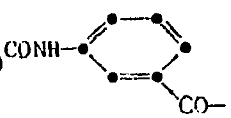 | 900 | 900[d] | 352 | 263 | 0.57 |
| 43 | $CH_3(CH_2)_{10}CONH$— 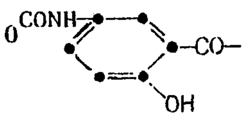 | 800 | 800[d] | 76 | 24 | 0.23 |
| 44 | $CH_3(CH_2)_{10}CONH$— 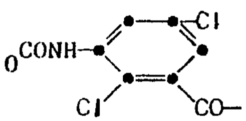 | 800 | 800[d] | 389 | 312 | 0.17 |
| 45 | (L)$CH_3(CH_2)_{10}CONHCH(CH_2C_6H_5)CO$— | 800 | 800[d] | 421 | 324 | 0.82 |
| 46 | (L)$CH_3(CH_2)_4CONHCHCO$– 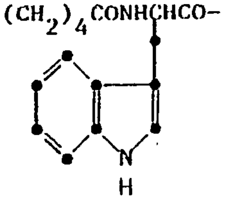 | 1000 | 1000 | — | 230 | 0.68 |

TABLE 13 (continued)

| Example No. | Product[a] R in formula 1 | Ester (mg) | A—21978C Nucleus (mg) | t-BOC A—21978C Product (mg)[b] | Product (mg) | $R_f^c$ |
|---|---|---|---|---|---|---|
| 47 | 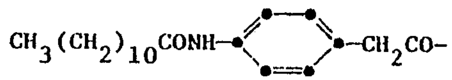 | 900 | 900[d] | 633 | 485 | 0.74 |
| 48 | 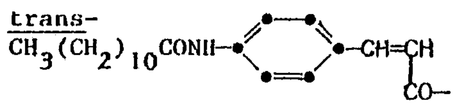 | 800 | 800[d] | 245 | 186 | 0.58 |
| 49 | 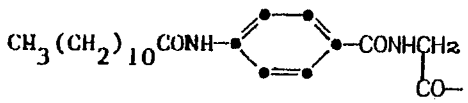 | 350 | 700[d] | 376 | 304 | 0.38 |
| 50 | 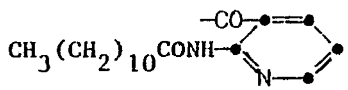 | 900 | 900[d] | 320 | 218 | 0.65 |
| 51 | $CH_3(CH_2)_5COHN(CH_2)_{10}$—CO— | 900 | 900[d] | 503 | 428 | 0.60 |
| 52 | 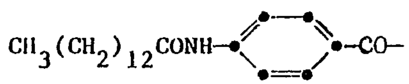 | 900 | 900[d] | 273 | 212 | 0.83 |
| 53 | 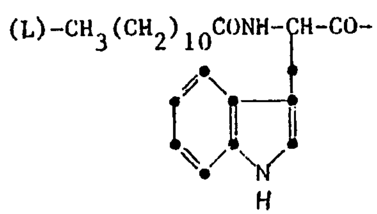 | 1000 | 1000[d] | 426 | 286 | 0.73 |
| 54 | (L)$CH_3(CH_2)_6CONHCH(CH_2C_6H_5)CO$— | 800 | 800[d] | 444 | 343 | 0.48 |
| 55 | (L)$CH_3(CH_2)_7CONHCH(CH_2C_6H_5)CO$— | 800 | 800[d] | 412 | 312 | 0.50 |
| 56 | (L)$CH_3(CH_2)_8CONHCH(CH_2C_6H_5)CO$— | 800 | 800[d] | 519 | 453 | 0.52 |

TABLE 13 (continued)

| Example No. | Product[a] R in formula 1 | Ester (mg) | A—21978C Nucleus (mg) | t-BOC A—21978C Product (mg)[b] | Product (mg) | $R_f^c$ |
|---|---|---|---|---|---|---|
| 57 | (L)$CH_3(CH_2)_9CONHCH(CH_2C_6H_5)CO$— | 800 | 800[d] | 371 | 287 | 0.53 |
| 58 | (L)$CH_3(CH_2)_{11}CONHCH(CH_2C_6H_5)CO$— | 800 | 800[d] | 412 | 335 | |
| 59 | (L)$CH_3(CH_2)_{12}CONHC(CH_2C_6H_5)CO$— | 800 | 800[d] | 468 | 328 | |

[a]$R^{1-5}$=H, [b]$N_{Orn}$-t-BOC-$N_{Trp}$-Acyl-Nucleus,
[c]Thin-layer chromatography on silica gel (Merck), using a water:$CH_3CN$:acetone (1:2:2) solvent system
[d] $N_{Orn}$-t-BOC-Nucleus, [e]A21978C Nucleus

Other N-alkanoylamino acid derivatives of formula *1* prepared in a similar manner are summarized in Table 14.

TABLE 14

A—21978C Cyclic Peptides of Formula *1*

| Example No. | R | $R^{2a}$ |
|---|---|---|
| 60 | 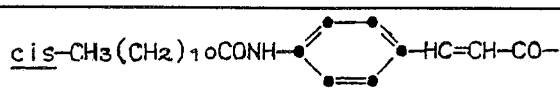 | H |
| 61 | 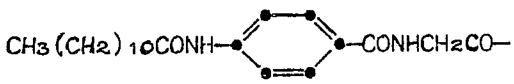 | H |
| 62 | H | 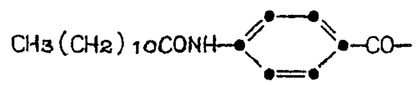 |
| 63 | H | $CH_3(CH_2)_9CONHCH(CH_2C_6H_9)CO—$ |

[a]$R^1$, $R^3$, $R^4$, $R^5$ = H

Table 15 summarizes a group of A—21978C derivatives prepared according to the general procedure, but using A—21978C factors as starting materials.

TABLE 15

Diacyl Derivatives of A—21978C

| Example No. | $R^b$ in formula *1* | $R^2$ in formula *1* | Starting Factor | Ester (mg) | A—21978C Factor (mg) | Product (mg) | $R_f^a$ |
|---|---|---|---|---|---|---|---|
| 64 | $CH_3CH_2CH(CH_3)(CH_2)_6CO$— | (L)-$CH_3(CH_2)_4CONHCH$-CO- 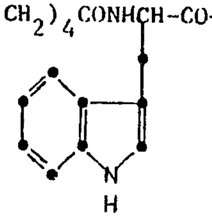 | $C_1$ | 100 | 48 | 43 | 0.81 |
| 65 | $CH_3CH(CH_3)(CH_2)_8CO$— | " | $C_2$ | 100 | 48 | 25 | 0.73 |
| 66 | $CH_3CH_2CH(CH_3)(CH_2)_8CO$— | " | $C_3$ | 400 | 1000 | 413 | 0.87 |
| 67 | $CH_3CH_2CH(CH_3)(CH_2)_6CO$— | $H_2N(CH_2)_{10}$—CO— | $C_1$ | 1000 | 1000 | 732 | 0.65 |

[a]TLC on silica gel (Merck) using a water:$CH_3CN$:acetone (1:2:2) solvent system
[b]$R^1$, $R^3$, $R^4$, $R^5$ = H

Other $N_{Trp}$-$N_{Orn}$-diacyl derivatives of A—21978C prepared according to the general procedure are listed in Table 16.

TABLE 16

Diacyl Derviatives of A—21978C

| Example No. | R in Formula 1[a] | $R^2$ in Formula 1 |
|---|---|---|
| 68 | 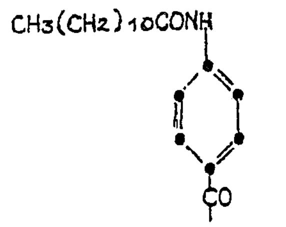 | 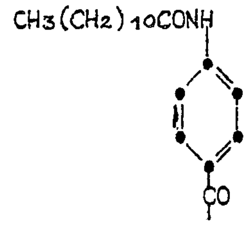 |
| 69 | 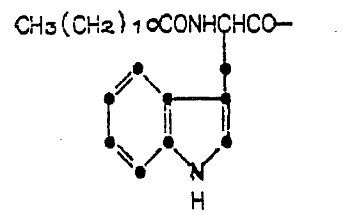 | t-BOC |
| 70 | $CH_3(CH_2)_{10}CONHCH\ (CH_2C_6H_5)CO—$ | t-BOC |

[a] $R^1, R^3, R^4, R^5 = H$

## Example 71

Preparation of $N_{Trp}$-[N-(*n*-Decanoyl)-*L*-phenylalanyl]-A—21978C Nucleus (Compound of Example 56)

This example illustrates the large-scale preparation of compounds by the active-ester method.

A. Preparation of N-(*n*-Decanoyl)-*L*-Phenylalanyl-2,4,5-Trichlorophenolate

A solution of N-(*n*-decanoyl)-*L*-phenylalanine (31.9 g, 0.1 mole) and 2,4,5-trichlorophenol (19.7 g, 0.1 mole) in 1 liter of anhydrous ether was treated with N,N'-dicyclohexylcarbodiimide (20.6 g, 0.1 mole). The reaction was stirred overnight at room temperature. The precipitated N,N'-dicyclohexylurea was removed by filtration and discarded. The filtrate was concentrated under vacuum to dryness. The residue obtained was triturated with ether, and the solids (residual cyclohexylurea) were removed by filtration. The filtrate was evaporated to dryness under reduced pressure. The residue was crystallized from acetonitrile to give 36.9 g of crystalline N-(*n*-decanoyl)-*L*-phenylalanyl 2,4,5-trichlorophenolate, m.p. 122—124°C.

B. Preparation of $N_{Trp}$-N-(*n*-Decanoyl)-*L*-phenylalanyl]-$N_{Orn}$-t-BOC—A—21978C Nucleus

A solution of N-(*n*-decanoyl)-*L*-phenylalanyl 2,4,5-trichlorophenolate (10 g, 0.02 mole), $N_{Orn}$-t-BOC-A—21978C nucleus (10 g, 0.0006 mole) in anhydrous DMF (1 L) was stirred at room temperature for 96 hours under an atmosphere of nitrogen. The solvent was removed by evaporation under reduced pressure. The residual material was stirred with a mixture of diethyl ether (800 ml) and chloroform (200 ml) for 2 hours. The product was separated by filtration to give a light brown powder (10.3 g). This material (9.9 g) was dissolved in methanol (200 ml) and purified by preparative HPLC, using a "Prep LC/System 500" unit and a PrepPak—500/$C_{18}$ Column as the stationary phase. The column was eluted isocratically, using a water:methanol:acetonitrile (2:1:2) solvent system and collecting 250-ml fractions at a rate of one fraction per minute. The desired compound was eluted in the 9th through the 22nd fractions.

Fractions were combined on the basis of TLC [reversed phase silica gel/$C_{18}$; developed with water:methanol:acetonitrile (3:3:4); detected with Van Urk spray]. Combined fractions were examined by bioautography [silica gel TLC acetonitrile:acetone:water (2:2:1) solvent system and *Micrococcus luteus* as the detecting organism] and were shown to consist of a single bioactive component. This procedure gave 6.02 g of $N_{Trp}$-N-(*n*-decanoyl)-*L*-phenylalanyl]-$N_{Orn}$-t-BOC-A—21978C nucleus [compound of formula 1: R = N-(*n*-decanoyl)-*L*-phenylalanyl); $R^2$ = t-BOC].

C. Preparation of $N_{Trp}$-[N-(*n*-Decanoyl)-*L*-phenylalanyl]-A—21978C Nucleus

A flask (100 ml) was cooled to 5°C in an icebath. $N_{Trp}$-[N-(*n*-decanoyl)-*L*-phenylalanyl]-$N_{Orn}$-t-BOC-A—21978C nucleus (6.02 g, 0.008 mole), prepared as described in Section B, and then anhydrous

trifluoroacetic acid containing 2% anisole (50 ml) were added to the flask. The mixture, which went into solution in approximately two minutes, was stirred under an atmosphere of nitrogen for ten minutes. The solution was evaporated to dryness under reduced pressure at below 40°C to give a gummy solid which was triturated twice with a diethyl ether: dichloromethane (4:1) solution (two 100-ml volumes). The solids were collected by filtration and washed with diethyl ether to give the TFA salt. This was dissolved in water (50 ml), and the pH of the solution was adjusted to 5.4 with pyridine. The solution was then lyophilized to give 6.1 g of off-white lyophilizate.

The lyophilizate, dissolved in methanol (35 ml), was purified using a reverse-phase $C_{18}$ silicagel column (Waters Associates, Prep 500), eluting in stepwise gradient with $H_2O$:$CH_3OH$:$CH_3CN$ containing 0.1% pyridinium acetate at ratios of 3:1:2, 2:1:2 and 1:2:2 and collecting fractions having a volume of 250 ml. The desired product was eluted during the 2:1:2 elution. The fractions containing the product were lyophilized to give 2.23 g of cream-colored $N_{Trp}$-[N-(*n*-decanoyl)-*L*-phenylalanyl]-A—21978C nucleus (compound of formula 1: R = N-(*n*-decanoyl)-*L*-phenylalanyl; $R^{1-5}$ = H).

The product was evaluated by analytical HPLC [reversed-phase $C_{18}$ silica-gel column, MeOH:$CH_3CN$:$H_2O$:PyOAc (15:35:49:1) solvent and UV detection at 230 nm], by TLC [reversed-phase $C_{18}$ silica-gel plates (Whatman) $H_2O$:$CH_3OH$:$CH_3CN$ (3:3:4) solvent and Van Urk spray and short-wave UV for detection] and by bioautography [silica-gel TLC (Merck), an $H_2O$:$CH_3CN$:acetone (1:2:2) solvent, and *Micrococcus luteus* as the detecting organism]. Each of these methods demonstrated that the product was homogenous. Substitution at the tryptophan N-terminus position was confirmed by 360 MHz PMR. Amino-acid analysis confirmed the incorporation of one equivalent of L-phenylalanine into the product.

Example 72

The antibiotic activity of the compounds of formula 1 can be demonstrated *in vitro*, using standard agar-dilution tests. The results of the antibacterial testing of representative compounds of formula 1 are set forth in Table 17. In Table 17 activity is measured by the minimal inhibitory concentration (MIC), i.e. the lowest concentration of compound at which growth of the microorganism is inhibited by the test compound.

TABLE 17
Antibiotic Activity of A—21978C Cyclic Peptides

| Test Organism | MIC[a] of Test Compound [b] | | | |
|---|---|---|---|---|
| | 38 | 39[e] | 40 | 41[f] |
| *Staphylococcus aureus* X1.1 | 0.5 | 4 | 2 | 4, 8 |
| *Staphylococcus aureus* V41[c] | 0.5 | 4 | 2 | 4, 16 |
| *Staphylococcus aureus* X400[d] | 1 | 4 | 8 | 8, 16 |
| *Staphylococcus aureus* S13E | 0.5 | 4 | 2 | 4, 8 |
| *Staphylococcus epidermidis* EPI1 | 2 | 4 | 8 | 16, 64 |
| *Staphylococcus epidermidis* EPI2 | 1 | 2 | 8 | 16, 64 |
| *Streptococcus pyogenes* C203 | 0.125 | 1 | 2 | 4, 8 |
| *Streptococcus pneumoniae* Park I | 0.125 | 4 | 0.5 | 4, 16 |
| *Streptococcus* Group D X66 | 1 | 32 | 8 | 32, >64 |
| *Streptococcus* Group 9960 | 0.5 | 8 | 8 | 8, 32 |

[a] MIC in mcg/ml
[b] Numbers = example numbers in Tables 13—16
[c] Penicillin-resistant strain
[d] Methicillin-resistant-strain
[e] Median of five experiments
[f] Two experiments
[g] Median of three experiments

TABLE 17 continued

Antibiotic Activity of A—21978C Cyclic Peptides

| Test Organism | MIC[a] of Test Compound [b] | | | | | | | | | |
|---|---|---|---|---|---|---|---|---|---|---|
| | 42 | 43 | 44 | 45 | 46[f] | 47[f] | 48 | 49 | 50 | 51 |
| *Staphylococcus aureus* X1.1 | 0.5 | 1 | 0.5 | 0.25 | 32, 64 | 0.5, 0.25 | 0.5 | 1 | 0.25 | 4 |
| *Staphylococcus aureus* V41[c] | 0.5 | 2 | 1 | 0.25 | 64, 128 | 0.5, 0.5 | 0.25 | 2 | 0.25 | 4 |
| *Staphylococcus aureus* X400[d] | 1 | 4 | 2 | 0.5 | 64, 128 | 1, 0.5 | 2 | 4 | 0.5 | 8 |
| *Staphylococcus aureus* S13E | 0.5 | 2 | 0.5 | 0.25 | 32, 64 | 0.5, 0.5 | 0.5 | 2 | 0.25 | 4 |
| *Staphylococcus epidermidis* EPI1 | 2 | 2 | 2 | 1 | 128, >128 | 2, 1 | 0.5 | 4 | 0.5 | 8 |
| *Staphylococcus epidermidis* EPI2 | 1 | 2 | 2 | 1 | 128, >128 | 1, 0.5 | 0.5 | 4 | 0.25 | 8 |
| *Streptococcus pyogenes* C203 | 0.25 | 0.5 | 1 | 0.25 | 16, 16 | 0.125, 0.06 | 0.125 | 2 | 0.03 | 2 |
| *Streptococcus pneumoniae* Park I | 0.25 | 2 | 0.125 | 0.125 | 32, 64 | 0.25, 0.25 | 0.06 | 1 | 0.03 | 8 |
| *Streptococcus* Group D X66 | 4 | 16 | 2 | 1 | >128, >128 | 4, 4 | 2 | 16 | 1 | >128 |
| *Streptococcus* Group 9960 | 1 | 2 | 2 | 0.25 | 128, >128 | 2, 1 | 4 | 4 | 0.25 | 64 |

TABLE 17 continued

Antibiotic Activity of A—21978C Cyclic Peptides

| Test Organism | MIC[a] of Test Compound [b] | | | | | | | | | |
|---|---|---|---|---|---|---|---|---|---|---|
| | 52 | 53 | 54 | 55 | 56[g] | 57 | 58 | 59 | 60 | 61 |
| *Staphylococcus aureus* X1.1 | 1 | 0.5 | 4 | 2 | 0.5 | 0.5 | 0.5 | 0.5 | 0.5 | 1 |
| *Staphylococcus aureus* V41[c] | 2 | 0.5 | 8 | 2 | 0.5 | 0.5 | 0.5 | 1 | 1 | 2 |
| *Staphylococcus aureus* X400[d] | 8 | 1 | 8 | 4 | 1 | 2 | 2 | 2 | 4 | 4 |
| *Staphylococcus aureus* S13E | 2 | 0.5 | 8 | 2 | 0.5 | 0.5 | 0.5 | 1 | 1 | 2 |
| *Staphylococcus epidermidis* EPI1 | 4 | 1 | 8 | 4 | 1 | 2 | 2 | 4 | 2 | 4 |
| *Staphylococcus epidermidis* EPI2 | 4 | 1 | 8 | 2 | 0.5 | 2 | 2 | 4 | 2 | 4 |
| *Streptococcus pyogenes* C203 | 1 | 0.5 | 2 | 0.5 | 0.25 | 0.125 | 0.125 | 0.25 | 0.25 | 2 |
| *Streptococcus pneumoniae* Park I | 0.25 | 0.125 | — | — | 0.75 | — | — | — | 0.25 | 1 |
| *Streptococcus* Group D X66 | 4 | 1 | 128 | 32 | 8 | 4 | 1 | 1 | 4 | 16 |
| *Streptococcus* Group 9960 | 4 | 0.5 | 32 | 8 | 2 | 2 | 0.25 | 0.5 | 2 | 4 |

TABLE 17 continued

Antibiotic Activity of A—21978C Cyclic Peptides

| | MIC[a] of Test Compound [b] | | | | | | | | |
|---|---|---|---|---|---|---|---|---|---|
| Test Organism | 62 | 63 | 64 | 65 | 66[f] | 67 | 68[f] | 69 | 70 |
| *Staphylococcus aureus* X1.1 | 8 | 8 | 0.5 | 0.5 | 0.5, 1 | 1 | 16, 2 | 1 | 0.5 |
| *Staphylococcus aureus* V41[c] | 16 | >128 | 2 | 2 | 4, 4 | 1 | 32, 4 | 1 | 2 |
| *Staphylococcus aureus* X400[c] | 16 | >128 | 1 | 1 | 1, 1 | 2 | 32, 8 | 2 | 2 |
| *Staphylococcus aureus* S13E | 8 | 16 | 0.5 | 0.5 | 0.5, 1 | 1 | 16, 4 | 1 | 1 |
| *Staphylococcus epidermidis* EPI1 | 16 | >128 | 4 | 4 | 8, 16 | 2 | 128, 16 | 4 | 8 |
| *Staphylococcus epidermidis* EPI2 | 16 | >128 | 4 | 4 | 8, 16 | 1 | 64, 8 | 4 | 8 |
| *Streptococcus pyogenes* C203 | 4 | 1 | 0.5 | 0.5 | 1, 0.5 | 0.25 | 8, 4 | 1 | 1 |
| *Streptococcus pneumoniae* Park I | 8 | — | 0.5 | 0.25 | 0.25, 0.5 | 0.25 | 8, 4 | 0.5 | 0.5 |
| *Streptococcus* Group D X66 | 128 | >128 | 8 | 4 | 8, 16 | 64 | >128, 32 | 8 | 16 |
| *Streptococcus* Group 9960 | 32 | 32 | 4 | 4 | 32, 32 | 32 | 64, 8 | 4 | 4 |

The A—21978C cyclic peptides of formula 1 have shown *in vivo* antimicrobial activity against experimental bacterial infections. When two doses of test compound were administered subcutaneously or orally to mice in illustrative infections, the activity observed was measured as an $ED_{50}$ value [effective dose in mg/kg to protect fifty percent of the test animals: See Warren Wick, *et al., J. Bacteriol. 81,* 233—235 (1961)]. The $ED_{50}$ values observed for A—21978C compounds are given in Tables 18 and 19.

TABLE 18

In Vivo Activity of A—21978C Cyclic Peptides

| Example No. | Formula 1 Compound[a] R | $ED_{50}$ Values[b] *Staphylococcus aureus* Subcutaneous | *Streptococcus pyogenes* Subcutaneous | *Streptococcus pyogenes* Oral |
|---|---|---|---|---|
| 38 | (D)$CH_3(CH_2)_{10}CONHCH(CH_2C_6H_5)CO$— | 1.4, 2.05[c] | <0.25, 0.21 | >200 |
| 39 | $CH_3(CH_2)_{10}CONH(CH_2)_4$—CO— | 0.65, 0.93 | <0.25, 0.107 | 117 |
| 40 | $CH_3(CH_2)_{10}CONH(CH_2)_{10}$—CO— | >18 | 6.2 | >200 |
| 41 | 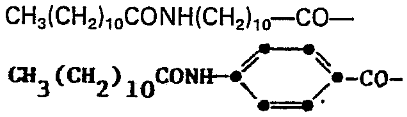 | <5 | 18.8 | >200 |
| 42 | 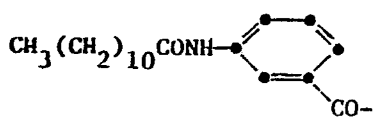 | 1.67 | >0.25, 0.46 | >200 |
| 44 | 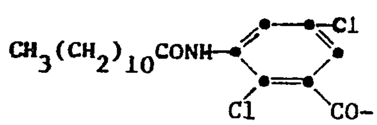 | 19 | 0.23 | >200 |
| 45 | (L)$CH_3(CH_2)_{10}CONHCH(CH_2C_6H_5)CO$— | 2.35 | 0.32 | 150 |
| 47 | 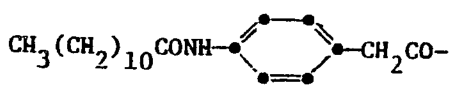 | 3.56, 4.12[c] | 0.69 | >200 |
| 48 | 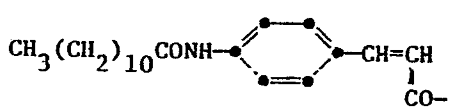 | 0.65 | 0.04 | 59 |
| 49 | 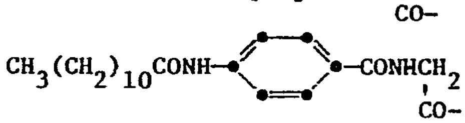 | 2.3 | <0.25, 0.12[c] | >200 |
| 50 | 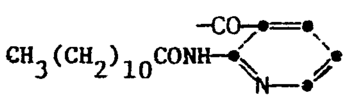 | 0.54 | 0.05 | 69 |
| 51 | $CH_3(CH_2)_5CONH(CH_2)_{10}$—CO— | >18 | >9 | >200 |
| 52 | 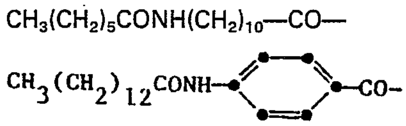 | >18 | 1.02 | >200 |
| 53 | 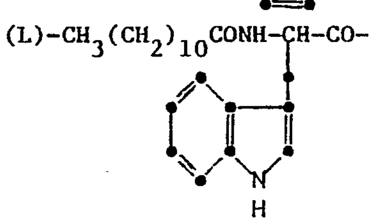 | 5.19 | 3.14 | 200 |

TABLE 18 Continued

In Vivo Activity of A—21978C Cyclic Peptides

| Example No. | Formula 1 Compound[a] R | ED$_{50}$ Values[b] *Staphylococcus aureus* Subcutaneous | *Streptococcus pyogenes* Subcutaneous | *Streptococcus pyogenes* Oral |
|---|---|---|---|---|
| 54 | (L)$CH_3(CH_2)_6CONHCH(CH_2C_6H_5)CO$— | 2.58 | 1.48 | >200 |
| 55 | (L)$CH_3(CH_2)_7CONHCH(CH_2C_6H_5)CO$— | 1.38 | 0.59 | 184 |
| 56 | (L)$CH_3(CH_2)_8CONHCH(CH_2C_6H_5)CO$— | 0.7, 0.98[c] | 0.39 | >200 |
| 57 | (L)$CH_3(CH_2)_9CONHCH(CH_2C_6H_5)CO$— | 1.25 | 0.35 | >200 |
| 58 | (L)$CH_3(CH_2)_{11}CONHCH(CH_2C_6H_5)CO$— | 0.76 | 0.14 | >200 |
| 59 | (L)$CH_3(CH_2)_{12}CONHCH(CH_2C_6H_5)CO$— | 4.8 | <0.27, 0.36[c] | >200 |
| 61 | $CH_3(CH_2)_{10}CONH$—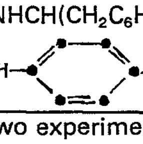—$CONHCH_2CO$— | 2.3 | <0.25, 0.12[c] | >200 |

[a] $R^{1-5}$ = H, [b] mg/kg × 2, [c] Two experiments

TABLE 19

In Vivo Activity of A—21978C Cyclic Peptides

| Example No. | Formula 1 Compound R[c] | Formula 1 Compound $R^2$ | ED$_{50}$ Values[a] *Staphylococcus aureus* Subcutaneous | *Streptococcus pyogenes* Subcutaneous | *Streptococcus pyogenes* Oral |
|---|---|---|---|---|---|
| 62 | H | $CH_3(CH_2)_{10}CONH$—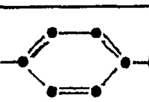—CO— | >70 | >22 | >200 |
| 63 | H | $CH_3(CH_2)_9CONHCH(CH_2C_6H_5)CO$— | 9.2 | >18 | >200 |
| 66 | $CH_3CH_2CH(CH_3)(CH_2)_8CO$— | (L)—$CH_3(CH_2)_4CONHCH$—CO—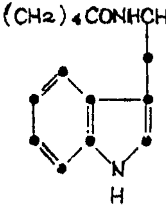 | >2.2, >70[b] | 10.6, 6.0 | >200 |

[a] mg/kg × 2
[b] Two experiments
[c] $R^1$, $R^3$, $R^4$, $R^5$ = H

The results of toxicity tests on some of the A—21978C cyclic peptides are summarized in Table 20.

TABLE 20

Toxicity of A—21978C Cyclic Peptides

Formula 1 Compound

| Example No. | $R^c$ | $R^2$ | $LD_{50}$ (mg/kg) in Mice[a] |
|---|---|---|---|
| 38 | $(D)CH_3(CH_2)_{10}CONHCH(CH_2C_6H_5)CO—$ | H | 250 |
| 39 | $CH_3(CH_2)_{10}CONH(CH_2)_4—CO—$ | H | >600 |
| 40 | $CH_3(CH_2)_{10}CONH(CH_2)_{10}—CO—$ | H | 600 |
| 41 | 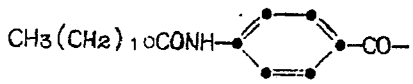 | H | >300 |
| 42 | 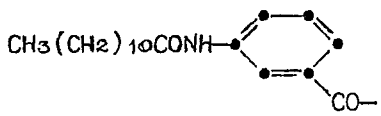 | H | 277 |
| 44 | 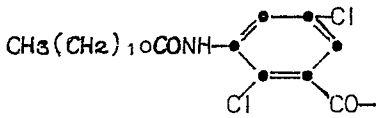 | H | 200 |
| 45 | $(L)CH_3(CH_2)_{10}CONHCH(CH_2C_6H_5)CO—$ | H | 250 |
| 46 | 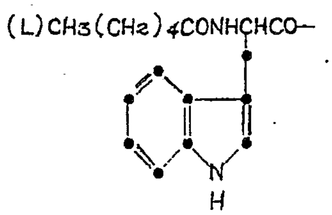 | H | 450[b] |
| 47 | 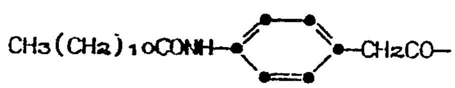 | H | 450 |
| 48 | 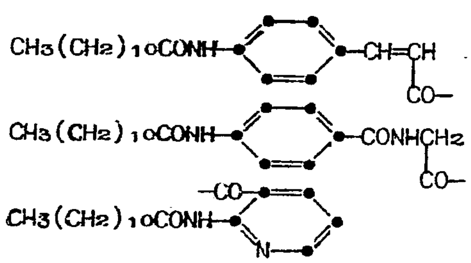 | H | 450 |
| 49 | | H | 450 |
| 50 | | H | 300 |
| 51 | $CH_3(CH_2)_5CONH(CH_2)_{10}—CO—$ | H | >600 |
| 52 | 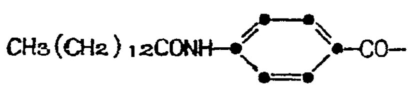 | H | 250 |
| 53 | 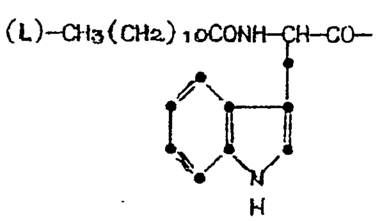 | H | 225 |

TABLE 20 Continued

Toxicity of A—21978C Cyclic Peptides

Formula 1 Compound

| Example No. | $R^c$ | $R^2$ | $LD_{50}$ (mg/kg) in Mice[a] |
|---|---|---|---|
| 54 | $(L)CH_3(CH_2)_6CONHCH(CH_2C_6H_5)CO—$ | H | 450 |
| 55 | $(L)CH_3(CH_2)_7CONHCH(CH_2C_6H_5)CO—$ | H | >600 |
| 56 | $(L)CH_3(CH_2)_8CONHCH(CH_2C_6H_5)CO—$ | H | 600 |
| 57 | $(L)CH_3(CH_2)_9CONHCH(CH_2C_6H_5)CO—$ | H | 400 |
| 58 | $(L)CH_3(CH_2)_{11}CONHCH(CH_2C_6H_5)CO—$ | H | 225 |
| 59 | $(L)CH_3(CH_2)_{12}CONHCH(CH_2C_6H_5)CO—$ | H | 225 |
| 61 | 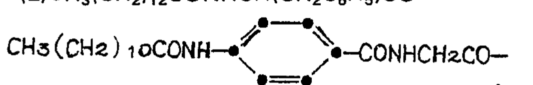 | H | 450 |
| 62 | H | 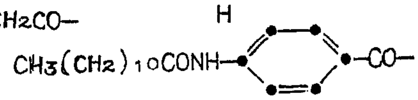 | 300 |
| 63 | H | $CH_3(CH_2)_9CONHCH(CH_2C_6H_5)CO—$ | 225 |
| 66 | $CH_3CH_2CH(CH_3)(CH_2)_8CO—$ | 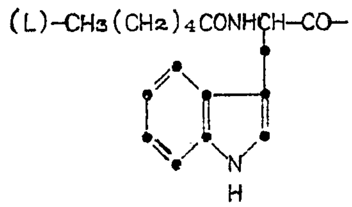 | 37.5 |

[a] Administered intravenously
[b] Material was in suspension
[c] $R^1$, $R^3$, $R^4$, $R^5$ = H

Example 73

A. Preparation of $N_{Trp}$-*p*-(*n*-Dodecyloxy)benzoyl-$N_{Orn}$-t-BOC-A—21978C Nucleus

A solution of 2,4,5-trichlorophenyl *p*-(*n*-dodecyloxy)benzoate (0.9 g), and, A—21978C t-BOC nucleus (0.9 g) in 400 ml of anhydrous dimethylformamide was allowed to stir at room temperature for 120 hours under an atmosphere of nitrogen. The solvent was removed by evaporation under reduced pressure. The residual material was stirred with a mixture of diethyl ether (400 ml) and chloroform (400 ml) for 2 hours. The product was separated by filtration and dried to give a light brown powder (0.962 g). A portion of this material (0.78 g) was dissolved in methanol (200 ml) and purified by preparative HPLC, using a "Prep LC/System 500" unit (Waters Associates, Inc., Milford Mass.) and a Prep Pak-500/$C_{18}$ Column (Waters Associates) as a stationary phase. The column was operated isocratically, using a water:methanol:acetonitrile (2:1:2) solvent system and collecting 250-ml fractions (1 fraction/min.). The desired compound was eluted in the 2nd to the 6th fractions.

Fractions were combined on the basis of TLC [reverse phase/$C_{18}$ silica gel, developed with water:methanol:acetonitrile (3:3:4), detected with Van Urk spray]. Bioautography of the combined fractions, using silica gel TLC, an acetonitrile:acetone:water (2:2:1) solvent, and *Staphylococcus aureus* as the detecting organism, indicated that the product was a single bioactive component. This procedure gave 0.421 g of $N_{Trp}$-*p*-(*n*-dodecyloxy)benzoyl-$N_{Orn}$-t-BOC-A—21978C nucleus.

B. Preparation of $N_{Trp}$-*p*-(*n*-dodecyloxy)benzoyl-A—21978C Nucleus

$N_{Trp}$-*p*-(*n*-Dodecyloxy)benzoyl-$N_{Orn}$-t-BOC-A—21978C nucleus (230 mg) was dissolved in 5 ml of trifluoroacetic acid containing 2% anisole and stirred for 5 minutes at 0°C. The solution was concentrated to

an oil under vacuum, and the oil was triturated with $Et_2O$ (100 ml). The solids were separated, air-dried, and taken up in water (10 ml). The pH of this solution was adjusted from 3.25 to 7 by the addition of pyridine. The resulting solution was lyophilized to give 179 mg of white amorphous $N_{Trp}$-*p*-(*n*-dodecyloxy)benzoyl-A—21978C nucleus. This compound has an $R_f$ value of about 0.78 on silica-gel TLC, using an acetonitrile:acetone:water (2:2:1) solvent system and Van Urk spray for detection.

Example 74

The antibacterial activity of the compounds of Example 73 can be demonstrated *in vitro*. The results of the antibacterial testing of these compounds using standard agar-plate disc-diffusion tests are set forth in Table 21. In Table 21 activity is measured by the size (diameter in mm) of the observed zone in which growth of the microorganism is inhibited by the test compound.

TABLE 21

Antibacterial Activity of Formula 1 Compounds by the Agar-Plate Disc-Diffusion Test

| Compound | | Size of Zone of Inhibition (mm)[a] | | | |
|---|---|---|---|---|---|
| | | *Staphylococcus aureus* | *Bacillus subtilis* | *Micrococcus luteus* | *B. subtilis* |
| $R^c$ | $R^2$ | ATCC 6738P | ATCC 6633 | ATCC 9341 | ATCC 6633[b] |
| *p*-(*n*-dodecyloxy)benzoyl | H | 20 | 13 | 18 | 20 |
| *p*-(*n*-dodecyloxy)benzoyl | t-BOC | 20 | 10 | 15 | 22 |

[a] Compounds were suspended in water at a concentration of 1 mg/ml; a 7-mm disc was dipped into the suspension and then placed on the agar surface; incubation — 24—48 hours at 25—37°C.
[b] Grown on minimal nutrient agar
[c] $R^1$, $R^3$, $R^4$, $R^5$ = H

The results of the antibacterial testing of a representative benzoic acid derivative, using the standard agar-dilution test, are summarized in Table 22. In Table 22 activity is measured by the minimal inhibitory concentration (MIC), i.e. the lowest concentration of compound which inhibits the growth of the microorganism.

TABLE 22
Antibiotic Activity of $N_{Trp}$-*p*-(*n*-Dodecyloxy)benzoyl-A—21978C

| Test Organism | MIC Values[a] |
|---|---|
| *Staphylococcus aureus* X1.1 | 1 |
| *Staphylococcus aureus* V41[b] | 1 |
| *Staphylococcus aureus* X400[c] | 2 |
| *Staphylococcus aureus* S13E | 1 |
| *Staphylococcus epidermidis* EPI1 | 4 |
| *Staphylococcus epidermidis* EPI2 | 2 |
| *Streptococcus pyogenes* C203 | 0.25 |
| *Streptococcus pneumoniae* Park I | 0.015 |
| *Streptococcus* Group D X66 | 2 |
| *Streptococcus* Group 9960 | 1 |

[a] MIC in mcg/ml
[b] Penicillin-resistant strain
[c] Methicillin-resistant strain

The representative benzoic acid derivatives of A—21978C nucleus have shown *in vivo* antimicrobial activity against experimental bacterial infections. When two doses of test compound were administered to mice in illustrative infections, the activity observed was measured as an $ED_{50}$ value [effective dose in mg/kg to protect fifty percent of the test animals: See Warren Wick, et al., *J. Bacteriol. 81*, 233—235 (1961)]. The $ED_{50}$ values observed are given in Table 23.

TABLE 23
*In Vivo* Activity of $N_{Trp}$-*p*-(*n*-Dodecyloxy)benzoyl-A—21978C

| Formula 1 Compound[a] | $ED_{50}$ Values[b] | | |
|---|---|---|---|
| | *Staphylococcus aureus* | *Streptococcus pyogenes* | |
| R | Subcutaneous | Subcutaneous | Oral |
| $N_{Trp}$-*p*-(*n*-Dodecyloxy)benzoyl | 3.35 | 0.31 | >200 |

[a]$R^{1-5}$ = H
[b]mg/kg × 2

The acute toxicity of $N_{Trp}$-*p*-(*n*-dodecyloxy)benzoyl-A—21978C, when administered intravenously to mice and expressed as $LD_{50}$, was 67.5 mg/kg.

**Claims**

1. An A—21973C cyclic peptide derivative of formula (1):

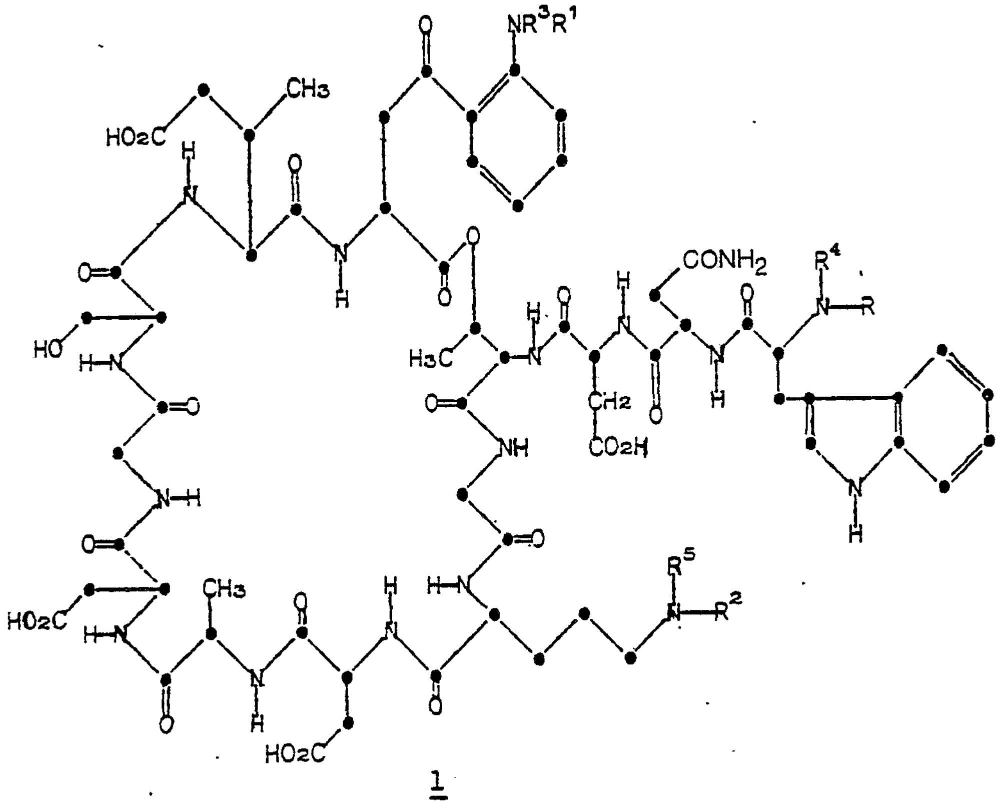

1

in which R, $R^1$, and $R^2$ are, independently, hydrogen, 8-methyldecanoyl, 10-methyldodecanoyl, 10-methylundecanoyl, the specific $C_{10}$-alkanoyl group of A—21978$C_0$ the specific $C_{12}$-alkanoyl groups of A—21978C factors $C_4$ or $C_5$, an amino-protecting group,

(A) an aminoacyl group of the formula

$$-\underset{\underset{O}{\parallel}}{C}-Q-NH_2$$

in which Q is $C_1$—$C_{16}$ alkylene, or an N-alkanoylaminoacyl group of the formula

$$-W-\underset{\underset{O}{\parallel}}{C}-R^7$$

in which W is a divalent aminoacyl radical of the formula:

(1) $$-\overset{\overset{O}{\parallel}}{C}-A-NH-$$

in which A is $C_1$—$C_{10}$ alkylene or $C_5$—$C_6$ cycloalkylene;

(2) $$-\overset{\overset{O}{\parallel}}{C}-\overset{\overset{R^8}{|}}{C}H-NH-$$

in which $R_8$ is hydroxymethyl, hydroxyethyl, mercaptomethyl, mercaptoethyl, methylthioethyl, 2-thienyl, 3-indole-methyl, phenyl, benzyl, or substituted phenyl or substituted benzyl, in which the benzene ring

thereof is substituted with chloro, bromo, iodo, nitro, $C_1$—$C_3$ alkyl, hydroxy, $C_1$—$C_3$-alkoxy, $C_1$—$C_3$ alkylthio, carbamyl, or $C_1$—$C_3$ alkylcarbamyl;

(3) 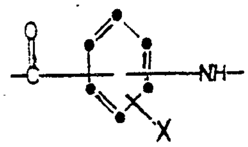

in which X is hydrogen, chloro, bromo, iodo, amino, nitro, $C_1$—$C_3$ alkyl, hydroxy, $C_1$—$C_3$ alkoxy, mercapto, $C_1$—$C_3$ alkylthio, carbamyl, or $C_1$—$C_3$ alkylcarbamyl;

(4) 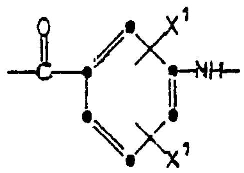 or 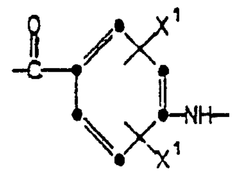

in which $X^1$ is chloro, bromo, iodo, amino, hydroxy, $C_1$—$C_3$ alkyl or $C_1$—$C_3$ alkoxy;

(5) 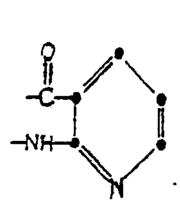 or 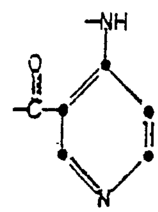 ; or

(6) 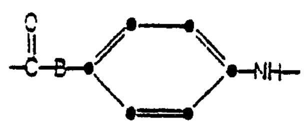

in which B is a divalent radical of the formula: $-(CH_2)_n-$ and n is an integer from 1 to 3; $-CH{=}CH-$; $-CH{=}CH-CH_2-$; or $-CH_2-NH-\overset{\overset{O}{\|}}{C}-$ ;

$R^7$ is $C_1$—$C_{17}$ alkyl or $C_2$—$C_{17}$ alkenyl;

(B) a substituted benzoyl group of the formula

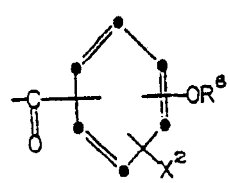

in which $R^6$ is $C_8$—$C_{15}$ alkyl;

$X^2$ is hydrogen, chloro, bromo, iodo, nitro, $C_1$—$C_3$ alkyl, hydroxy, $C_1$—$C_3$ alkoxy or $C_1$—$C_3$ alkylthio;

(C) optionally substituted $C_2$—$C_{19}$ alkanoyl, $C_5$—$C_{19}$ alkenoyl, $C_4$—$C_{14}$ alkyl provided that R, $R^1$ and $R^2$ groups together must contain at least four carbon atoms;

and in which $R^3$, $R^4$ and $R^5$ (i) may represent hydrogen or (ii) taken together with an appropriate adjacent R, $R^1$, $R^2$ group may represent a $C_4$—$C_{14}$ alkylidene group; provided that when $R^1$ and $R^2$ are both selected from hydrogen, R cannot be 8-methyldecanoyl, 10-methylundecanoyl, 10-methyldodecanoyl, the specific $C_{10}$-alkanoyl group of A—21978C factor $C_0$ or the specific $C_{12}$-alkanoyl groups of A—21978C factors $C_4$ and $C_5$; or a pharmaceutically-acceptable salt thereof.

2. An A—21978C cyclic peptide derivative of formula (1) as claimed in claim 1 in which R is hydrogen, 8-methyldecanoyl, 10-methyldodecanoyl, 10-methylundecanoyl, the specific $C_{10}$-alkanoyl group of A—21978$C_0$ or the specific $C_{12}$-alkanoyl groups of A—21978C factors $C_4$ and $C_5$, an amino-protecting group, an aminoacyl group of the formula

$$-\overset{\overset{\large O}{\|}}{C}-Q-NH_2$$

wherein Q is $C_1$—$C_{16}$ alkylene, or an N-alkanoylamino acyl group of the formula

$$-W-\overset{\overset{\large O}{\|}}{C}-R^7 \text{ in which:}$$

W is a divalent aminoacyl radical of the formula:

(a) $$-\overset{\overset{\large O}{\|}}{C}-A-NH-$$

in which A is $C_1$—$C_{10}$ alkylene or $C_5$—$C_6$ cycloalkylene;

(b) $$-\overset{\overset{\large O}{\|}}{C}-\overset{\overset{\large R^8}{|}}{CH}-NH-$$

in which $R^8$ is hydroxymethyl, hydroxyethyl, mercaptomethyl, mercaptoethyl, methylthioethyl, 2-thienyl, 3-indole-methyl, phenyl, benzyl, or substituted phenyl or substituted benzyl in which the benzene ring thereof is substituted with chloro, bromo, iodo, nitro, $C_1$—$C_3$ alkyl, hydroxy, $C_1$—$C_3$-alkoxy, $C_1$—$C_3$ alkylthio, carbamyl, or $C_1$—$C_3$ alkylcarbamyl;

(c) 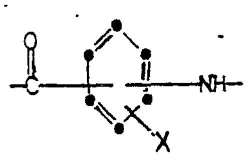

in which X is hydrogen, chloro, bromo, iodo, amino, nitro, $C_1$—$C_3$ alkyl, hydroxy, $C_1$—$C_3$ alkoxy, mercapto, $C_1$—$C_3$ alkylthio, carbamyl, or $C_1$—$C_3$ alkylcarbamyl;

(d) 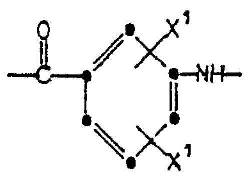 or 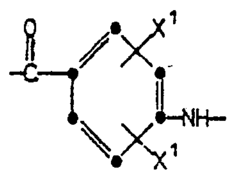

in which $X^1$ is chloro, bromo, iodo, amino, hydroxy, $C_1$—$C_3$ alkyl or $C_1$—$C_3$ alkoxy;

(e) 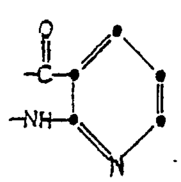 or 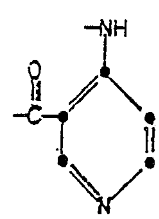 ; or

(f) 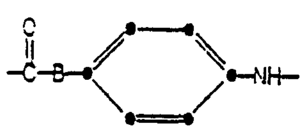

in which B is a divalent radical of the formula: $—(CH_2)_n—$ and n is an integer from 1 to 3; $—CH{=}CH—$; $—CH{=}CH—CH_2—$; or

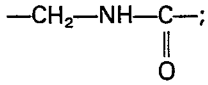

$R^7$ is $C_1$—$C_{17}$ alkyl or $C_2$—$C_{17}$ alkenyl; and $R^2$ is hydrogen, an amino-protecting group, an aminoacyl group of the formula

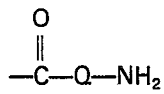

as herein defined, or an N-alkanoylaminoacyl group of the formula

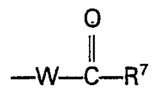

as herein defined; or a pharmaceutically-acceptable salt thereof.

3. A compound according to claim 2 in which $R^1$ and $R^2$ are hydrogen and R is selected from 5-[N-(n-dodecanoyl)amino]-n-pentanoyl, m-[N-(n-dodecanoyl)amino]benzoyl, p-[N-(n-dodecanoyl]amino]cinnamoyl, 2-[N-(n-dodecanoyl)amino]nicotinoyl, or N-(n-decanoyl)-phenylalanyl; or a pharmaceutically-acceptable salt thereof.

4. An A—21978C cyclic peptide of formula (1) as claimed in claim 1 in which R is selected from the group consisting of hydrogen, an amino-protecting group, 8-methyldecanoyl, 10-methylundecanoyl, 10-methyldodecanoyl, the specific $C_{10}$-alkanoyl group of A—21978C factor $C_0$ and the specific $C_{12}$-alkanoyl groups of A—21978C factors $C_4$ and $C_5$; $R^1$ and $R^2$ are, independently, hydrogen or an amino-protecting group; provided that, when R is other than hydrogen or an amino-protecting group, at least one of $R^1$ and $R^2$ must be an amino-protecting group; or a pharmaceutically-acceptable salt thereof.

5. A compound according to claim 4 in which R is hydrogen or a pharmaceutically-acceptable salt thereof.

6. A compound according to claim 5 in which $R^2$ is hydrogen or a pharmaceutically-acceptable salt thereof.

7. A compound according to claim 5 or 6 in which $R^1$ is hydrogen or a pharmaceutically-acceptable salt thereof.

8. A compound according to claim 5 in which $R^2$ is an amino-protecting group or a pharmaceutically-acceptable salt thereof.

9. An A—21978C cyclic peptide derivative of formula (1) as claimed in claim 1 in which R is a substituted benzoyl group of the formula

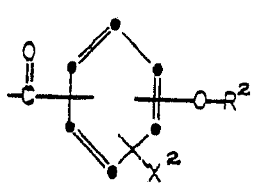

in which $R^6$ is $C_8$—$C_{15}$ alkyl; X is hydrogen, chloro, bromo, iodo, nitro, $C_1$—$C_3$ alkyl, hydroxy, $C_1$—$C_3$ alkoxy, or $C_1$—$C_3$ alkylthio; and $R^2$ is hydrogen or an amino-protecting group; or a pharmaceutically-acceptable salt thereof.

10. An A—21978C cyclic peptide derivative of formula (1) as claimed in claim 1 in which R, $R^1$ and $R^2$ are, independently, hydrogen, $C_4$—$C_{14}$-alkyl, optionally substituted $C_2$—$C_{19}$-alkanoyl, $C_5$—$C_{19}$-alkenoyl or an amino-protecting group, $R^3$, $R^4$ and $R^5$ are all hydrogen, or (i) $R^3$ and $R^1$; and/or (ii) $R^4$ and R; and/or (iii) $R^5$ and $R^2$, taken together may represent a $C_4$—$C_{14}$ alkylidene group, provided that the R, $R^1$ and $R^2$ groups must together contain at least four carbon atoms; or a pharmaceutically-acceptable salt thereof.

11. A compound according to claim 10 in which $R^1$ and $R^2$ are hydrogen and R is selected from 9-tetradecenoyl, 10-undecenoyl, or dodecyl or a pharmaceutically-acceptable salt thereof.

12. A process for preparing an A—21978C cyclic peptide derivative of formula (1) as claimed in any one of claims 2, 3 or 9 which process comprises acylating an A—21978C cyclic peptide factor, the A—21978C nucleus of formula (3),

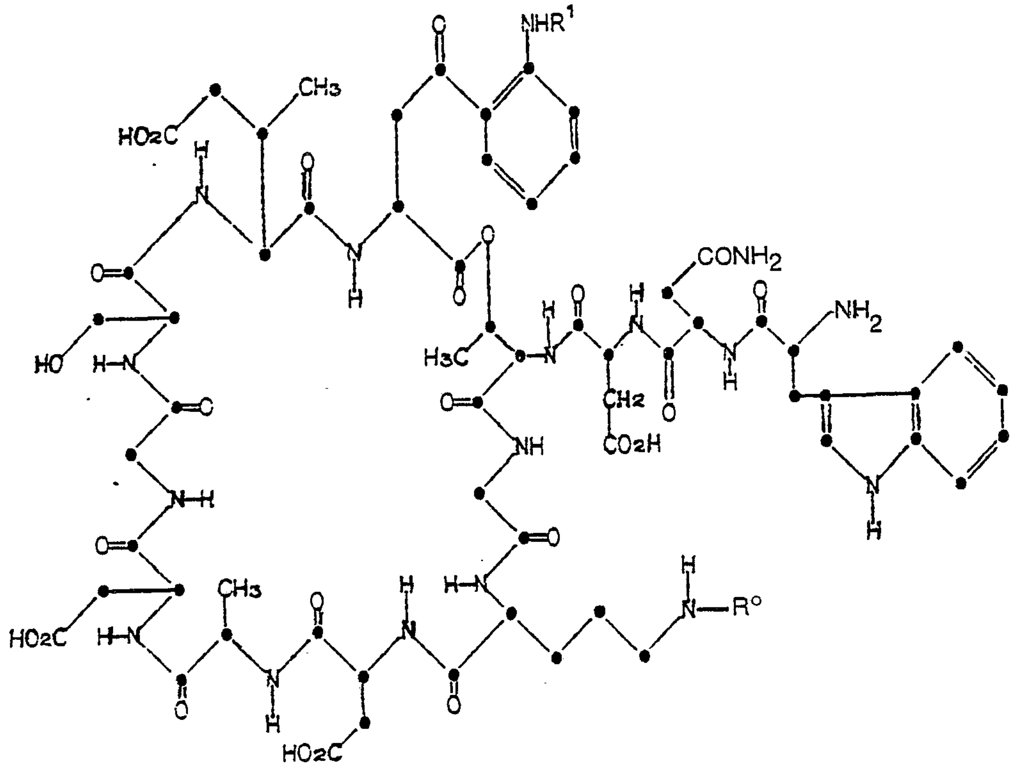

(3)

or an appropriately protected derivative thereof in which R′ or R° are, independently, hydrogen or an amino-protecting group or a pharmaceutically-acceptable salt thereof, with an acylating agent of formula (5)

5 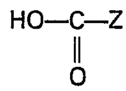

in which Z is —Q—NH$_2$, 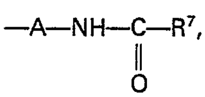, 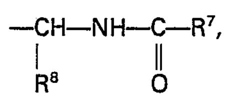, 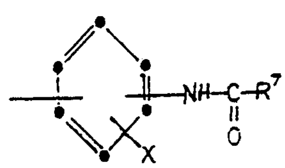,

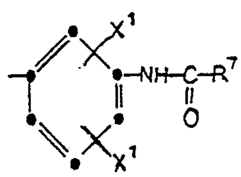 or 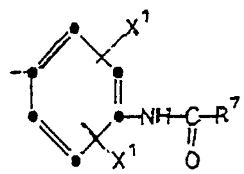, 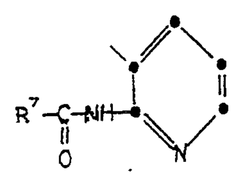

or 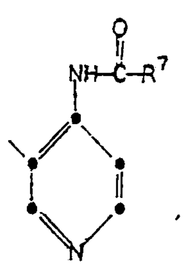, or 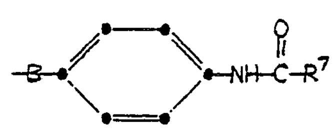, or 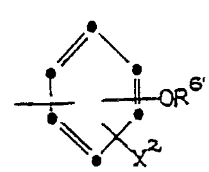

or an activated derivative thereof, in which Q, A, $R^6$, $R^7$, $R^8$, X, $X^1$, $X^2$, and B are as previously defined, and optionally, if desired, removing any protecting group which may be present in the product of the reaction.

13. A process for preparing an A—21978C cyclic peptide as claimed in any of claims 4 to 8 which process comprises

(a) the protection of one or more of the amino groups NHR, $NHR^1$ and $NHR^2$ so as to prepare a compound of formula (1) in which one or more of R, $R^1$ and $R^2$ represent an amino-protection group, provided that one or more of R, $R^1$, and $R^2$ were initially hydrogen, and/or

(b) the deacylation of a compound of formula (1) in which R is other than hydrogen or an amino-protecting group, so as to prepare a compound of formula (1) in which R is hydrogen, and, optionally, if so desired, removing any $R^1$ or $R^2$ amino protecting groups which may be present in the product of the reaction.

14. A process according to claim 13 in which the method of deacylation comprises exposing the compound of formula 1 in an aqueous medium to an enzyme which deacylates and which is produced by a micro-organism of the family *Actinoplanaceae* until substantial deacylation is accomplished.

15. A process according to claim 14 in which the microorganism of the family *Actinoplanaceae* is a member of the genus *Actinoplanes.*

16. A process according to claim 15 in which the microorganism is selected from *A. utahensis* NRRL 12052, *Streptosporangium roseum* var. *hollandensis* NRRL 12064, *Actinoplanes missouriensis* NRRL 12053, *Actinoplanes sp.* NRRL 12065, or *Actinoplanes sp.* NRRL 8122; or a mutant or variant thereof.

17. A process as claimed in any one of claims 14 to 16 in which the enzyme is present in a culture of the producing *Actinoplanaceae* microorganism.

18. A process for preparing an A—21978C cyclic peptide derivative of formula 1 as claimed in claims 10 or 11 which comprises reacting an A—21978C cyclic peptide nucleus of formula 3, as defined in claim 12,

(a) with an acylating agent of formula (7):

$$R^{10}\text{—COOH} \qquad (7)$$

or an activated derivative thereof, in which $R^{10}$ is optionally substituted $C_1$—$C_{18}$ alkyl or $C_4$—$C_{18}$ alkenyl; or

(b) with a carbonyl derivative of formula (8):

$$\begin{array}{c} R^{11}\text{—C—}R^{12} \\ \| \\ \text{O} \end{array} \qquad (8)$$

in which the $R^{11}R^{12}C=$ group represents a $C_4$—$C_{14}$ alkylidene grouping; and

(c) optionally reducing a $C_4$—$C_{14}$ alkylidene product of the above process (b) to form a compound of formula 1 in which R, $R^1$ or $R^2$ represent a $C_4$—$C_{14}$ alkyl group;

(d) and, if desired, removing any protecting group which may be present in the product of any of reactions (a), (b), or (c) above.

19. A pharmaceutical formulation which comprises as an active ingredient an A—21978C derivative of formula (1), or a pharmaceutically-acceptable salt thereof, as claimed in any one of claims 1 to 11, associated with one or more physiologically-acceptable carriers or vehicles therefor.

20. An A—21978C derivative of formula (1), or a pharmaceutically-acceptable salt thereof, as claimed in any one of claims 1 to 11, for use as an antibiotic.

21. A compound according to claim 1 which is $N_{Trp}$-(n-decanoyl)A—21978C nucleus.

22. Purified $N_{Trp}$-(m-decanoyl)A—21978C nucleus or a pharmaceutially-acceptable salt thereof.

**Patentansprüche**

1. Derivat des Cyclopeptids A—21978C der Formel (1)

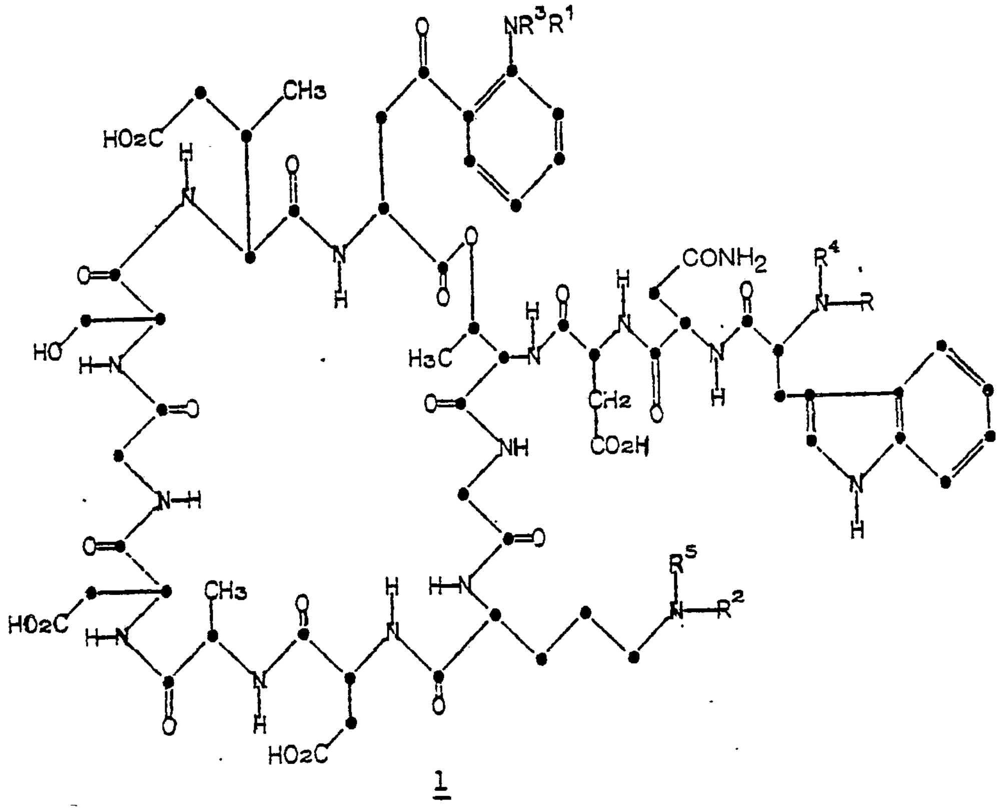

1

worin R, $R^1$ und $R^2$ unabhängig für Wasserstoff, 8-Methyldecanoyl, 10-Methyldodecanoyl, 10-Methylundecanoyl, die spezifische $C_{10}$-Alkanoylgruppe von A—21978$C_0$, die spezifische $C_{12}$-Alkanoylgruppe der Faktoren $C_4$ oder $C_5$ von A—21978C, eine Aminoschutzgruppe oder folgende Gruppen stehen,

(A) eine Aminoacylgruppe der Formel

$$-\overset{\overset{\displaystyle O}{\|}}{C}-Q-NH_2$$

worin Q für $C_1$ bis $C_{16}$-Alkylen oder eine N-Alkanoylaminoacrylgruppe der Formel

$$-W-\overset{\overset{\displaystyle O}{\|}}{C}-R^7$$

steht, worin W ein zweiwertiger Aminoacylrest der folgenden Formeln ist:

(1) $$-\overset{\overset{\displaystyle O}{\|}}{C}-A-NH-$$

worin A für $C_1$ bis $C_{10}$-Alkylen oder $C_5$ bis $C_6$-Cycloalkylen steht,

(2) $$-\overset{\overset{\displaystyle O}{\|}}{C}-\overset{\overset{\displaystyle R^8}{|}}{CH}-NH-$$

worin $R^8$ für Hydroxymethyl, Hydroxyethyl, Mercaptomethyl, Mercaptoethyl, Methylthioethyl, 2-Thienyl, 3-Indolmethyl, Phenyl, Benzyl oder substituiertes Phenyl oder substituiertes Benzyl steht, worin der

Benzylring durch Chlor, Brom, Iod, Nitro, $C_1$ bis $C_3$-Alkyl, Hydroxy, $C_1$ bis $C_3$-Alkoxy, $C_1$ bis $C_3$-Alkylthio, Carbamyl oder $C_1$ bis $C_3$-Alkylcarbamyl substituiert ist,

(3) 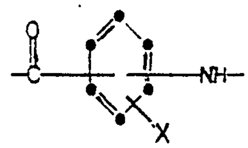

worin X Wasserstoff, Chlor, Brom, Iod, Amino, Nitro, $C_1$ bis $C_3$-Alkyl, Hydroxy, $C_1$ bis $C_3$-Alkoxy, Mercapto, $C_1$ bis $C_3$-Alkylthio, Carbamyl oder $C_1$ bis $C_3$-Alkylcarbamyl ist,

(4) 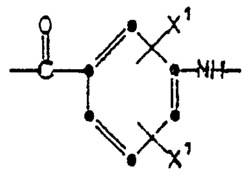 or 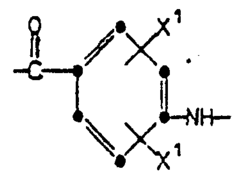

worin $X^1$ Chlor, Brom, Iod, Amino, Hydroxy, $C_1$ bis $C_3$-Alkyl oder $C_1$ bis $C_3$-Alkoxy bedeutet,

(5) 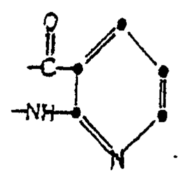 oder 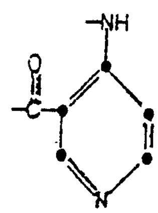 ; oder

(6) 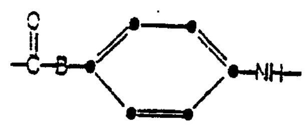

worin B einen zweiwertigen Rest mit den Formeln $-(CH_2)_n-$, wobei n für eine ganze Zahl von 1 bis 3 steht, $-CH=CH-$, $-CH=CH-CH_2-$ oder

$$-CH_2-NH-\underset{\underset{O}{\|}}{C}-$$

bedeutet und

$R^7$ für $C_1$ bis $C_{17}$-Alkyl oder $C_2$ bis $C_{17}$-Alkenyl steht

(B) eine substituierte Benzoylgruppe der Formel

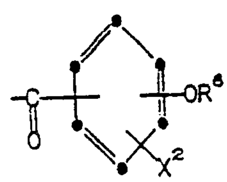

worin $R^6$ für $C_8$ bis $C_{15}$-Alkyl steht und $X^2$ Wasserstoff, Chlor, Brom, Iod, Nitro, $C_1$ bis $C_3$-Alkyl, Hydroxy, $C_1$ bis $C_3$-Alkoxy oder $C_1$ bis $C_3$-Alkylthio ist, oder

(C) gegebenenfalls substituiertes $C_2$ bis $C_{19}$-Alkanoyl, $C_5$ bis $C_{19}$-Alkenoyl oder $C_4$ bis $C_{14}$-Alkyl, mit der Maßgabe, daß die Gruppen R, $R^1$ und $R^2$ zusammen wenigstens 4 Kohlenstoffatome enthalten müssen, und worin $R^3$, $R^4$ und $R^5$ entweder (1) Wasserstoff sein können oder (2) zusammen mit einer geeigneten benachbarten Gruppe, R, $R^1$ oder $R^2$ für eine $C_4$ bis $C_{14}$-Alkylidengruppe stehen können, mit der Maßgabe, daß R nicht 8-Methyldecanoyl, 10-Methylundecanoyl, 10-Methyldodecanoyl, die

spezifische $C_{10}$-Alkanoylgruppe des Faktors $C_0$ von A—21978C oder die spezifische $C_{12}$-Alkanoylgruppe der Faktoren $C_4$ und $C_5$ von A—21978C sein kann, falls $R^1$ und $R^2$ jeweils zugleich Wasserstoff sind, oder ein pharmazeutisch annehmbares Salz hiervon.

2. Derivat des Cyclopeptids A—21978C der Formel (1) nach Anspruch 1, dadurch gekennzeichnet, daß R für Wasserstoff, 8-Methyldecanoyl, 10-Methyldodecanoyl, 10-Methylundecanoyl, die spezifische $C_{10}$-Alkanoylgruppe von A—21978$C_0$ oder die spezifische $C_{12}$-Alkanoylgruppe der Faktoren $C_4$ und $C_5$ von A—21978C, eine Aminoschutzgruppe, eine Aminoacylgruppe der Formel

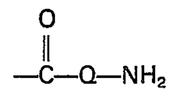

worin Q für $C_1$ bis $C_{16}$-Alkylen steht, oder eine N-alkanoylaminoacylgruppe der Formel

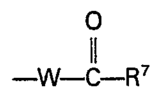

steht, worin

W ein zweiwertiger Aminoacylrest der folgenden Formeln ist:

(a) 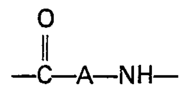

worin A für $C_1$ bis $C_{10}$-Alkylen oder $C_5$ bis $C_6$-Cycloalkylen steht,

(b) 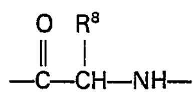

worin $R^8$ für Hydroxymethyl, Hydroxyethyl, Mercaptomethyl, Mercaptoethyl, Methylthioethyl, 2-Thienyl, 3-Indolmethyl, Phenyl, Benzyl oder substituiertes Phenyl oder substituiertes Benzyl steht, worin der Benzylring durch Chlor, Brom, Iod, Nitro, $C_1$ bis $C_3$-Alkyl, Hydroxy, $C_1$ bis $C_3$-Alkoxy, $C_1$ bis $C_3$-Alkylthio, Carbamyl oder $C_1$ bis $C_3$-Alkylcarbamyl substituiert ist,

(c) 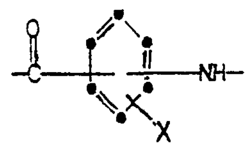

worin X Wasserstoff, Chlor, Brom, Iod, Amino, Nitro, $C_1$ bis $C_3$-Alkyl, Hydroxy, $C_1$ bis $C_3$-Alkoxy, Mercapto, $C_1$ bis $C_3$-Alkylthio, Carbamyl oder $C_1$ bis $C_3$-Alkylcarbamyl ist,

(d) 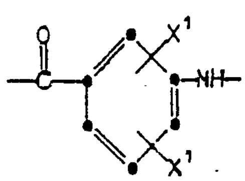 oder 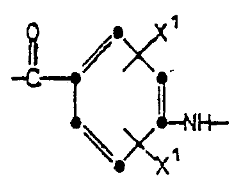

worin $X^1$ Chlor, Brom, Iod, Amino, Hydroxy, $C_1$ bis $C_3$-Alkyl oder $C_1$ bis $C_3$-Alkoxy bedeutet,

(e) 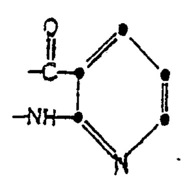 oder 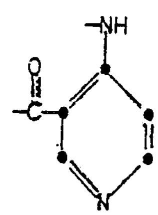 ; oder

(f) 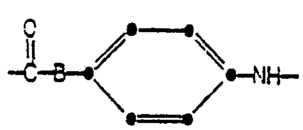

worin B einen zweiwertigen Rest mit den Formeln $—(CH_2)_n—$, wobei n für eine ganze Zahl von 1 bis 3 steht, $—CH{=}CH—$, $—CH{=}CH—CH_2—$ oder

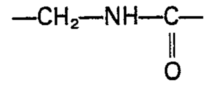

bedeutet, und

$R^7$ für $C_1$ bis $C_{17}$-Alkyl oder $C_2$ bis $C_{17}$-Alkenyl steht, und

$R^2$ Wasserstoff, eine Aminoschutzgruppe, eine Aminoacylgruppe der Formel

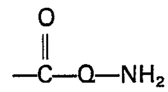

gemäß obiger Definition oder eine N-Alkanoylaminoacylgruppe der Formel

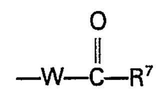

gemäß obiger Definition ist,

oder ein pharmazeutisch annehmbares Salz hiervon.

3. Verbindung nach Anspruch 2, dadurch gekennzeichnet, daß $R^1$ und $R^2$ Wasserstoff sind und R ausgewählt ist aus 5-[N-(n-Dodecanoyl)amino]-n-pentanoyl, m-[N-(n-Dodecanoyl)amino]benzoyl, p-[N-(n-Dodecanoyl)amino]cinnamoyl, 2-[N-(n-Dodecanoyl)amino]nicotinoyl oder N-(n-Decanoyl)-phenylalanyl oder ein pharmazeutisch annehmbares Salz hiervon.

4. Derivat des Cyclopeptids A—21978C der Formel (1) nach Anspruch 1, dadurch gekennzeichnet, daß R für Wasserstoff eine Aminoschutzgruppe, 8-Methyldecanoyl, 10-Methylundecanoyl, 10-Methyldodecanoyl, die spezifische $C_{10}$-Alkanoylgruppe des Faktors $C_0$ von A—21978C oder die spezifische $C_{12}$-Alkanoylgruppe der Faktoren $C_4$ und $C_5$ von A—21978C steht, $R^1$ und $R^2$ unabhängig Wasserstoff oder eine Aminoschutzgruppe sind, mit der Maßgabe, daß wenigstens einer der Substituenten $R^1$ und $R^2$ eine Aminoschutzgruppe sein muß, falls R etwas anderes als Wasserstoff oder eine Aminoschutzgruppe ist, oder ein pharmazeutisch annehmbares Salz hiervon.

5. Verbindung nach Anspruch 4, dadurch gekennzeichnet, daß R Wasserstoff ist, oder ein pharmazeutisch annehmbares Salz hiervon.

6. Verbindung nach Anspruch 5, dadurch gekennzeichnet, daß $R^2$ Wasserstoff ist, oder ein pharmazeutisch annehmbares Salz hiervon.

7. Verbindung nach Anspruch 5 oder 6, dadurch gekennzeichnet, daß $R^1$ Wasserstoff ist, oder ein pharmazeutisch annehmbares Salz hiervon.

8. Verbindung nach Anspruch 5, dadurch gekennzeichnet, daß $R^2$ Aminoschutzgruppe ist, oder ein pharmazeutisch annehmbares Salz hiervon.

9. Derivat des Cyclopeptids A—21978C der Formel (1) nach Anspruch 1, dadurch gekennzeichnet, daß R eine substituierte Benzoylgruppe der Formel

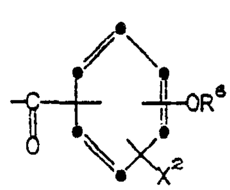

ist, worin $R^6$ für $C_8$ bis $C_{15}$-Alkyl steht, X für Wasserstoff, Chlor, Brom, Iod, Nitro, $C_1$ bis $C_3$-Alkyl, Hydroxy, $C_1$ bis $C_3$-Alkoxy oder $C_1$ bis $C_3$-Alkylthio steht und $R^2$ Wasserstoff oder eine Aminoschutzgruppe ist, oder ein pharmazeutisch annehmbares Salz hiervon.

10. Derivat des Cyclopeptids A—21978A der Formel (1) nach Anspruch 1, dadurch gekennzeichnet, daß R, $R^1$ und $R^2$ unabhängig für Wasserstoff, $C_4$ bis $C_{14}$-Alkyl, gegebenenfalls substituiertes $C_2$ bis $C_{19}$-Alkanoyl, $C_5$ bis $C_{19}$-Alkenoyl oder eine Aminoschutzgruppe stehen, $R^3$, $R^4$ und $R^5$ insgesamt Wasserstoff sind, oder (1) $R^3$ und $R^1$ und/oder (2) $R^4$ und R und/oder (3) $R^5$ und $R^2$ zusammen eine $C_4$ bis $C_{14}$-Alkylidengruppe bilden, mit der Maßgabe, daß die Gruppen R, $R^1$ und $R^2$ zusammen wenigstens 4 Kohlenstoffatome enthalten müssen, oder ein pharmazeutisch annehmbares Salz hiervon.

11. Verbindung nach Anspruch 10, dadurch gekennzeichnet, daß $R^1$ und $R^2$ Wasserstoff sind und R für 9-Tetradecenoyl, 10-Undecenoyl oder Dodecyl steht, oder ein pharmazeutisch annehmbares Salz hiervon.

12. Verbindung nach Herstellung eines Derivats des Cyclopeptids A—21978C der Formel (1) nach irgendeinem der Ansprüche 2, 3 oder 9, dadurch gekennzeichnet, daß man einen Faktor des Cyclopeptids A—21978C, den Kern von A—21978C der Formel (3)

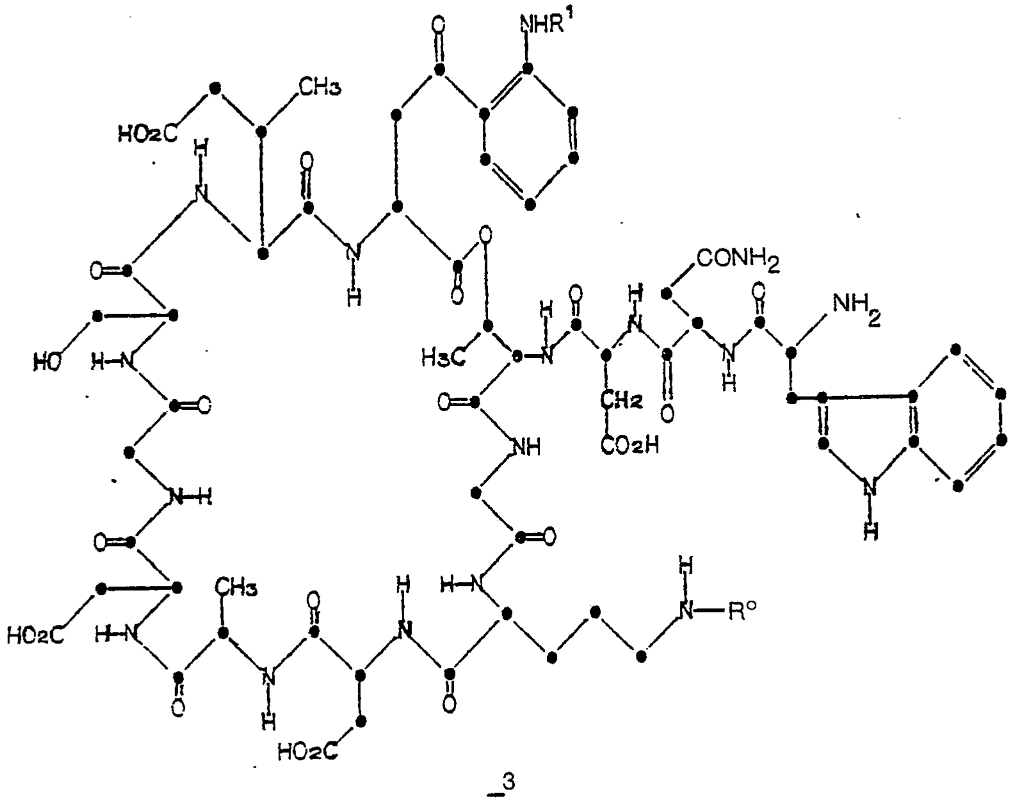

3

oder ein geeignet geschütztes Derivat hiervon, worin R' oder $R^0$ unabhängig Wasserstoff oder eine Aminoschutzgruppe sind, oder ein pharmazeutisch annehmbares Salz hiervon, mit einem Acylierungsmittel der Formel (5)

$$HO-\underset{\underset{O}{\|}}{C}-Z \qquad 5$$

worin Z für $-Q-NH_2$, $-A-NH-\underset{\underset{O}{\|}}{C}-R^7$, $-\underset{\underset{R^8}{|}}{CH}-NH-\underset{\underset{O}{\|}}{C}-R^7$ 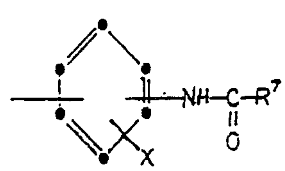 ,

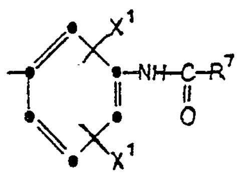 oder 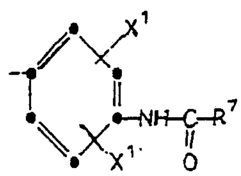 , 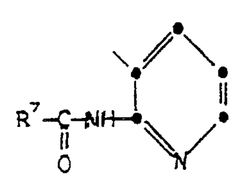

oder 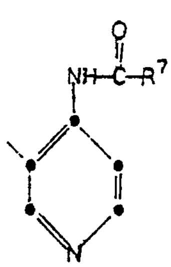 , oder 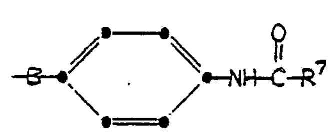 , oder 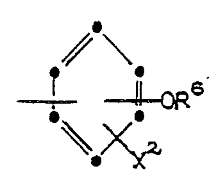

steht, oder ein aktiviertes Derivat hiervon, worin Q, A, $R^6$, $R^7$, $R^8$, X, $X^1$, $X^2$ und B wie oben angegeben definiert sind, acyliert und gegebenenfalls irgendwelche Schutzgruppen, die im Reaktionsprodukt vorhanden sein können, entfernt.

13. Verfahren zur Herstellung eines Derivats des Cyclopeptide A—21978C nach irgendeinem der Ansprüche 4 bis 8, dadurch gekennzeichnet, daß man

(a) eine oder mehrere der Aminogruppen, NHR, $NHR^1$ und $NHR^2$ schützt und so eine Verbindung der Formel (1) herstellt, worin ein oder mehr der Substituenten R, $R^1$ und $R^2$ eine Aminoschutzgruppe bedeuten, sofern ein oder mehr Substituenten R, $R^1$ und $R^2$ ursprünglich Wasserstoff waren, und/oder

(b) eine Verbindung der Formel (1), worin R etwas anderes als Wasserstoff oder eine Aminoschutzgruppe ist, deacyliert und so eine Verbindung der Formel (1) herstellt, worin R Wasserstoff ist, und gegebenenfalls irgendwelche Aminoschutzgruppen $R^1$ oder $R^2$, die im Reaktionsprodukt vorhanden sein können, entfernt.

14. Verfahren nach Anspruch 13, dadurch gekennzeichnet, daß man die Deacylierung durchführt, indem man auf die Verbindung der Formel (1) in einem wäßrigen Medium solange ein von einem Mikroorganismus aus der Familie der Actinoplanaceae gebildetes Deacylierungsenzym einwirken läßt, bis die Deacylierung praktisch vollständig ist.

15. Verfahren nach Anspruch 14, dadurch gekennzeichnet, daß der Mikroorganismus aus der Familie der Actinoplanaceae ein Vertreter der Gattung Actinoplanes ist.

16. Verfahren nach Anspruch 15, dadurch gekennzeichnet, daß der Mikroorganismus Actiniplanes utahensis NRRL 12052, Streptosporangium roseum var. hollandensis NRRL 12064, Acinoplanes missouriensis NRRL 12053, Actinoplanes sp. NRRL 12065 und/oder Actinoplanes sp. NRRL 8122 oder eine Mutante oder Variante hiervon ist.

17. Verfahren nach irgendeinem der Ansprüche 14 bis 16, dadurch gekennzeichnet, daß das Enzym in einer Kultur des Actinoplanaceae produzierenden Mikroorganismus vorhanden ist.

18. Verfahren zur Herstellung eines Derivats des Cyclopeptids A—21978C der Formel (1) nach Anspruch 10 oder 11, dadurch gekennzeichnet, daß man einen Kern des Cyclopeptids A—21978C der in Anspruch 12 angegebenen Formel (3) umsetzt mit

(a) einem Acylierungsmittel der Formel (7)

$$R^{10}\text{—COOH} \qquad (7)$$

oder einem aktivierten Derivat hiervon, worin $R^{10}$ für gegebenenfalls substituiertes $C_1$ bis $C_{18}$-Alkyl oder $C_4$ bis $C_{18}$-Alkenyl steht, oder

(b) mit einem Carbonylderivat der Formel (8)

$$\begin{array}{c} R^{11}\text{—C—}R^{12} \\ \| \\ O \end{array} \qquad (8)$$

worin die Gruppe $R^{11}R^{12}C=$ für eine $C_4$ bis $C_{14}$-Alkylidengruppe steht, und

(c) ein nach obigem Verfahren (b) erhaltenes $C_4$ bis $C_{14}$-Alkylidenprodukt durch Reduktion gegebenenfalls in eine Verbindung der Formel (1) überführt, worin R, $R^1$ oder $R^2$ für eine $C_4$ bis $C_{14}$-Alkylgruppe steht, und

(d) irgendwelche Schutzgruppen, die in einem nach den obigen Verfahren (a), (b) oder (c) erhaltenen Produkten vorhanden sein können, gewünschtenfalls entfernt.

19. Pharmazeutische Formulierung, dadurch gekennzeichnet, daß sie ein Derivat des Cyclopeptids A—21978C der Formel (1) oder ein pharmazeutisch annehmbares Salz hiervon nach irgendeinem der Ansprüche 1 bis 11 als Wirkstoff in Verbindung mit einem oder mehreren physiologisch annehmbaren Trägern oder Hilfsstoffen hierfür enthält.

20. Verwendung eines Derivats des Cyclopeptids A—21978C der Formel (1) oder eines pharmazeutisch annehmbaren Salzes hiervon nach irgendeinem der Ansprüche 1 bis 11 als Antibiotikum.

21. Verbindung nach Anspruch 1, dadurch gekennzeichnet, daß es sich hierbei um $N_{Trp}$-(n-Decanoyl)-A—21978C-Kern handelt.

22. Gereinigter $N_{Trp}$-(n)-Decanoyl)-A-21978C-Kern oder ein pharmazeutisch annehmbares Salz hiervon.

**Revendications**

1. Dérivé de peptide cyclique A—21978C de formule (1):

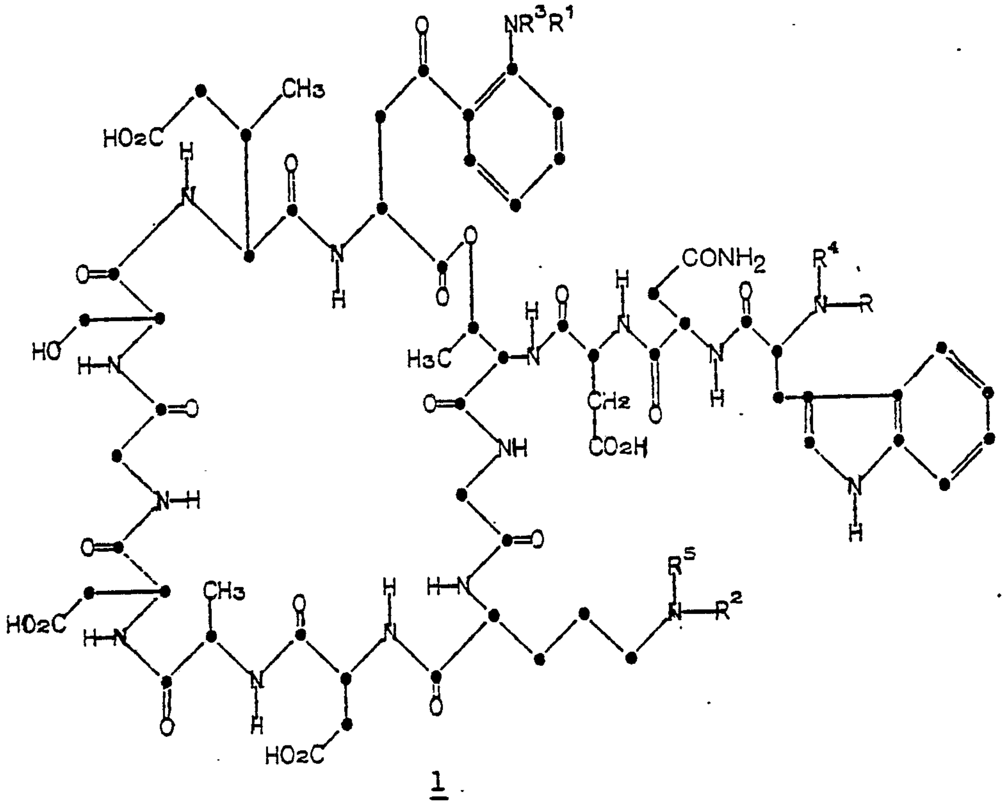

1

dans laquelle R, $R^1$ et $R^2$ représentent chacun indépendamment l'un de l'autre un atome d'hydrogène, un groupe 8-méthyldécanoyle, un groupe 10-méthyldodécanoyl, un groupe 10-méthylundécanoyle, le groupe $C_{10}$-alcanoyle spécifique d'A—21978$C_0$, les groupes $C_{12}$-alcanoyle spécifiques d'A—21978C, facteurs $C_4$ ou $C_5$, un groupe protecteur du groupe amino,

(A) un groupe amino-acyle de formule:

—C(=O)—Q—NH$_2$

dans laquelle Q représente un groupe alkylène en $C_1$—$C_{16}$ ou un groupe N-alcanoylamino-acyle de formule:

—W—C(=O)—$R^7$

dans laquelle W est un radical aminoacyle bivalent de formule:

(1) —C(=O)—A—NH—

où A représente un groupe alkylène en $C_1$—$C_{10}$ ou un groupe cycloalkylène en $C_5$—$C_6$;

(2) —C(=O)—CH($R^8$)—NH—

où $R^8$ représente un groupe hydroxyméthyle, un groupe hydroxyéthyle, un groupe mercaptométhyle, un groupe mercaptoéthyle, un groupe méthylthioéthyle, un groupe 2-thiényle, un groupe 3-indole-méthyle, un groupe phényle, un groupe benzyle ou un groupe phényle substitué ou encore un groupe benzyle substitué dans lequel le noyau benzène est substitué par un atome de chlore, un atome de brome, un atome d'iode, un groupe nitro, un groupe alkyle en $C_1$—$C_3$, un groupe hydroxy, un groupe alcoxy en $C_1$—$C_3$, un groupe alkyl(en $C_1$—$C_3$)thio, un groupe carbamyle ou un groupe alkyl(en $C_1$—$C_3$)carbamyle;

(3) 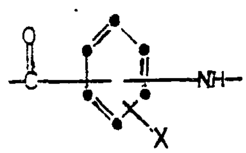

où X représente un atome d'hydrogène, un atome de chlore, un atome de brome, un atome d'iode, un groupe amino, un groupe nitro, un groupe alkyle en $C_1$—$C_3$, un groupe hydroxy, un groupe alcoxy en $C_1$—$C_3$, un groupe mercapto, un groupe alkyl(en $C_1$—$C_3$)thio, un groupe carbamyle ou un groupe alkyl(en $C_1$—$C_3$)carbamyle;

(4) 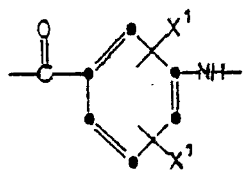 ou 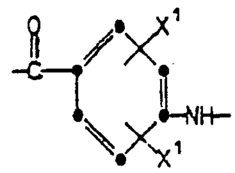

où $X^1$ représente un atome de chlore, un atome de brome, un atome d'iode, un groupe amino, un groupe hydroxy, un groupe alkyle en $C_1$—$C_3$ ou un groupe alcoxy en $C_1$—$C_3$;

(5) 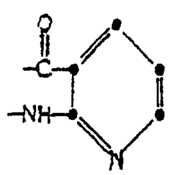 ou 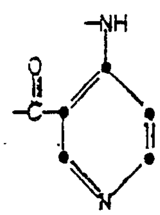 ; ou

(6) 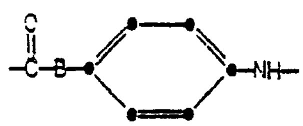

où B représente un radical bivalent de formule $-(CH_2)_n-$, et n est un nombre entier de 1 à 3; $-CH=CH-$, $-CH=CH-CH_2-$ ou

$$-CH_2-NH-\underset{\underset{O}{\|}}{C}-$$

$R^7$ représente un groupe alkyle en $C_1$—$C_{17}$ ou un groupe alcényle en $C_2$—$C_{17}$;

(B) un groupe benzoyle substitué de formule:

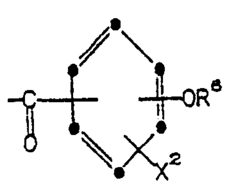

dans laquelle $R^6$ représente un groupe alkyle en $C_8$—$C_{15}$;

$X^2$ représente un atome d'hydrogène, un atome de chlore, un atome de brome, un atome d'iode, un groupe nitro, un groupe alkyle en $C_1$—$C_3$, un groupe hydroxy, un groupe alcoxy en $C_1$—$C_3$ ou un groupe alkyl(en $C_1$—$C_3$)thio;

(C) un groupe alkyle en $C_4$—$C_{14}$, un groupe alcénoyle en $C_5$—$C_{19}$ ou un groupe alcanoyle en $C_2$—$C_{19}$ éventuellement substitué, avec cette réserve que R, $R^1$ et $R^2$ ensemble doivent contenir au moins 4 atomes de carbone;

tandis que $R^3$, $R^4$ et $R^5$ (i) peuvent représenter chacun un atome d'hydrogène ou (ii) pris ensemble avec un groupe R, $R^1$, $R^2$ adjacent approprié, ils peuvent représenter un groupe alcylidène en $C_4$—$C_{14}$, avec cette réserve que, lorsque $R^1$ et $R^2$ représentent chacun un atome d'hydrogène, R ne peut être un groupe 8-méthyldécanoyle, un groupe 10-méthylundécanoyle, un groupe 10-méthyldodécanoyle, le groupe $C_{10}$-alcanoyle spécifique d'A—21978C, facteur $C_0$ ou les groupes $C_{12}$-alcanoyle spécifiques d'A—21978C, facteurs $C_4$ et $C_5$; ou un de ses sels pharmaceutiquement acceptables.

2. Dérivé de peptide cyclique A—21978C de formule (1) selon la revendication 1, dans lequel R est un atome d'hydrogène, un groupe 8-méthyldécanoyle, un groupe 10-méthyldodécanoyle, un groupe 10-méthylundécanoyle, le groupe $C_{10}$-alcanoyle spécifique d'A—21978$C_0$ ou les groupes $C_{12}$-alcanoyle spécifiques d'A—21978C, facteurs $C_4$ et $C_5$, un groupe protecteur du groupe amino, un groupe aminoacyle de formule:

$$-\overset{O}{\overset{\|}{C}}-Q-NH_2$$

dans laquelle Q représente un groupe alkylène en $C_1$—$C_{16}$ ou un groupe N-alcanoylamino-acyle de formule

$$-W-\overset{O}{\overset{\|}{C}}-R^7$$

dans laquelle

W représente un radical aminoacyle bivalent de formule:

(a) $$-\overset{O}{\overset{\|}{C}}-A-NH-$$

où A représente un groupe alkylène en $C_1$—$C_{10}$ ou un groupe cycloalkylène en $C_5$—$C_6$;

(b) $$-\overset{O}{\overset{\|}{C}}-\overset{R^8}{\overset{|}{C}H}-NH-$$

où $R^8$ représente un groupe hydroxyméthyle, un groupe hydroxyéthyle, un groupe mercaptométhyle, un groupe mercaptoéthyle, un groupe méthylthioéthyle, un groupe 2-thiényle, un groupe 3-indole-méthyle, un groupe phényle, un groupe benzyle ou un groupe phényle substitué ou encore un groupe benzyle substitué dans lequel le noyau benzène est substitué par un atome de chlore, un atome de brome, un atome d'iode, un groupe nitro, un groupe alkyle en $C_1$—$C_3$, un groupe hydroxy, un groupe alcoxy en $C_1$—$C_3$, un groupe alkyl(en $C_1$—$C_3$)thio, un groupe carbamyle ou un groupe alkyle(en $C_1$—$C_3$)carbamyle;

(c) 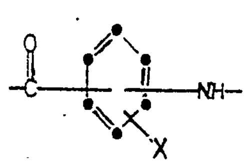

où X représente un atome d'hydrogène, un atome de chlore, un atome de brome, un atome d'iode, un groupe amino, un groupe nitro, un groupe alkyle en $C_1$—$C_3$, un groupe hydroxy, un groupe alcoxy en $C_1$—$C_3$, un groupe mercapto, un groupe alkyl(en $C_1$—$C_3$)thio, un groupe carbamyle ou un groupe alkyl(en $C_1$—$C_3$)carbamyle;

(d) 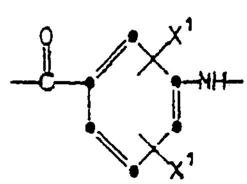 ou 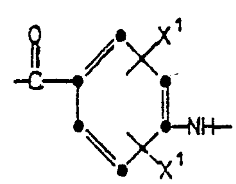

où $X^1$ représente un atome de chlore, un atome de brome, un atome d'iode, un groupe amino, un groupe hydroxy, un groupe alkyle en $C_1—C_3$ ou un groupe alcoxy en $C_1—C_3$;

(e) 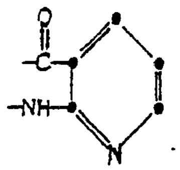 ou 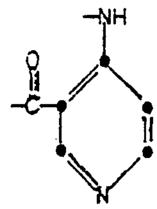 ; ou

(f) 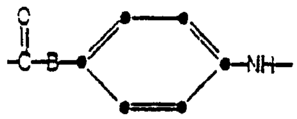

où B représente un radical bivalent de formule $—(CH_2)_n—$ et n est un nombre entier de 1 à 3; $—CH{=}CH—$; $—CH{=}CH—CH_2—$; ou

$$—CH_2—NH—\underset{\underset{O}{\|}}{C}—$$

$R^7$ représente un groupe alkyle en $C_1—C_{17}$ ou un groupe alcényle en $C_2—C_{17}$; et

$R^2$ représente un atome d'hydrogène, un groupe protecteur du groupe amino, un groupe aminoacyle de formule

$$—\overset{\overset{O}{\|}}{C}—Q—NH_2$$

comme défini ci-dessus, ou un groupe N-alcanoylaminoacyle de formule:

$$—W—\overset{\overset{O}{\|}}{C}—R^7$$

comme défini ci-dessus; ou un de ses sels pharmaceutiquement acceptables.

3. Composé selon la revendication 2, dans lequel $R^1$ et $R^2$ représentent chacun un atome d'hydrogène et R est choisi parmi le groupe 5-[N-(n-dodécanoyl)amino]-n-pentanoyle, le groupe m-[N-(n-dodécanoyl)amino]benzoyle, le groupe p-[N-(n-dodécanoyl)amino]cinnamoyle, le groupe 2-[N-(n-dodécanoyl)amino]nicotinoyle ou le groupe N-(n-décanoyl)phénylalanyle; ou un de ses sels pharmaceutiquement acceptables.

4. Peptide cyclique A—21978C de formule (1) selon la revendication 1, dans lequel R est choisi parmi le groupe comprenant un atome d'hydrogène, un groupe protecteur du groupe amino, un groupe 8-méthyldécanoyle, un groupe 10-méthylundécanoyle, un groupe 10-méthyldodécanoyle, le groupe $C_{10}$-alcanoyle spécifique d'A—21978C, facteur $C_0$ et les groupes $C_{12}$-alcanoyle spécifiques d'A—21978C, facteurs $C_4$ et $C_5$; $R^1$ et $R^2$ représentent chacun indépendamment l'un de l'autre un atome d'hydrogène ou un groupe protecteur du groupe amino, avec cette réserve que, lorsque R est différent de l'hydrogène ou d'un groupe protecteur du groupe amino, au moins un des radicaux $R^1$ et $R^2$ doit être un groupe protecteur du groupe amino; ou un de ses sels pharmaceutiquement acceptables.

5. Composé selon la revendication 4, dans lequel R est un atome d'hydrogène, ou un sel pharmaceutiquement acceptable de ce composé.

6. Composé selon la revendication 5, dans lequel $R^2$ est un atome d'hydrogène, ou un sel pharmaceutiquement acceptable de ce composé.

7. Composé selon la revendication 5 ou 6, dans lequel $R^1$ est un atome d'hydrogène, ou un sel pharmaceutiquement acceptable de ce composé.

8. Composé selon la revendication 5, dans lequel $R^2$ est un groupe protecteur du groupe amino, ou un sel pharmaceutiquement acceptable de ce composé.

9. Dérivé de peptide cyclique A—21978C de formule (1) selon la revendication 1, dans lequel R est un groupe benzoyle substitué de formule:

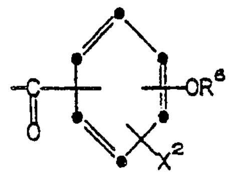

dans laquelle $R^6$ est un groupe alkyle en $C_8$—$C_{15}$; X est un atome d'hydrogène, un atome de chlore, un atome de brome, un atome d'iode, un groupe nitro, un groupe alkyle en $C_1$—$C_3$, un groupe hydroxy, un groupe alcoxy en $C_1$—$C_3$ ou un groupe alkyl(en $C_1$—$C_3$)thio et $R^2$ est un atome d'hydrogène ou un groupe protecteur du groupe amino; ou un de ses sels pharmaceutiquement acceptables.

10. Dérivé de peptide cyclique A—21978C de formule (1) selon la revendication 1, dans lequel R, $R^1$ et $R^2$ représentent chacun indépendamment l'un de l'autre un atome d'hydrogène, un groupe alkyle en $C_4$—$C_{14}$, un groupe protecteur du groupe amino, un groupe alcénoyle en $C_5$—$C_{19}$ ou un groupe alcanoyle en $C_2$—$C_{19}$ éventuellement substitué, $R^3$, $R^4$ et $R^5$ représentent chacun un atome d'hydrogène ou (i) $R^3$ et $R^1$ et/ou (ii) $R^4$ et R et/ou (iii) $R^5$ et $R^2$ pris ensemble peuvent représenter un groupe alcylidène en $C_4$—$C_{14}$, avec cette réserve que les groupes R, $R^1$ et $R^2$ doivent contenir ensemble au moins 4 atomes de carbone; ou un de ses sels pharmaceutiquement acceptables.

11. Composé selon la revendication 10, dans lequel $R^1$ et $R^2$ représentent chacun un atome d'hydrogène et R est choisi parmi un groupe 9-tétradécénoyle, un groupe 10-undécénoyle ou un groupe dodécyle, ou encore un sel pharmaceutiquement acceptable de ce composé.

12. Procédé de préparation d'un dérivé de peptide cyclique A—21978C de formule (1) selon l'une quelconque des revendications 2, 3 ou 9, caractérisé en ce qu'il consiste à acyler un facteur du peptide cyclique A—21978C, le noyau A—21978C de formule (3):

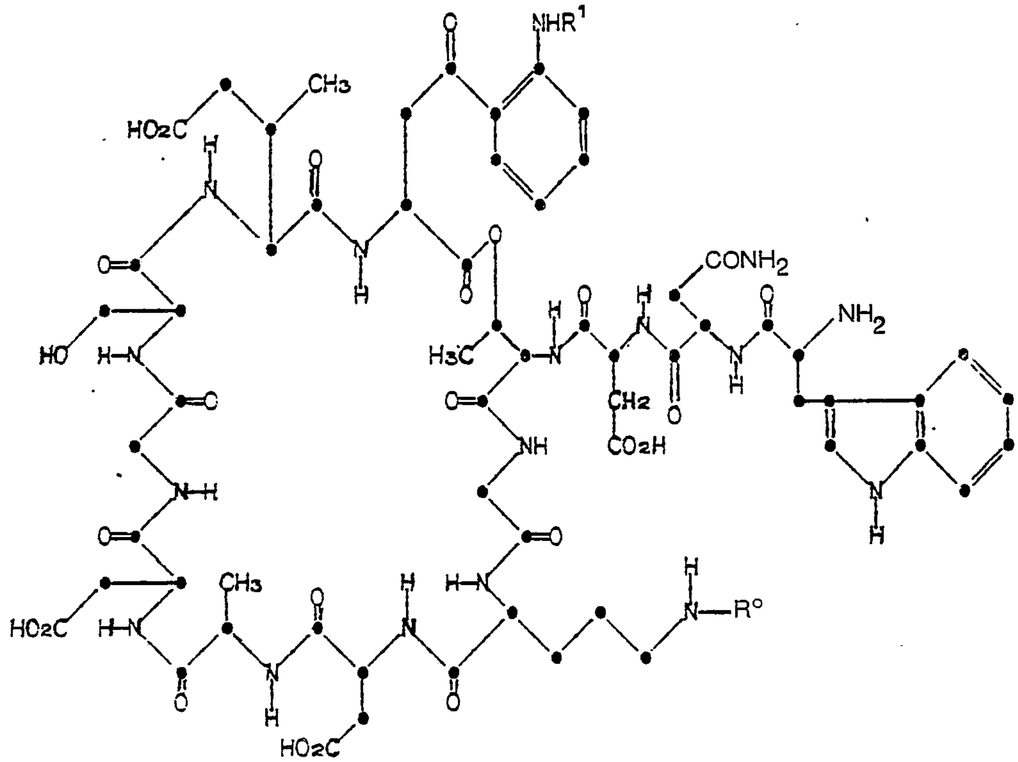

3

ou un de ses dérivés protégés de manière appropriée ou R' et R° représentent chacun indépendamment l'un de l'autre un atome d'hydrogène ou un groupe protecteur du groupe amino, ou encore un de ses sels pharmaceutiquement acceptables, avec un agent d'acylation de formule (5):

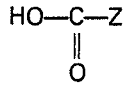

dans laquelle Z représente $—Q—NH_2$,

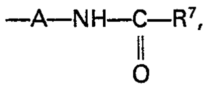, 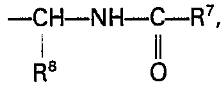,

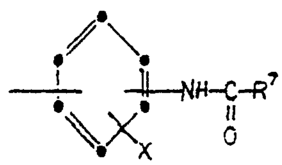,

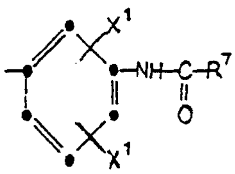 ou 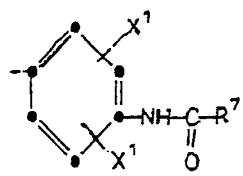,

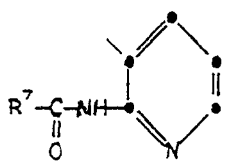 ou 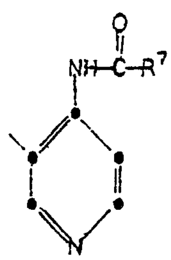, ou 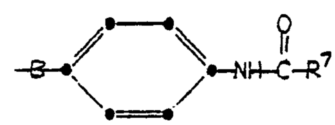,

ou 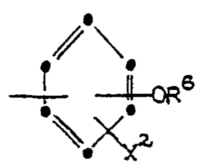

ou un de ses dérivés activés où Q, A, $R^6$, $R^7$, $R^8$, X, $X^1$, $X^2$ et B ont les significations définies précédemment et éventuellement, si on le désire, éliminer l'un ou l'autre groupe protecteur qui peut être présent dans le produit de la réaction.

13. Procédé de préparation d'un peptide cyclique A—21978C selon l'une quelconque des revendications 4 à 8, ce procédé comprenant:

(a) la protection d'un ou plusieurs des groupes amino NHR, $NHR^1$ et $NHR^2$ afin de préparer un composé de formule (1) dans lequel un ou plusieurs des radicaux R, $R^1$ et $R^2$ représentent un groupe protecteur du groupe amino, avec cette réserve qu'un ou plusieurs des radicaux R, $R^1$ et $R^2$ est ou sont initialement des atomes d'hydrogène, et/ou

(b) la déacylation d'un composé de formule (1) dans laquelle R est différent de l'hydrogène ou d'un groupe protecteur du groupe amino, afin de préparer un composé de formule (1) dans laquelle R est un atome d'hydrogène et, éventuellement, si on le désire, éliminer l'un ou l'autre groupe protecteur du groupe amino $R^1$ ou $R^2$ pouvant être présent dans le produit de la réaction.

14. Procédé selon la revendication 13, dans lequel la méthode de déacylation consiste à exposer le composé de formule 1 dans un milieu aqueux à une enzyme qui déacyle et qui est produite par un micro-organisme de la famille *Actinoplanaceae* jusqu'à ce qu'une importante déacylation soit effectuée.

15. Procédé selon la revendication 14, dans lequel le micro-organisme de la famille *Actinoplanaceae* est un membre du genre *Actinoplanes.*

16. Procédé selon la revendication 15, dans lequel le micro-organisme est choisi parmi *A. utahensis* NRRL 12052, *Streptosporangium roseum* var. *hollandensis* NRRL 12064, *Actinoplanes missouriensis* NRRL 12053, *Actinoplanes sp.* NRRL 12065 ou *Actinoplanes sp.* NRRL 8122; ou encore un de leurs mutants ou variants.

17. Procédé selon l'une quelconque des revendications 14 à 16, dans lequel l'enzyme est présente dans une culture du micro-organisme producteur d'*Actinoplanaceae*.

18. Procédé de préparation d'un dérivé de peptide cyclique A—21978C de formule *1* selon l'une quelconque des revendications 10 ou 11, caractérisé en ce qu'il consiste à faire réagir un noyau de peptide cyclique A—21978C de formule *3* comme défini dans la revendication 12,

(a) avec un agent d'acylation de formule (*7*):

$$R^{10}\text{—COOH} \qquad (7)$$

ou un de ses dérivés activés, où $R^{10}$ représente un groupe alcényle en $C_4$—$C_{18}$ ou un groupe alkyle en $C_1$—$C_{18}$ éventuellement substitué; ou

(b) avec un dérivé carbonyle de formule (*8*):

$$R^{11}\text{—}\underset{\underset{O}{\|}}{C}\text{—}R^{12} \qquad (8)$$

où le groupe $R^{11}R^{12}C$= représente un groupement alkylidène en $C_4$—$C_{14}$;

(c) éventuellement réduire un produit alkylidène en $C_4$—$C_{14}$ du procédé (b) ci-dessus pour former un composé de formule *1* dans laquelle R, $R^1$ ou $R^2$ représente un groupe alkyle en $C_4$—$C_{14}$;

(d) et, si on le désire, éliminer l'un ou l'autre groupe protecteur pouvant être présent dans le produit de l'une ou l'autre des réactions (a), (b) ou (c) ci-dessus.

19. Formulation pharmaceutique comprenant, comme ingrédient actif, un dérivé A—21978C de formule (1) ou un de ses sels pharmaceutiquement acceptables selon l'une quelconque des revendications 1 à 11, en association avec un ou plusieurs véhicules ou supports physiologiquement acceptables.

20. Dérivé A—21978C de formule (1) ou un de ses sels pharmaceutiquement acceptables selon l'une quelconque des revendications 1 à 11 en vue de l'utiliser come antibiotique.

21. Composé selon la revendication 1, à savoir le noyau $N_{Trp}$-(n-décanoyl)-A—21978C.

22. Noyau $N_{Trp}$-(n-décanoyl)-A—21978C purifié ou un de ses sels pharmaceutiquement acceptables.

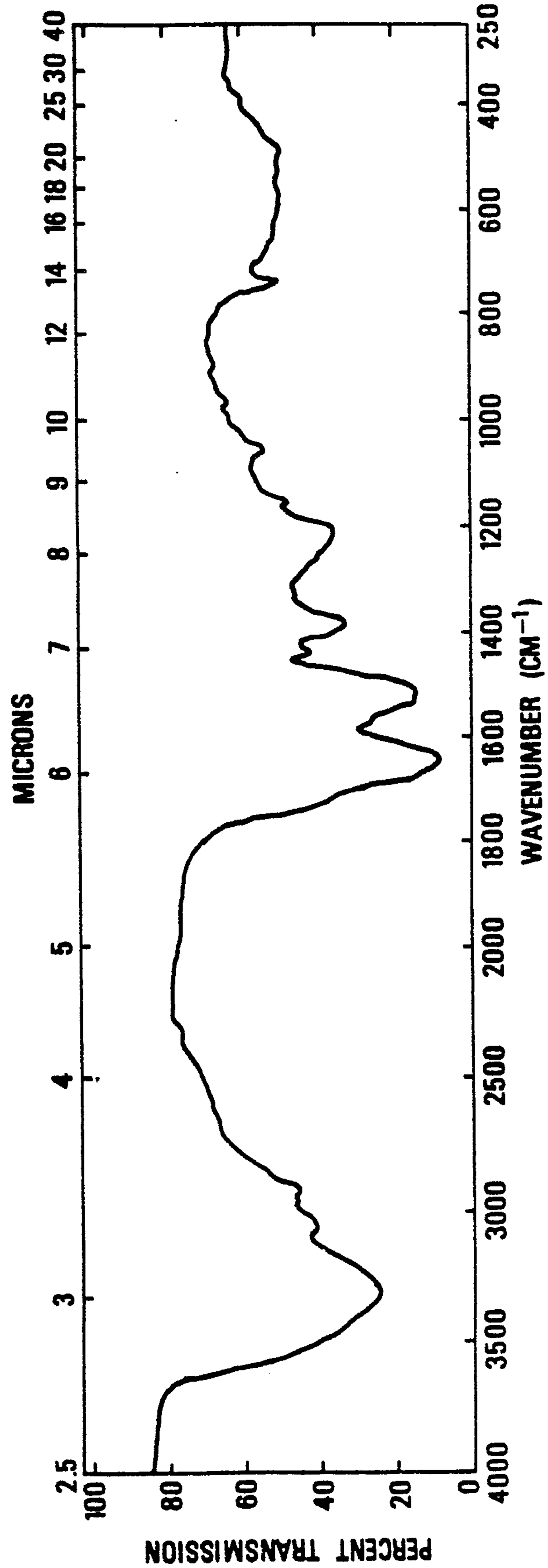
MICRONS
2.5
3
4
5
6
7
8
9
10
12
14
16 18 20
25 30 40
PERCENT TRANSMISSION
100
80
60
40
20
0
WAVENUMBER (CM$^{-1}$)
4000
3500
3000
2500
2000
1800
1600
1400
1200
1000
800
600
400
250

FIG. I

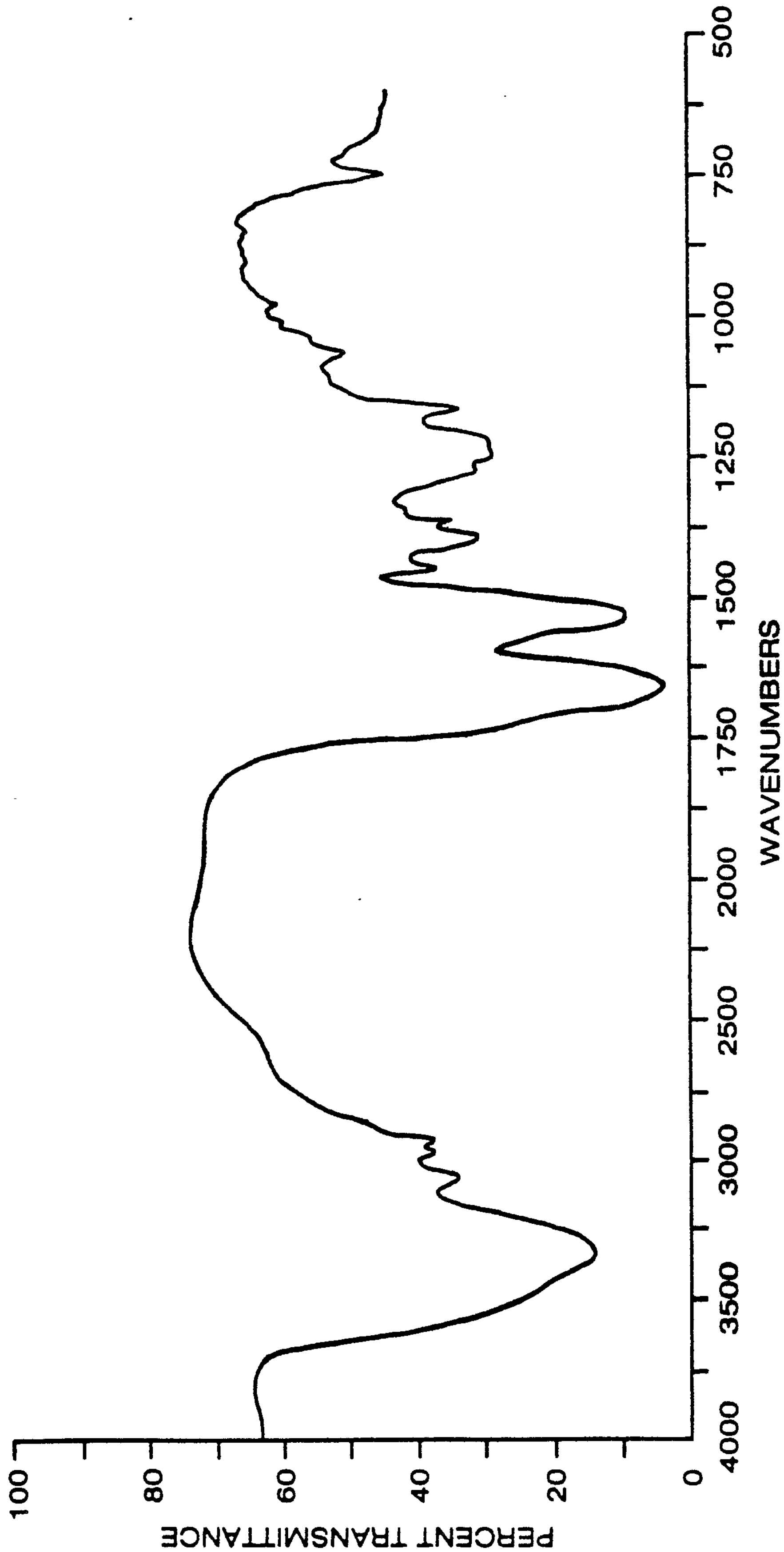
PERCENT TRANSMITTANCE
100
80
60
40
20
0
4000
3500
3000
2500
2000
1750
1500
1250
1000
750
500
WAVENUMBERS

FIG. 2